# EUROPEAN PATENT APPLICATION

(11) **EP 4 801 249 A1**
(43) Date of publication of application: **02.09.2026**
(21) Application number: 24882372.6
(22) Date of filing: 22.10.2024
(51) Int. Cl.: H10K 85/10, C07D 513/04, C08F 232/00, C08G 61/12, H10D 30/67, H10K 10/46

(54) **ORGANIC TRANSISTOR**

(30) Priority: 25.10.2023 JP 2023183025; 03.04.2024 JP 2024059858
(71) Applicant: Tosoh Corporation, Shunan-shi, Yamaguchi 746-8501 (JP)
(72) Inventor: HARADA, Shotaro, Yokkaichi-shi, Mie 510-8540 (JP); MIYASHITA, Masato, Yokkaichi-shi, Mie 510-8540 (JP); OKU, Shinya, Yokkaichi-shi, Mie 510-8540 (JP); WATANABE, Makoto, Yokkaichi-shi, Mie 510-8540 (JP)
(74) Representative: Novagraaf International SA
(86) International application number: PCT/JP2024/037510
(87) International publication number: WO 2025/089261

(57) **Abstract**

An organic transistor (1001) of the present disclosure includes a gate electrode (3), a source electrode (5), a drain electrode (6), an organic semiconductor film (1), and a first gate insulating film (4) in contact with the organic semiconductor film (1). The organic semiconductor film (1) includes a conjugated polymer indicated by a structural unit represented by general formula (2) below and a structural unit represented by general formula (3) below, and an interfacial energy between the first gate insulating film (4) and the organic semiconductor film is 2.0 mJ/m² or less. 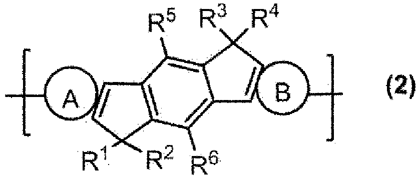 where A and B each represent an aromatic ring linking group which may be substituted, R¹, R², R³, and R⁴ each represent an alkyl group, and R⁵ and R⁶ each represent a hydrogen atom, a fluorine atom, or an alkyl group. 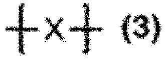 where X represents a heteroaromatic ring linking group.

## Description

### Technical Field

The present invention relates to an organic transistor.

### Background Art

Organic semiconductor devices, typified by organic transistors, have been attracting attention in recent years, because they have features that are not found in inorganic semiconductor devices, such as features of being energy-saving, low-cost, and flexible.

The organic semiconductor devices are constituted by several kinds of material, such as organic semiconductor layers, substrates, insulating layers, and electrodes. Among them, the organic semiconductor layers, which are responsible for carrier transfer of electric charges, play central roles in the devices.

The performance of the organic semiconductor devices depends on the carrier mobility of organic materials of which the organic semiconductor layers are made. Therefore, appearance of organic materials that impart high carrier mobility has been desired.

As a method for producing an organic semiconductor layer, methods such as a vacuum deposition method which is carried out by vaporizing an organic material at a high temperature under vacuum, and a coating method in which an organic material is dissolved in an appropriate solvent and the resultant solution is applied, are generally known. Coating can also be carried out using printing technology without using high-temperature and high-vacuum conditions. Therefore, coating is considered an economically preferable process, and an organic semiconductor layer having high coatability and excellent carrier mobility is demanded.

In general, carrier mobility and variation in performance are in a trade-off relationship, and highly crystalline low-molecular-weight semiconductors tend to readily provide high mobility, while exhibiting large variation in performance.

Polymer semiconductors are less susceptible to the effects of crystallinity of a compound and film quality after coating and film formation and have small variation. However, improvement in mobility is demanded, and realization of a coating-type organic TFT having high carrier mobility has become essential for device applications.

In addition to high carrier mobility, the organic semiconductors are desired to have all high heat resistance, high atmospheric stability, and high solubility. However, few examples of compounds having all of these properties have been reported. For example, a donor-acceptor polymer semiconductor disclosed in Patent Literature 1 is known, but further improvement in mobility is demanded.

Among these physical properties, heat resistance (melting point or glass transition temperature), atmospheric stability (ionization potential), and solubility (solubility in an organic solvent) are values inherent to a compound, whereas mobility is known to vary depending on a crystal state of a compound and a device structure.

### Citation List

### [Patent Literature]

[Patent Literature 1]
Japanese Patent Application Publication Tokukai No. 2023-008031

### Summary of Invention

### Technical Problem

The present invention is accomplished in view of the above problems, and an object of the present invention is to provide an organic transistor having high carrier mobility.

### Solution to Problem

As a result of diligent studies to solve the above problems, the inventors of the present invention have found that a polymer semiconductor-type organic transistor having high carrier mobility can be formed based on an interfacial energy difference between a first gate insulating film and an organic semiconductor film, and thus have accomplished the present invention.

That is, the present invention is as follows.

<1> An organic transistor including: a gate electrode, a source electrode, a drain electrode, and an organic semiconductor film; and a first gate insulating film in contact with the organic semiconductor film, or a first gate insulating film in contact with the organic semiconductor film and a second gate insulating film not in contact with the organic semiconductor film, the organic semiconductor film containing a conjugated polymer composed of a structural unit represented by general formula (2) below and a structural unit represented by general formula (3) below, and an interfacial energy between the first gate insulating film and the organic semiconductor film being 2.0 mJ/m² or less, 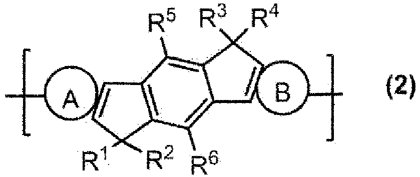 where A and B each independently represent a monovalent aromatic ring linking group which may be substituted with an alkyl group having 1 to 50 carbon atoms or an alkoxy group having 1 to 50 carbon atoms, and R¹, R², R³, and R4 each independently represent an alkyl group having 1 to 50 carbon atoms, R¹ and R² may be bonded to each other to form a ring together with a carbon atom to which R¹ and R² are bonded, R³ and R⁴ may be bonded to each other to form a ring together with a carbon atom to which R³ and R⁴ are bonded, and R⁵ and R⁶ each independently represent a hydrogen atom, a fluorine atom, or an alkyl group having 1 to 50 carbon atoms. 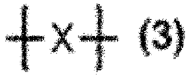 where X represents a divalent heteroaromatic ring linking group which may be substituted with an alkyl group having 1 to 50 carbon atoms or an alkoxy group having 1 to 50 carbon atoms.

### Advantageous Effects of Invention

The organic transistor in accordance with an aspect of the present invention has high carrier mobility.

### Brief Description of Drawings

Fig. 1 is a diagram illustrating a structure based on a cross-sectional shape of an organic transistor in accordance with an aspect of the present invention.
Fig. 2 is a diagram illustrating a structure of an organic transistor in accordance with an aspect of the present invention.

### Description of Embodiments

The present invention is described below in detail.

### [Organic transistor]

The organic transistor in accordance with an aspect of the present invention includes a gate electrode, a source electrode, a drain electrode, and an organic semiconductor film. The organic transistor further includes a first gate insulating film in contact with the organic semiconductor film and a second gate insulating film not in contact with the organic semiconductor film. Note that the second gate insulating film may be present or absent.

Examples of the gate electrode used in the organic transistor in accordance with an aspect of the present invention include: inorganic electrodes such as aluminum, gold, silver, copper, highly doped silicon, tin oxide, indium oxide, indium tin oxide, chromium, titanium, tantalum, chromium, graphene, and carbon nanotube; and organic electrodes such as a doped conductive polymer (PEDOT-PSS); and the like. Among these, inorganic electrodes are preferable from the viewpoint of good conductivity, and silver or gold is more preferable.

Materials of the source electrode and the drain electrode used in the organic transistor in accordance with an aspect of the present invention are not particularly limited, and materials similar to that of the gate electrode can be used. The materials may be identical to or different from that of the gate electrode, and different kinds of materials may be stacked. In order that the efficiency of injecting carriers is increased, a surface treatment can be carried out with respect to these electrode materials. Examples of such a surface treatment agent for an electrode material include benzenethiol and pentafluorobenzenethiol.

In a case where surface treatment of an electrode is carried out, a surface treatment agent may be diluted with a solvent and used. The solvent used for dilution is not particularly limited, and examples thereof include alcohol-based solvents such as methanol, ethanol, and 2-propanol; halogen-based solvents such as o-dichlorobenzene, chlorobenzene, 1,2-dichloroethane, 1,1,2,2-tetrachloroethane, and chloroform; ether-based solvents such as tetrahydrofuran (THF) and dioxane; hydrocarbon-based solvents of aromatic compounds such as toluene, xylene, and mesitylene; ester-based solvents such as ethyl acetate and γ-butyrolactone; and amide-based solvents such as N,N-dimethylformamide and N-methylpyrrolidone.

### (Interfacial energy)

The first gate insulating film used in the organic transistor in accordance with an aspect of the present invention has an interfacial energy between the gate insulating film and the organic semiconductor film of 2.0 mJ/m² or less, preferably 1.5 mJ/m² or less, further preferably 1.0 mJ/m² or less, and more preferably 0.5 mJ/m² or less. By setting the interfacial energy within the above range, adhesion property between the first gate insulating film and the organic semiconductor film is improved, and mobility in the organic transistor is improved.

In an aspect of the present invention, the interfacial energy between the first gate insulating film and the organic semiconductor film is calculated by the following formula (a).${γ}_{12} = {γ}_{1} + {γ}_{2} - 2 {\left({{γ}_{1}}^{d}{{γ}_{2}}^{d}\right)}^{1 / 2} - 2 {\left({{γ}_{1}}^{p}{{γ}_{2}}^{p}\right)}^{1 / 2}$ where γ₁₂ represents an interfacial energy, γ₁ represents a surface energy of the first gate insulating film, γ₁^{d} represents a dispersion force of the first gate insulating film, γ₁^{p} represents a polar component of the first gate insulating film, γ₂ represents a surface energy of the organic semiconductor film, γ₂^{d} represents a dispersion force of the organic semiconductor film, and γ₂^{p} represents a polar component of the organic semiconductor film. These values can be calculated by measuring a contact angle of water and a contact angle of iodomethane by a θ/2 method using respective thin films of the first gate insulating film and the organic semiconductor film, and by using the Owens-Wendt method.

### (Gate insulating film)

In an aspect of the present invention, a material used for the gate insulating film is not particularly limited. Specific examples thereof include inorganic insulating layers such as silicon oxide, silicon nitride, aluminum oxide, aluminum nitride, titanium oxide, tantalum dioxide, tantalum pentoxide, indium tin oxide, tin oxide, vanadium oxide, barium titanate, and bismuth titanate; polyethylene terephthalate, polyethylene naphthalate, polymethyl methacrylate, polymethyl acrylate, polyethylene, polypropylene, polystyrene, cyclic polyolefin, polyimide, polycarbonate, polyvinyl phenol, polyvinyl alcohol, poly(diisopropyl fumarate), poly(diethyl fumarate), poly(diisopropyl maleate), polyether sulfone, polyphenylene sulfide, cellulose triacetate, polycyclopentane, polyalkylnorbornene, a polycyclohexane-ethylene copolymer, polyfluorinated cyclopentane, CYTOP (registered trademark), polyfluorinated cyclohexane, a polyfluorinated cyclohexane-ethylene copolymer, Parylene N (registered trademark), Parylene C (registered trademark), Parylene D (registered trademark), Parylene HT (registered trademark), Parylene C-UVF (registered trademark), cycloolefin copolymers such as APEL (registered trademark) and TOPAS (registered trademark), fluorine resins containing a repeating unit represented by general formula (105) below and a repeating unit represented by general formula (106) below, and resins containing a repeating unit represented by general formula (107) below and a repeating unit represented by general formula (108) below. Among these, from the viewpoint of good insulating properties, polystyrene, Parylene C (registered trademark), a cycloolefin copolymer, a fluorine resin containing a repeating unit represented by general formula (105) below and a repeating unit represented by general formula (106) below, and a resin containing a repeating unit represented by general formula (107) below and a repeating unit represented by general formula (108) below are more preferable, and polystyrene, a cycloolefin copolymer, a fluorine resin containing a repeating unit represented by general formula (105) below and a repeating unit represented by general formula (106) below, and a resin containing a repeating unit represented by general formula (107) below and a repeating unit represented by general formula (108) below are especially preferable.

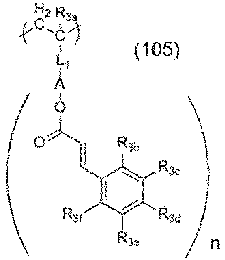

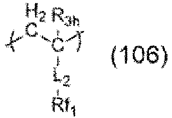

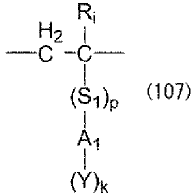

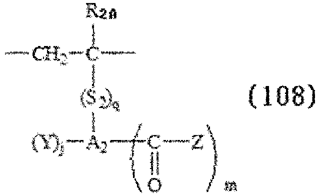

### (Repeating unit represented by general formula (105))

In general formula (105), R₃ₐ represents a hydrogen atom or a methyl group.

In general formula (105), L₁ represents a single bond or a divalent linking group. The divalent linking group at L₁ is preferably a divalent linking group obtained by combining at least two groups selected from the group consisting of linear alkylene groups having 1 to 10 carbon atoms, branched alkylene groups having 3 to 10 carbon atoms or cyclic alkylene groups having 3 to 10 carbon atoms, arylene groups having 6 to 12 carbon atoms, an ether group (-O-), a carbonyl group (-C(=O)-), and an imino group (-NH-). This makes it possible to form a flat and non-cracked film.

Specific examples of the linear alkylene group having 1 to 10 carbon atoms include a methylene group, an ethylene group, a propylene group, a butylene group, a pentylene group, a hexylene group, a decylene group, and the like.

Specific examples of the branched alkylene group having 3 to 10 carbon atoms include a dimethylmethylene group, a methylethylene group, a 2,2-dimethylpropylene group, a 2-ethyl-2-thylpropylene group, and the like.

Specific examples of the cyclic alkylene group having 3 to 10 carbon atoms include a cyclopropylene group, a cyclobutylene group, a cyclopentylene group, a cyclohexylene group, a cyclooctylene group, a cyclodecylene group, an adamantane-diyl group, a norbornane-diyl group, an exo-tetrahydrodicyclopentadiene-diyl group, and the like. Among these, the cyclohexylene group is preferable.

Specific examples of the arylene group having 6 to 12 carbon atoms include a phenylene group, a xylylene group, a biphenylene group, a naphthylene group, a 2,2'-methylenebisphenyl group, and the like. Among these, the phenylene group is preferable.

Among the above examples, the divalent linking group is more preferably an ester bond (-C(=O)O-) obtained by combining a carbonyl group and an ether group, or a linking group obtained by combining a phenylene group and an ether group, and is further preferably (-C(=O)O-).

In general formula (105), A represents a linking group having a valence of m. In order to achieve better solubility of the obtained resin in an organic solvent and in a fluorine-based solvent, A may be a hydrocarbon group of 1 to 24 carbon atoms with an m-valent value, which may contain a substituent.

Examples of the substituent which the hydrocarbon group A having a valence of m may have include an alkyl group, an alkoxy group, a halogen atom, a hydroxyl group, and the like.

For example, the alkyl group is preferably a linear, branched, or cyclic alkyl group having 1 to 18 carbon atoms, more preferably an alkyl group having 1 to 8 carbon atoms (e.g., a methyl group, an ethyl group, a propyl group, an isopropyl group, an n-butyl group, an isobutyl group, a sec-butyl group, a t-butyl group, a cyclohexyl group, or the like), further preferably an alkyl group having 1 to 4 carbon atoms, and particularly preferably a methyl group or an ethyl group.

Examples of the alkoxy group include alkoxy groups having 1 to 16 carbon atoms and having a linear or branched alkyl group, such as a methoxy group, an ethoxy group, an n-propoxy group, an n-butoxy group, an isobutoxy group, an n-pentyloxy group, an n-hexylixy group, an isohexyloxy group, an n-heptyloxy group, an n-octyloxy group, an n-nonyloxy group, an n-decyloxy group, an n-dodecyloxy group, an n-tetradecyloxy group, a 2-ethylhexyloxy group, a 3-ethylheptyloxy group, and a 2-hexyldecyloxy group, and is particularly preferably a group selected from the group consisting of a methoxy group, an ethoxy group, an n-propoxy group, an n-butoxy group, an isobutoxy group, an n-pentyloxy group, an n-hexyloxy group, an isohexyloxy group, an n-heptyloxy group, and an n-octyloxy group.

Examples of the halogen atom include a fluorine atom, a chlorine atom, a bromine atom, an iodine atom, and the like. Among these, the fluorine atom and the chlorine atom are preferable.

Among these, the hydrocarbon group A having a valence of m is preferably one linking group selected from the group consisting of general formulae (a-1) through (a-4) below.

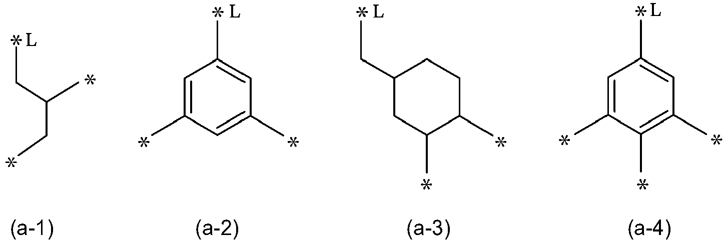

In general formulae (a-1) through (a-4), *L represents a linkage position with L₁ in the above general formula (105), and * attached to a carbon atom represents a linkage position with an oxygen atom constituting an ester group in the above general formula (105).

For the reason of ease of reaction in monomer synthesis, the hydrocarbon group A having a valence of m is preferably one trivalent linking group selected from the group consisting of general formulae (a-1), (a-2), and (a-3), more preferably a trivalent linking group of general formula (a-1) or (a-2), and further preferably a trivalent linking group of general formula (a-1).

In general formula (105), R_{3b}, R_{3c}, R_{3d}, R₃ₑ and R_{3f}, which are identical to each other or different from each other, each represent one selected from the group consisting of a hydrogen atom, a halogen atom, linear alkyl groups having 1 to 20 carbon atoms, branched alkyl groups having 3 to 20 carbon atoms, cyclic alkyl groups having 3 to 20 carbon atoms, linear halogenated alkyl groups having 1 to 20 carbon atoms, alkoxy groups having 1 to 20 carbon atoms, aryl groups having 6 to 20 carbon atoms, aryloxy groups having 6 to 20 carbon atoms, a cyano group, and an amino group.

Examples of the halogen atom include a fluorine atom, a chlorine atom, a bromine atom, an iodine atom, and the like. Among these, the fluorine atom and the chlorine atom are preferable.

The linear alkyl group having 1 to 20 carbon atoms is preferably an alkyl group having 1 to 6 carbon atoms, and specific examples thereof include a methyl group, an ethyl group, an n-propyl group, and the like. Among these, the methyl group or the ethyl group is preferable.

The branched alkyl group having 3 to 20 carbon atoms is preferably an alkyl group having 3 to 6 carbon atoms, and specific examples thereof include an isopropyl group, a tert-butyl group, and the like.

The cyclic alkyl group having 3 to 20 carbon atoms is preferably an alkyl group having 3 to 6 carbon atoms, and specific examples thereof include a cyclopropyl group, a cyclopentyl group, a cyclohexyl group, and the like. Among these, the cyclohexyl group is preferable.

The linear halogenated alkyl group having 1 to 20 carbon atoms is preferably a fluoroalkyl group having 1 to 4 carbon atoms, and specific examples thereof include a trifluoromethyl group, a perfluoroethyl group, a perfluoropropyl group, a perfluorobutyl group, and the like. Among these, the trifluoromethyl group is preferable.

The alkoxy group having 1 to 20 carbon atoms is preferably an alkoxy group having 1 to 8 carbon atoms, and specific examples thereof include a methoxy group, an ethoxy group, an n-butoxy group, a methoxyethoxy group, and the like.

The aryl group having 6 to 20 carbon atoms is preferably an aryl group having 6 to 12 carbon atoms, and specific examples thereof include a phenyl group, an α-methylphenyl group, a naphthyl group, and the like. Among these, the phenyl group is preferable.

The aryloxy group having 6 to 20 carbon atoms is preferably an aryloxy group having 6 to 12 carbon atoms, and specific examples thereof include a phenyloxy group, a 2-naphthyloxy group, and the like. Among these, the phenyloxy group is preferable.

Examples of the amino group include a primary amino group (-NH₂); a secondary amino group such as a methylamino group; and a tertiary amino group such as a dimethylamino group, a diethylamino group, a dibenzylamino group, and a group bonded through a nitrogen atom of a nitrogen-containing heterocyclic compound (e.g., pyrrolidine, piperidine, piperazine, and the like).

In order to enhance solubility in a fluorine-based solvent, photocurability, and liquid-repellency of the fluorine-based resin, R_{3b}, R_{3c}, R_{3d}, R₃ₑ and R_{3f} are each preferably a hydrogen atom, an alkyl group, a halogen atom, or a linear halogenated alkyl group having 1 to 20 carbon atoms, and are each further preferably a hydrogen atom.

Specific examples of the repeating unit (hereafter sometimes referred to as a repeating unit B) represented by general formula (105) include repeating units B-1 through B-26 indicated below. Among these, B-1 through B-16 and the like are preferable, and B-1, B-2, B-13, and B-16 are particularly preferable. Note that, in the following formulae, Me represents a methyl group, Et represents an ethyl group, and Pr represents an isopropyl group.

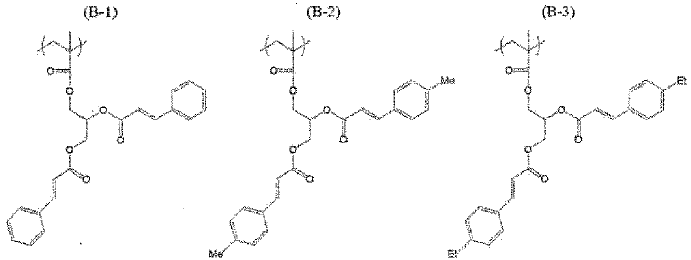

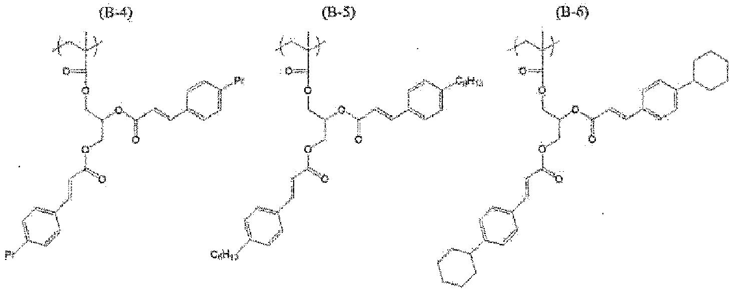

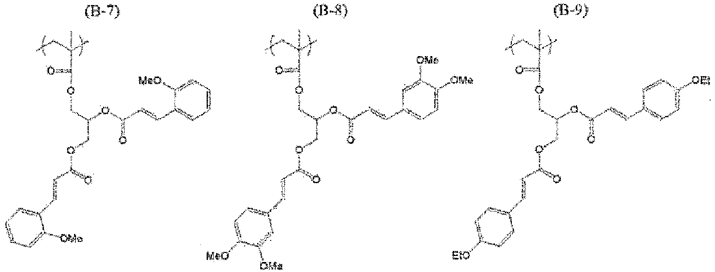

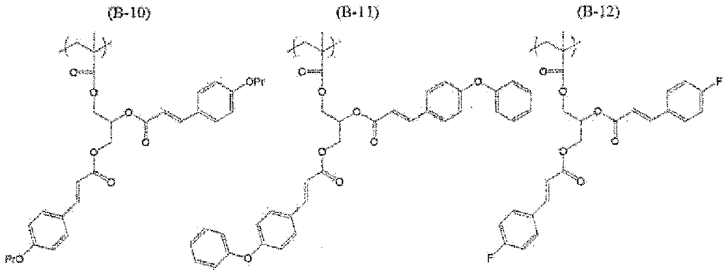

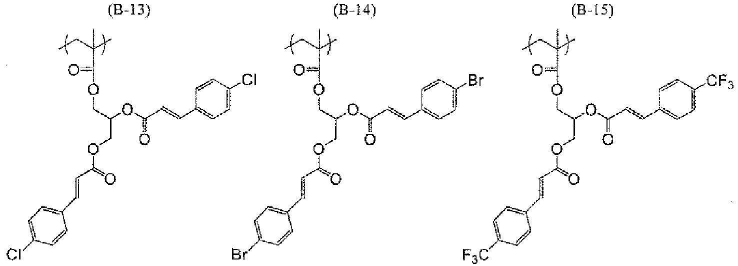

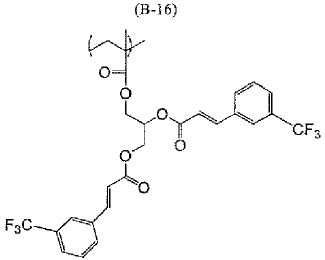

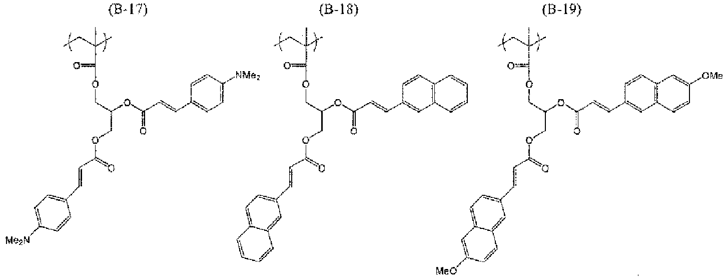

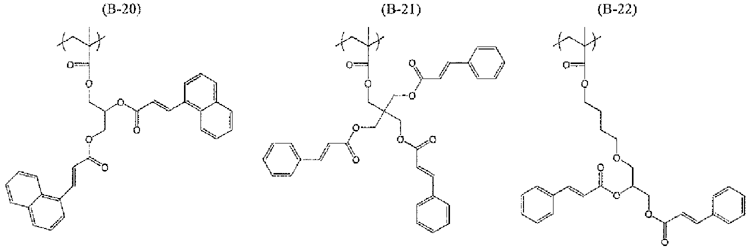

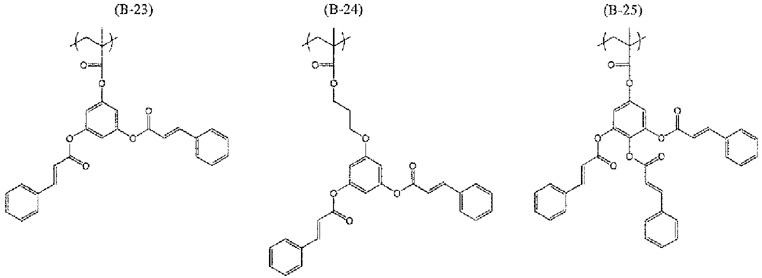

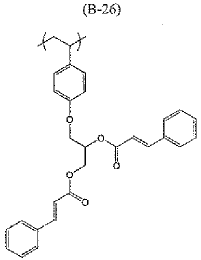

### (Repeating unit represented by general formula (106))

In general formula (106), R₃ₕ represents a hydrogen atom or a methyl group.

In general formula (106), L₂ represents a single bond or a divalent linking group. The divalent linking group in L₂ is preferably a divalent linking group obtained by combining at least two groups selected from the group consisting of linear alkylene groups having 1 to 10 carbon atoms, branched alkylene groups having 3 to 20 carbon atoms or cyclic alkylene groups having 3 to 20 carbon atoms, arylene groups having 6 to 12 carbon atoms, an ether group (-O-), a carbonyl group (-C(=O)-), and an imino group (-NH-). This makes it possible to form a flat and non-cracked film.

Specific examples of the linear alkylene group having 1 to 10 carbon atoms include a methylene group, an ethylene group, a propylene group, a butylene group, a pentylene group, a hexylene group, a decylene group, and the like.

Specific examples of the branched alkylene group having 3 to 10 carbon atoms include a dimethylmethylene group, a methylethylene group, a 2,2-dimethylpropylene group, a 2-ethyl-2-thylpropylene group, and the like.

Specific examples of the cyclic alkylene group having 3 to 10 carbon atoms include a cyclopropylene group, a cyclobutylene group, a cyclopentylene group, a cyclohexylene group, a cyclooctylene group, a cyclodecylene group, an adamantane-diyl group, a norbornane-diyl group, an exo-tetrahydrodicyclopentadiene-diyl group, and the like. Among these, the cyclohexylene group is preferable.

Specific examples of the arylene group having 6 to 12 carbon atoms include a phenylene group, a xylylene group, a biphenylene group, a naphthylene group, a 2,2'-methylenebisphenyl group, and the like. Among these, the phenylene group is preferable.

Among the above examples, the divalent linking group is more preferably an ester bond (-C(=O)O-) obtained by combining a carbonyl group and an ether group, or a linking group obtained by combining a phenylene group and an ether group, and is further preferably (-C(=O)O-).

In general formula (106), Rf₁ represents one selected from the group consisting of linear fluoroalkyl groups having 1 to 15 carbon atoms, branched fluoroalkyl groups having 3 to 15 carbon atoms, and cyclic fluoroalkyl groups having 3 to 15 carbon atoms.

In a case where Rf₁ is a fluoroalkyl group, the fluorine-based resin in accordance with an aspect of the present invention exhibits affinity with a fluorine-based solvent and liquid-repellency.

In a case where Rf₁ is a linear fluoroalkyl group, specific examples of Rf₁ include alkyl groups having 10 to 14 carbon atoms, a methyl group, an ethyl group, a propyl group, a butyl group, a pentyl group, a hexyl group, a heptyl group, an octyl group, and a nonyl group each of which is substituted by a fluorine atom. In a case where an element binding to Rf₁ in L₂ is oxygen, a position substituted by the fluorine atom in Rf₁ only needs to be on a carbon atom other than a carbon atom that is directly bound to oxygen in L₂.

In a case where Rf₁ is a linear fluoroalkyl group, Rf₁ is preferably a group represented by general formula (109) below.

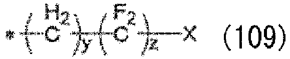

In general formula (109), * represents a linkage position with L₂ in general formula (106).

In general formula (109), X is a hydrogen atom or a fluorine atom.

In general formula (109), y is an integer of 1 to 4, and preferably 1 to 2.

In general formula (109), z is an integer of 1 to 14, preferably 2 to 10, and further preferably 4 to 8.

In a case where Rf₁ is a group represented by general formula (109), it becomes easier to synthesize a monomer that is used as a raw material for the repeating unit represented by general formula (106).

In a case where Rf₁ is a branched fluoroalkyl group, specific examples of Rf₁ include a 1,1,1,3,3,3-hexafluoroisopropyl group, a 1-(trifluoromethyl)-2,2,3,3,3-pentafluoropropyl group, a 1,1-bis(trifluoromethyl)-2,2,2-trifluoroethyl group, and a 1,1-bis(trifluoromethyl)ethyl group.

In a case where Rf₁ is a cyclic fluoroalkyl group, specific examples of Rf₁ include a 1,2,2,3,3,4,4,5,5-nonafluorocyclopentane group and a 1,2,2,3,3,4,4,5,5,6,6-undecafluorocyclohexane group.

The repeating unit represented by the above general formula (106) is preferably a repeating unit represented by general formula (110) below.

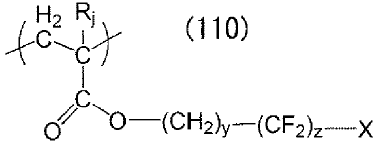

In general formula (110), Rⱼ represents either a hydrogen atom or a methyl group.

In general formula (110), X is a hydrogen atom or a fluorine atom.

In general formula (110), y is an integer of 1 to 4, and preferably 1 to 2.

In general formula (110), z is an integer of 1 to 14, preferably 2 to 10, and further preferably 4 to 8.

Specific examples of the repeating unit including a fluorine atom include one selected from the group consisting of repeating units represented by Formulae (C-1) through (C-33) below.

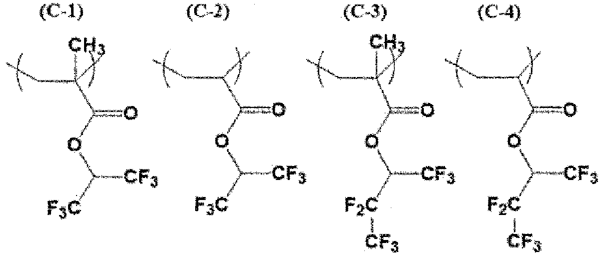

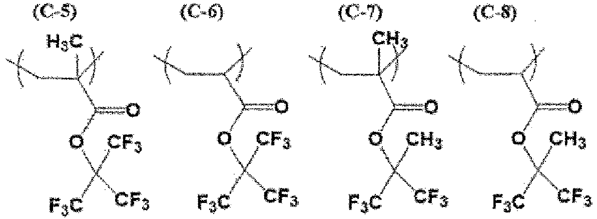

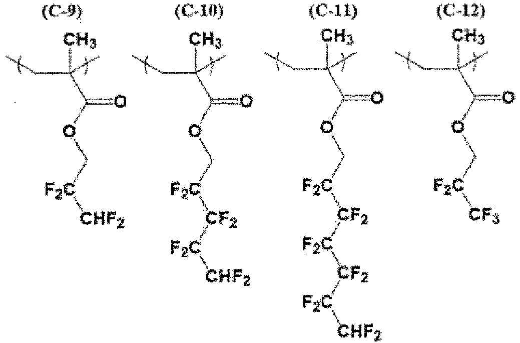

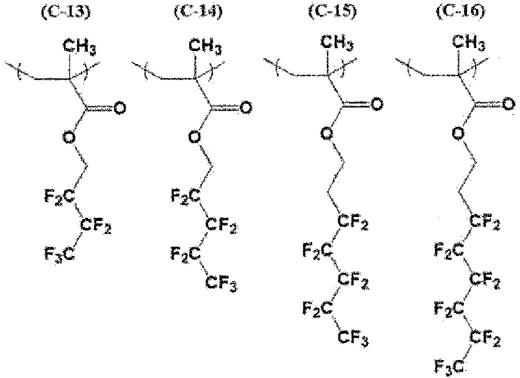

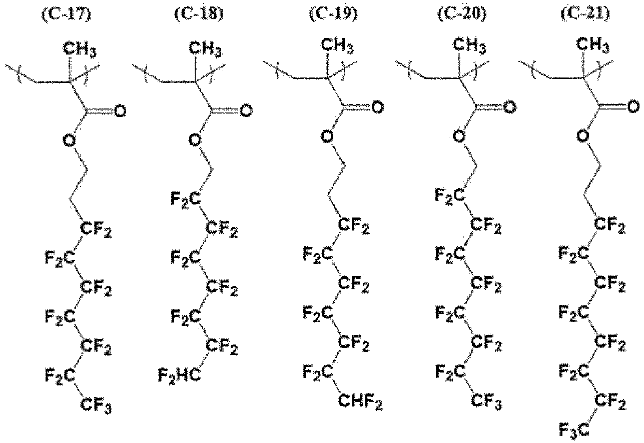

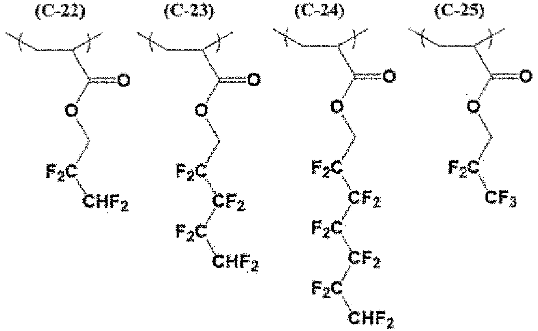

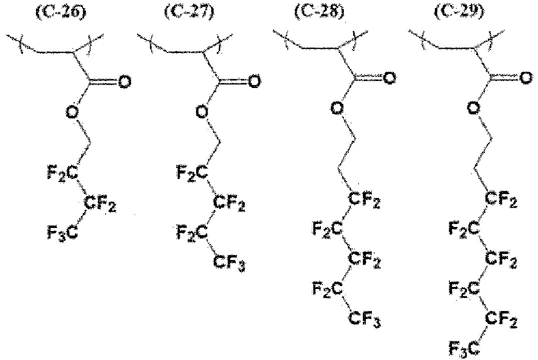

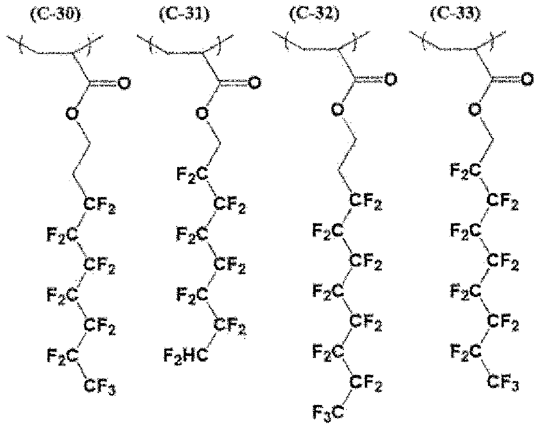

The repeating unit is preferably one selected from the group consisting of repeating units represented by the above Formulae (C-1) through (C-33), further preferably one selected from the group consisting of repeating units represented by Formulae (C-9) through (C-33), and particularly preferably one selected from either the group consisting of repeating units represented by Formulae (C-14) through (C-21) or the group consisting of repeating units represented by Formulae (C-27) through (C-33).

### (Repeating unit represented by general formula (107))

In general formula (107), Rᵢ represents a hydrogen atom or a C1-C6 alkyl group, and a hydrogen atom is preferable.

The C1-C6 alkyl group as Rᵢ in general formula (107) is not particularly limited, and examples thereof include a methyl group, an ethyl group, an n-propyl group, an isopropyl group, an n-butyl group, and the like.

In general formula (107), S₁ represents -O- or -C(O)-.

In general formula (107), p represents 0 or 1, and 0 is preferable.

In general formula (107), A₁ represents a C6-C19 aryl group.

The C6-C19 aryl group as A₁ in general formula (107) is not particularly limited, and examples thereof include a phenyl group, a naphthyl group, an anthryl group, a biphenyl group, and the like, and a phenyl group is preferable.

In general formula (107), Y represents a halogen atom, a cyano group, a nitro group, a carboxyalkyl group, an alkyl ether group, an aryl ether group, a C1-C18 alkyl group, a fluoroalkyl group, or a cycloalkyl group.

The halogen atom as Y in general formula (107) is not particularly limited, and examples thereof include a chlorine atom, a fluorine atom, a bromine atom, and the like.

The carboxyalkyl group as Y in general formula (107) is not particularly limited, and examples thereof include a carboxymethyl group (-COOCH₃), a carboxyethyl group (-COOCH₂CH₃), a carboxypropyl group (-COOCH₂CH₂CH₃, etc.), and the like.

The alkyl ether group as Y in general formula (107) is not particularly limited, and examples thereof include a methoxy group, an ethoxy group, an n-propoxy group, an isopropoxy group, a butoxy group, and the like.

The aryl ether group as Y in general formula (107) is not particularly limited, and examples thereof include a phenoxy group, a p-methylphenoxy group, a p-ethylphenoxy group, a p-methoxyphenoxy group, and the like.

The C1-C18 alkyl group as Y in general formula (107) is not particularly limited, and examples thereof include a methyl group, an ethyl group, an n-propyl group, an isopropyl group, an n-butyl group, and the like.

The fluoroalkyl group as Y in general formula (107) is not particularly limited, and examples thereof include a 1,1,1-trifluoroethyl group, a 1,1,1,2,2-pentafluoropropyl group, a 1,1,1,2,2,3,3-heptafluorobutyl group, a trifluoromethyl group, a pentafluoroethyl group, and the like.

The cycloalkyl group as Y in general formula (107) is not particularly limited, and examples thereof include a cyclobutyl group, a cyclopentyl group, a cyclohexyl group, and the like.

In general formula (107), k represents an integer of 0 to (s-1), and 0 is preferable. Here, s represents the number of carbon atoms constituting A₁.

### (Repeating unit represented by general formula (108))

In general formula (108), R_{2A} represents a hydrogen atom or a C1-C6 alkyl group, and a hydrogen atom is preferable.

The C1-C6 alkyl group as R_{2A} in general formula (108) is not particularly limited, and examples thereof include a methyl group, an ethyl group, an n-propyl group, an isopropyl group, an n-butyl group, and the like.

In general formula (108), S₂ represents -O- or -C(O)-.

In general formula (108), q represents 0 or 1, and 0 is preferable.

In general formula (108), A₂ represents a C6-C19 aryl group.

The C6-C19 aryl group as A₂ in general formula (108) is not particularly limited, and examples thereof include a phenyl group, a naphthyl group, an anthranil group, a biphenyl group, and the like, and a phenyl group is preferable.

In general formula (108), Y represents a substituent similar to the substituent defined in formula (1).

In general formula (108), m represents an integer of 1 to (r-j-1). Here, r represents the number of carbon atoms constituting A₂, and j represents an integer of 0 to (r-2). m is preferably 1, and j is preferably 0.

In general formula (108), Z represents at least one organic group selected from formulae (A) through (D), and among these, (A) is preferable.

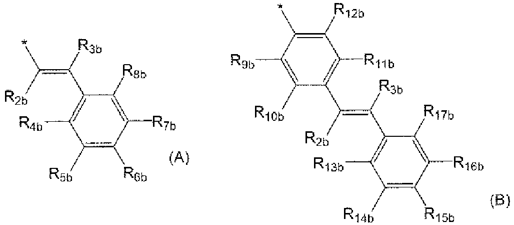

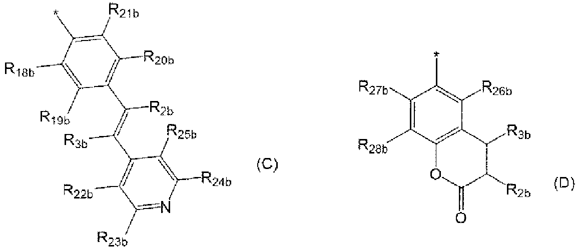

In formulae (A) through (D), R_{2b} and R_{3b} each independently represent a hydrogen atom, a halogen atom, a C1-C6 alkyl group, an aryl group, or a carboxyalkyl group, among which a hydrogen atom, a halogen atom, or a C1-C6 alkyl group is preferable, and a hydrogen atom is more preferable, and R₄ to R₂₈ each independently represent a hydrogen atom, a halogen atom, a cyano group, a nitro group, a carboxyalkyl group, an alkyl ether group, an aryl ether group, a C1-C18 alkyl group, a fluoroalkyl group, or a cycloalkyl group, among which a halogen atom, a cyano group, a nitro group, a carboxyalkyl group, or a fluoroalkyl group is preferable, and a halogen atom, a nitro group, or a fluoroalkyl group is more preferable.

The halogen atom as R_{2b} and R_{3b} in formulae (A) through (D) is not particularly limited, and examples thereof include a chlorine atom, a fluorine atom, a bromine atom, and the like. The C1-C6 alkyl group as R_{2b} and R_{3b} in formulae (A) through (D) is not particularly limited, and examples thereof include a methyl group, an ethyl group, an n-propyl group, an isopropyl group, an n-butyl group, and the like.

The aryl group as R_{2b} and R_{3b} in formulae (A) through (D) is not particularly limited, and examples thereof include a phenyl group, a naphthyl group, an anthryl group, a biphenyl group, and the like.

The carboxyalkyl group as R_{2b} and R_{3b} in formulae (A) through (D) is not particularly limited, and examples thereof include a carboxymethyl group (-COOCH₃), a carboxyethyl group (-COOCH₂CH₃), a carboxypropyl group (-COOCH₂CH₂CH₃, etc.), and the like.

The halogen atom as R_{4b} to R_{28b} in formulae (A) through (D) is not particularly limited, and examples thereof include a chlorine atom, a fluorine atom, a bromine atom, and the like, and a chlorine atom and a fluorine atom are preferable.

The carboxyalkyl group as R_{4b} to R_{28b} in formulae (A) through (D) is not particularly limited, and examples thereof include a carboxymethyl group (-COOCH₃), a carboxyethyl group (-COOCH₂CH₃), a carboxypropyl group (-COOCH₂CH₂CH₃, etc.), and the like.

The alkyl ether group as R_{4b} to R_{28b} in formulae (A) through (D) is not particularly limited, and examples thereof include a methoxy group, an ethoxy group, an n-propoxy group, an isopropoxy group, a butoxy group, and the like.

The aryl ether group as R_{4b} to R_{28b} in formulae (A) through (D) is not particularly limited, and examples thereof include a phenoxy group, a p-methylphenoxy group, a p-ethylphenoxy group, a p-methoxyphenoxy group, and the like.

The C1-C18 alkyl group as R_{4b} to R_{28b} in formulae (A) through (D) is not particularly limited, and examples thereof include a methyl group, an ethyl group, an n-propyl group, an isopropyl group, an n-butyl group, an n-hexyl group, an n-decyl group, an n-octadecyl group, and the like.

The fluoroalkyl group as R_{4b} to R_{28b} in formulae (A) through (D) is not particularly limited, and examples thereof include a 1,1,1-trifluoroethyl group, a 1,1,1,2,2-pentafluoropropyl group, a 1,1,1,2,2,3,3-heptafluorobutyl group, a trifluoromethyl group, a pentafluoroethyl group, and the like, and a trifluoromethyl group is preferable.

The cycloalkyl group as R_{4b} to R_{28b} in formulae (A) through (D) is not particularly limited, and examples thereof include a cyclobutyl group, a cyclopentyl group, a cyclohexyl group, and the like.

Specific examples of the organic group represented by formula (A) include, for example, the following.

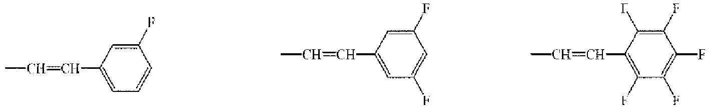

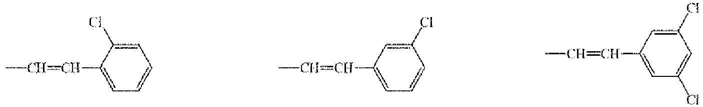

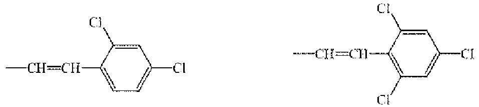

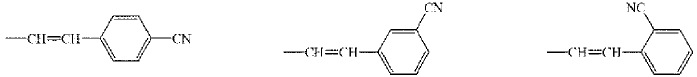

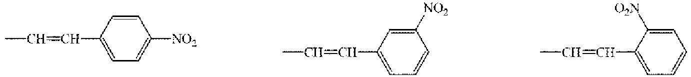

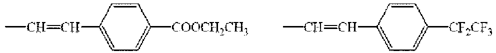

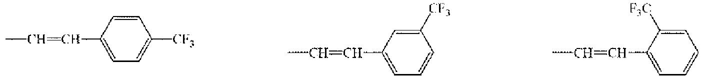

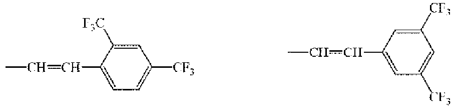

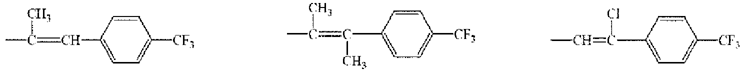

Specific examples of the organic group represented by formula (B) include, for example, the following.

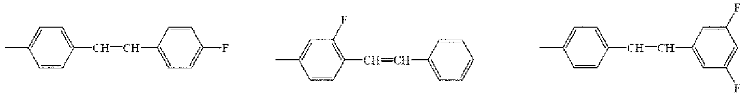

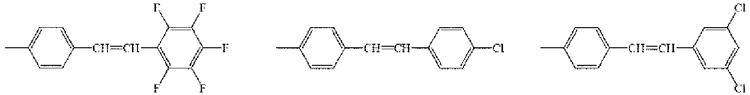

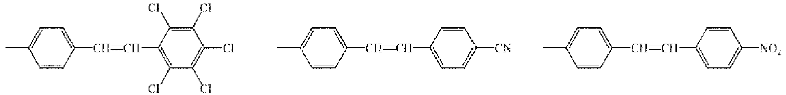

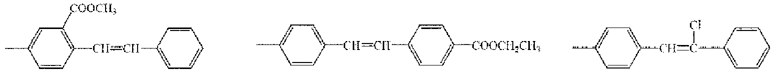

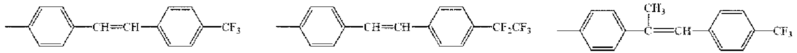

Specific examples of the organic group represented by formula (C) include, for example, the following.

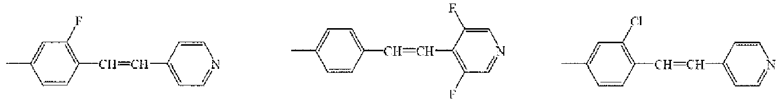

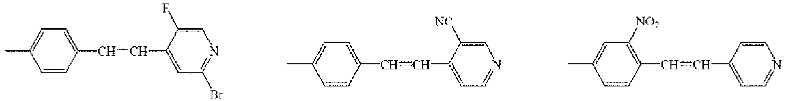

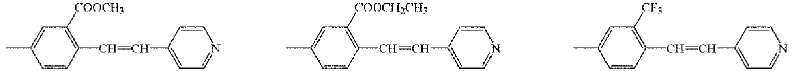

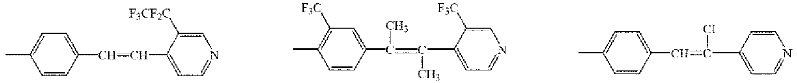

Specific examples of the organic group represented by formula (D) include, for example, the following.

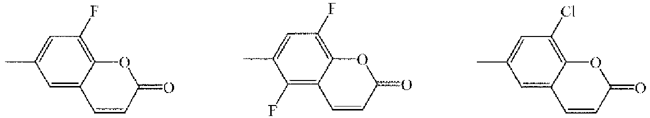

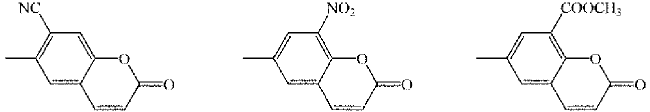

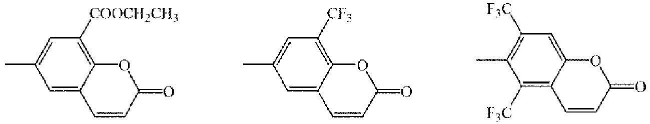

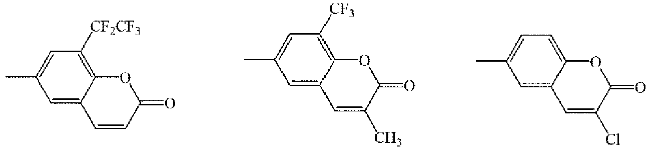

In a case where the organic transistor in accordance with an aspect of the present invention includes a second gate insulating film, a material used for the first gate insulating film and a material used for the second gate insulating film may be the same or may be different.

As the cycloolefin copolymer, a structure represented by general formula (4z-1) or (4z-2) below is preferable, and from the viewpoint of high mobility, a structure represented by general formula (4z-2) (TOPAS (registered trademark)) is more preferable. 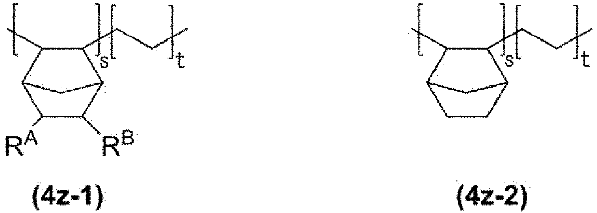 where s and t each represent the number of repetitions, s represents an integer of 1 or more, and t represents an integer of 0 or more. R^{A} and R^{B} each independently represent hydrogen, an alkyl group having 1 to 50 carbon atoms, or an alkoxy group having 1 to 50 carbon atoms. R^{A} and R^{B} may be bonded to each other to form a ring together with a carbon atom to which R^{A} and R^{B} are bonded.

In general formula (4z-1) or (4z-2) above, s may be 6000 or less. Further, t may be 20000 or less.

The alkyl group having 1 to 50 carbon atoms represented by R^{A} and R^{B} may be linear, branched, or cyclic, and examples thereof include linear alkyl groups such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a henicosyl group, a docosyl group, a tricosyl group, a tetracosyl group, a pentacosyl group, a hexacosyl group, a heptacosyl group, an octacosyl group, a nonacosyl group, a triacontyl group, a hentriacontyl group, a dotriacontyl group, a tritriacontyl group, a tetratriacontyl group, a pentatriacontyl group, a hexatriacontyl group, a tetracontyl group, a hentetracontyl group, a dotetracontyl group, a tritetracontyl group, a tetratetracontyl group, and a pentacontyl group; branched alkyl groups such as an isopropyl group, an isobutyl group, a sec-butyl group, a tert-butyl group, a 2-ethylhexyl group, a 3,7-dimethyloctyl group, a 2-hexyloctyl group, a 2-hexyldecyl group, a 2-octyldodecyl group, a 2-decyltetradecyl group, a 2-dodecyltetradecyl group, a 2-dodecylhexadecyl group, a 2-tetradecylhexadecyl group, a 3-decylpentadecyl group, a 3-dodecylheptadecyl group, a 3-tetradecylnonacosyl group, a 4-decylhexadecyl group, a 4-dodecyloctadecyl group, and a 4-tetradecylicosyl group; and cyclic alkyl groups such as a cyclopentyl group and a cyclohexyl group.

As the alkyl group represented by R^{A} and R^{B}, from the viewpoint of improving solubility of the conjugated polymer, an alkyl group having 1 to 34 carbon atoms is preferable, an alkyl group having 1 to 20 carbon atoms is more preferable, and a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 3,7-dimethyloctyl group, a 2-hexyloctyl group, a 2-hexyldecyl group, or a 2-octyldodecyl group is further preferable, and a decyl group or a hexadecyl group is especially preferable.

The alkoxy group having 1 to 50 carbon atoms represented by R^{A} and R^{B} may be either linear or branched, and examples thereof include linear alkoxy groups such as a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, a henicosyloxy group, a docosyloxy group, a tricosyloxy group, a tetracosyloxy group, a pentacosyloxy group, a hexacosyloxy group, a heptacosyloxy group, an octacosyloxy group, a nonacosyloxy group, a triacontyloxy group, a hentriacontyloxy group, a dotriacontyloxy group, a tritriacontyloxy group, a tetratriacontyloxy group, a pentatriacontyloxy group, a hexatriacontyloxy group, a tetracontyloxy group, a hentetracontyloxy group, a dotetracontyloxy group, a tritetracontyloxy group, a tetratetracontyloxy group, and a pentacontyloxy group; and branched alkoxy groups such as an isopropyloxy group, a 1-(2-methylpropyl)oxy group, a 2-butyloxy group, a tert-butoxy group, a 2-ethylhexyloxy group, a 3,7-dimethyloctyloxy group, a 2-decyltetradecyloxy group, a 2-dodecyltetradecyloxy group, a 2-dodecylhexadecyloxy group, and a 2-tetradecylhexadecyloxy group. From the viewpoint of increasing carrier mobility of the organic transistor, an alkoxy group having 1 to 34 carbon atoms is preferable, and an alkoxy group having 1 to 20 carbon atoms is more preferable, and a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, an isopropyloxy group, a 1-(2-methylpropyl)oxy group, a 2-butyloxy group, a tert-butoxy group, a 2-ethylhexyloxy group, or a 3,7-dimethyloctyloxy group is further preferable, and a methoxy group is especially preferable.

R^{A} and R^{B} may be bonded to each other to form a ring together with a carbon atom to which R^{A} and R^{B} are bonded. Examples of such a ring include a cyclopropane-1,1-diyl group, a cyclobutane-1,1-diyl group, a cyclopentane-1,1-diyl group, a cyclohexane-1,1-diyl group, a cycloheptane-1,1-diyl group, a cyclooctane-1,1-diyl group, an indene-1,1-diyl group, a fluorene-9,9-diyl group, and the like. From the viewpoint of improving solubility of the conjugated polymer, such a ring is preferably a cyclopentane-1,1-diyl group, a cyclohexane-1,1-diyl group, an indene-1,1-diyl group, or a fluorene-9,9-diyl group, and more preferably a cyclohexane-1,1-diyl group or a fluorene-9,9-diyl group.

Among these, for R^{A} and R^{B}, it is preferable that either one is hydrogen, and it is particularly preferable that both are hydrogen.

Further, the surfaces of these insulating layers can be modified with, for example, silanes such as octadecyltrichlorosilane, decyltrichlorosilane, decyltrimethoxysilane, octyltrichlorosilane, octadecyltrimethoxysilane, β-phenethyltrichlorosilane, β-phenethyltrimethoxysilane, phenyltrichlorosilane, phenyltrimethoxysilane, and phenyltriethoxysilane; and silylamines such as hexamethyldisilazane, and still be used.

The insulating film (the first gate and/or second gate insulating film) may contain an additive in order to adjust a surface energy. Examples of the additive include a silicone-based surfactant, a fluorine-based surfactant, and a hydrocarbon-based surfactant, among which a silicone-based surfactant is preferable.

The silicone-based surfactant is not particularly limited, and examples thereof include polydimethylsiloxane, polymethylphenylsiloxane, polyether-modified polydimethylsiloxane, polyether ester-modified polydimethylsiloxane, hydroxyl group-containing polyether-modified polydimethylsiloxane, acrylic group-containing polyether-modified polydimethylsiloxane, acrylic group-containing polyester-modified polydimethylsiloxane, perfluoropolyether-modified polydimethylsiloxane, perfluoropolyester-modified polydimethylsiloxane, polyether-modified polymethylphenylsiloxane, polyether ester-modified polymethylphenylsiloxane, hydroxyl group-containing polyether-modified polymethylphenylsiloxane, acrylic group-containing polyether-modified polymethylphenylsiloxane, acrylic group-containing polyester-modified polymethylphenylsiloxane, perfluoropolyether-modified polymethylphenylsiloxane, perfluoropolyester-modified polymethylphenylsiloxane, silicone-modified acrylic compounds, and the like, and polydimethylsiloxane and polymethylphenylsiloxane are preferable, and polymethylphenylsiloxane is more preferable.

Examples of the fluorine-based surfactant include Surflon S-111, S-112, S-113, S-121, S-131, S-132, S-141, and S-145 (all manufactured by Asahi Glass Co., Ltd.); Fluorad FC-93, FC-95, FC-98, FC-129, FC-135, FC-170C, FC-430, FC-431, and FC-4430 (all manufactured by Sumitomo 3M Ltd.); MEGAFAC F-470, F-1405, and F-474 (all manufactured by Dainippon Ink and Chemicals, Inc.); Zonyl FS-300, FSN, FSN-100, and FSO (all manufactured by DuPont); and Eftop EF-351, EF-352, EF-801, and EF-802 (all manufactured by Jemco). Among these, Zonyl FS-300, FSN, FSN-100, and FSO (all manufactured by DuPont), which are favorable in terms of reliability and improvement in color development, are particularly suitable.

Examples of the silicone-based surfactant include modified silicones KF-351A, KF-353A, KF354L, KF355A, KF-615A, KF-640, KF-642, KF-643, and KF-6011 (all manufactured by Shin-Etsu Chemical Co., Ltd.); and silicones FZ-77, FZ-2104, FZ-2105, and L-7604 (all manufactured by Dow Corning Toray Co., Ltd.). Among these, KF-355A, KF-640, KF-642, and Kf-643 (all manufactured by Shin-Etsu Chemical Co., Ltd.), which are favorable in terms of reliability and improvement in color development, are particularly suitable.

### (Organic semiconductor film)

The organic semiconductor film used in the organic transistor in accordance with an aspect of the present invention contains a conjugated polymer including a structural unit represented by general formula (2) below and a structural unit represented by general formula (3) below. 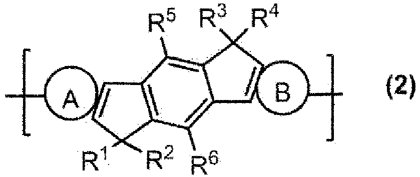 where A and B each independently represent a monovalent aromatic ring linking group which may be substituted with an alkyl group having 1 to 50 carbon atoms or an alkoxy group having 1 to 50 carbon atoms, and R¹, R², R³, and R4 each independently represent an alkyl group having 1 to 50 carbon atoms, R¹ and R² may be bonded to each other to form a ring together with a carbon atom to which R¹ and R² are bonded, R³ and R⁴ may be bonded to each other to form a ring together with a carbon atom to which R³ and R⁴ are bonded, and R⁵ and R⁶ each independently represent a hydrogen atom, a fluorine atom, or an alkyl group having 1 to 50 carbon atoms. 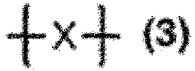 where X represents a divalent heteroaromatic ring linking group which may be substituted with an alkyl group having 1 to 50 carbon atoms or an alkoxy group having 1 to 50 carbon atoms.

The alkyl group having 1 to 50 carbon atoms represented by R¹, R², R³, R⁴, R⁵, and R⁶ may be linear, branched, or cyclic, and examples thereof include linear alkyl groups such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a henicosyl group, a docosyl group, a tricosyl group, a tetracosyl group, a pentacosyl group, a hexacosyl group, a heptacosyl group, an octacosyl group, a nonacosyl group, a triacontyl group, a hentriacontyl group, a dotriacontyl group, a tritriacontyl group, a tetratriacontyl group, a pentatriacontyl group, a hexatriacontyl group, a tetracontyl group, a hentetracontyl group, a dotetracontyl group, a tritetracontyl group, a tetratetracontyl group, and a pentacontyl group; branched alkyl groups such as an isopropyl group, an isobutyl group, a sec-butyl group, a tert-butyl group, a 2-ethylhexyl group, a 3,7-dimethyloctyl group, a 2-hexyloctyl group, a 2-hexyldecyl group, a 2-octyldodecyl group, a 2-decyltetradecyl group, a 2-dodecyltetradecyl group, a 2-dodecylhexadecyl group, a 2-tetradecylhexadecyl group, a 3-decylpentadecyl group, a 3-dodecylheptadecyl group, a 3-tetradecylnonacosyl group, a 4-decylhexadecyl group, a 4-dodecyloctadecyl group, and a 4-tetradecylicosyl group; and cyclic alkyl groups such as a cyclopentyl group and a cyclohexyl group.

As the alkyl group represented by R¹, R², R³ and R⁴, from the viewpoint of improving solubility of the conjugated polymer, an alkyl group having 1 to 34 carbon atoms is preferable, an alkyl group having 1 to 20 carbon atoms is more preferable, and a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 3,7-dimethyloctyl group, a 2-hexyloctyl group, a 2-hexyldecyl group, or a 2-octyldodecyl group is further preferable, and a decyl group or a hexadecyl group is especially preferable.

R¹ and R² may be bonded to each other to form a ring together with a carbon atom to which R¹ and R² are bonded. Examples of such a ring include a cyclopropane-1,1-diyl group, a cyclobutane-1,1-diyl group, a cyclopentane-1,1-diyl group, a cyclohexane-1,1-diyl group, a cycloheptane-1,1-diyl group, a cyclooctane-1,1-diyl group, an indene-1,1-diyl group, a fluorene-9,9-diyl group, and the like. From the viewpoint of improving solubility of the conjugated polymer, such a ring is preferably a cyclopentane-1,1-diyl group, a cyclohexane-1,1-diyl group, an indene-1,1-diyl group, or a fluorene-9,9-diyl group, and more preferably a cyclohexane-1,1-diyl group or a fluorene-9,9-diyl group.

R³ and R⁴ may be bonded to each other to form a ring together with a carbon atom to which R³ and R⁴ are bonded. Examples of such a ring include a cyclopropane-1,1-diyl group, a cyclobutane-1,1-diyl group, a cyclopentane-1,1-diyl group, a cyclohexane-1,1-diyl group, a cycloheptane-1,1-diyl group, a cyclooctane-1,1-diyl group, an indene-1,1-diyl group, a fluorene-9,9-diyl group, and the like. From the viewpoint of improving solubility of the conjugated polymer, such a ring is preferably a cyclopentane-1,1-diyl group, a cyclohexane-1,1-diyl group, an indene-1,1-diyl group, or a fluorene-9,9-diyl group, and more preferably a cyclohexane-1,1-diyl group or a fluorene-9,9-diyl group.

From the viewpoint of increasing carrier mobility of the organic transistor, R⁵ and R⁶ are preferably a hydrogen atom, a fluorine atom, or an alkyl group having 1 to 34 carbon atoms, more preferably a hydrogen atom, a fluorine atom, or an alkyl group having 1 to 20 carbon atoms, and further preferably a hydrogen atom, a fluorine atom, a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 3,7-dimethyloctyl group, a 2-hexyloctyl group, a 2-hexyldecyl group, or a 2-octyldodecyl group. Among these, from the viewpoint of increasing carrier mobility, R⁵ and R⁶ are each especially preferably a hydrogen atom.

Examples of the monovalent aromatic ring linking group represented by A and B include the following structures. 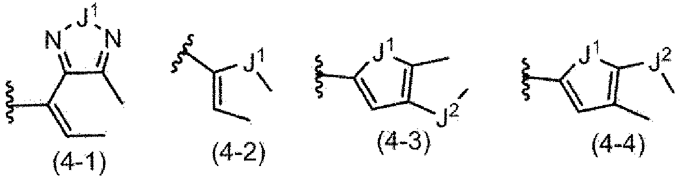 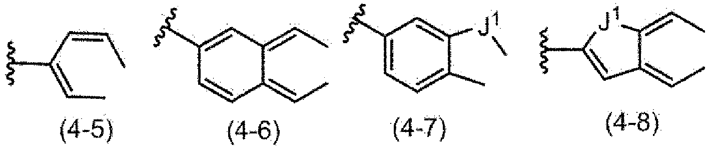 where J¹ and J² each independently represent a chalcogen atom. As the chalcogen atom represented by J¹ and J², from the viewpoint of increasing carrier mobility of the compound and the conjugated polymer in accordance with the present embodiment, an oxygen atom, a sulfur atom, and a selenium atom are preferable, an oxygen atom and a sulfur atom are more preferable, and a sulfur atom is further preferable.

As the monovalent aromatic ring linking group represented by A and B, from the viewpoint of increasing carrier mobility of the organic transistor, either one or both of A and B are preferably a linking group represented by general formulae (4-1) through (4-8), more preferably a linking group represented by general formulae (4-1) and (4-2), and especially preferably a linking group represented by general formula (4-1). In this case, A and B may be the same or different. 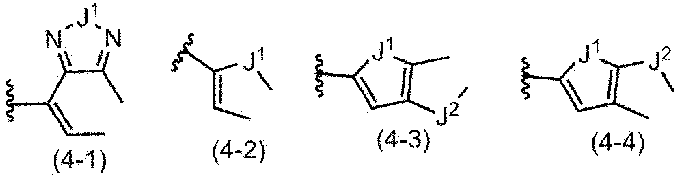 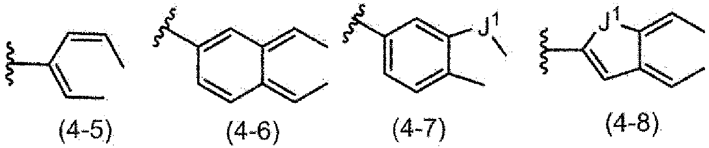 where J¹ and J² each independently represent a chalcogen atom.

The alkyl group that has 1 to 50 carbon atoms and that may be a substituent of A and B may be linear, branched, or cyclic, and examples thereof include linear alkyl groups such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a henicosyl group, a docosyl group, a tricosyl group, a tetracosyl group, a pentacosyl group, a hexacosyl group, a heptacosyl group, an octacosyl group, a nonacosyl group, a triacontyl group, a hentriacontyl group, a dotriacontyl group, a tritriacontyl group, a tetratriacontyl group, a pentatriacontyl group, a hexatriacontyl group, a tetracontyl group, a hentetracontyl group, a dotetracontyl group, a tritetracontyl group, a tetratetracontyl group, and a pentacontyl group; branched alkyl groups such as an isopropyl group, an isobutyl group, a sec-butyl group, a tert-butyl group, a 2-ethylhexyl group, a 3,7-dimethyloctyl group, a 2-hexyloctyl group, a 2-hexyldecyl group, a 2-octyldodecyl group, a 2-decyltetradecyl group, a 2-dodecyltetradecyl group, a 2-dodecylhexadecyl group, a 2-tetradecylhexadecyl group, a 3-decylpentadecyl group, a 3-dodecylheptadecyl group, a 3-tetradecylnonacosyl group, a 4-decylhexadecyl group, a 4-dodecyloctadecyl group, and a 4-tetradecylicosyl group; and cyclic alkyl groups such as a cyclopentyl group and a cyclohexyl group. From the viewpoint of improving solubility of the conjugated polymer, an alkyl group having 1 to 34 carbon atoms is preferable, an alkyl group having 1 to 20 carbon atoms is more preferable, and a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 3,7-dimethyloctyl group, a 2-hexyloctyl group, a 2-hexyldecyl group, or a 2-octyldodecyl group is further preferable, and a decyl group or a hexadecyl group is especially preferable.

The alkoxy group that has 1 to 50 carbon atoms and that may be a substituent of A and B may be either linear or branched, and examples thereof include linear alkoxy groups such as a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, a henicosyloxy group, a docosyloxy group, a tricosyloxy group, a tetracosyloxy group, a pentacosyloxy group, a hexacosyloxy group, a heptacosyloxy group, an octacosyloxy group, a nonacosyloxy group, a triacontyloxy group, a hentriacontyloxy group, a dotriacontyloxy group, a tritriacontyloxy group, a tetratriacontyloxy group, a pentatriacontyloxy group, a hexatriacontyloxy group, a tetracontyloxy group, a hentetracontyloxy group, a dotetracontyloxy group, a tritetracontyloxy group, a tetratetracontyloxy group, and a pentacontyloxy group; and branched alkoxy groups such as an isopropyloxy group, a 1-(2-methylpropyl)oxy group, a 2-butyloxy group, a tert-butoxy group, a 2-ethylhexyloxy group, a 3,7-dimethyloctyloxy group, a 2-decyltetradecyloxy group, a 2-dodecyltetradecyloxy group, a 2-dodecylhexadecyloxy group, and a 2-tetradecylhexadecyloxy group. From the viewpoint of increasing carrier mobility of the organic transistor, an alkoxy group having 1 to 34 carbon atoms is preferable, and an alkoxy group having 1 to 20 carbon atoms is more preferable, and a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, an isopropyloxy group, a 1-(2-methylpropyl)oxy group, a 2-butyloxy group, a tert-butoxy group, a 2-ethylhexyloxy group, or a 3,7-dimethyloctyloxy group is further preferable, and a methoxy group is especially preferable.

As the structural unit represented by general formula (2), a structural unit represented by general formula (2-1) below is preferable from the viewpoint of high carrier mobility.

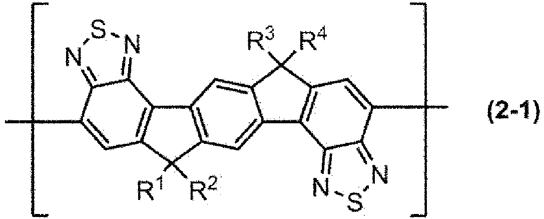

R¹ to R⁴ have the same meanings as described above.

From the viewpoint of improving film formability of the conjugated polymer and increasing carrier mobility of the organic transistor, examples of the structural unit represented by general formula (2) can be structural units shown below and the like. Note, however, that the present invention is not limited to those examples. In the formulae, n represents a natural number of 1 to 50, which may be the same or different. x represents a natural number of 2 to 24, which may be the same or different. y represents a natural number of 1 to 24, which may be the same or different. z represents a natural number of 1 to 20, which may be the same or different. Specific examples of structural units encompassed therein are also indicated below.

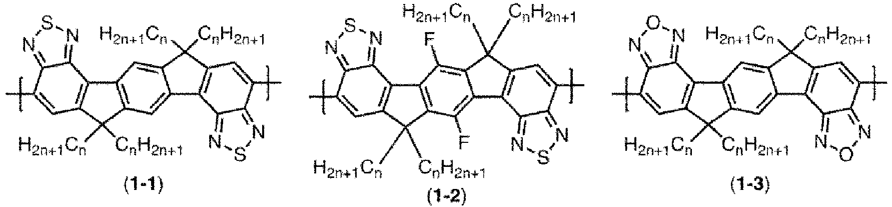

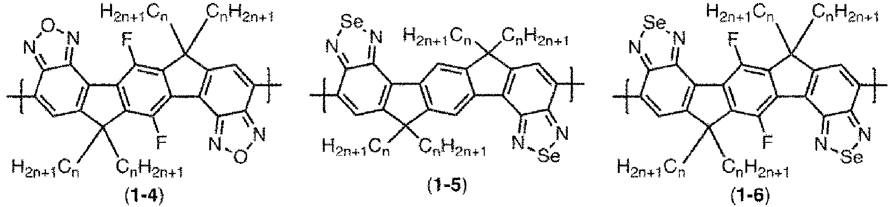

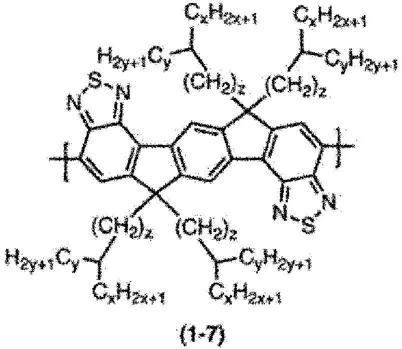

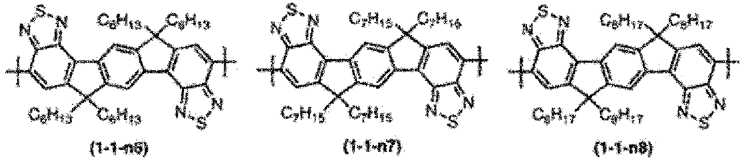

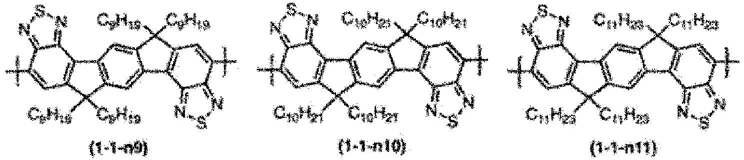

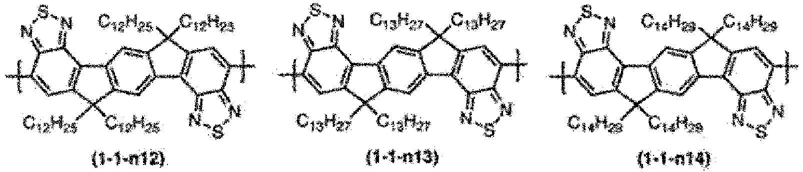

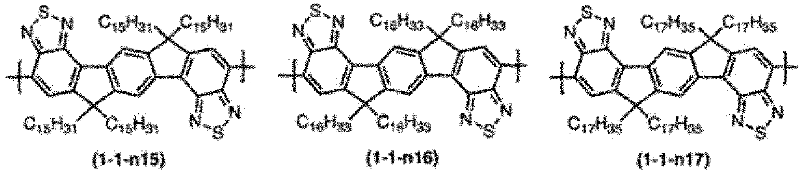

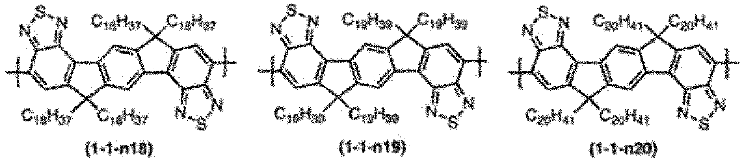

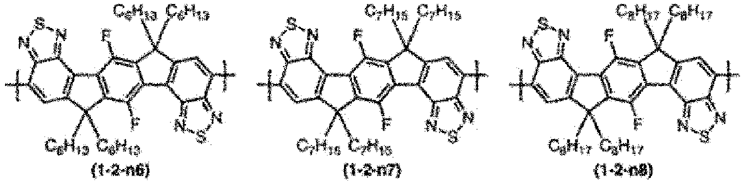

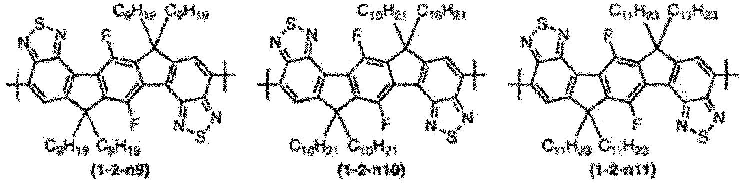

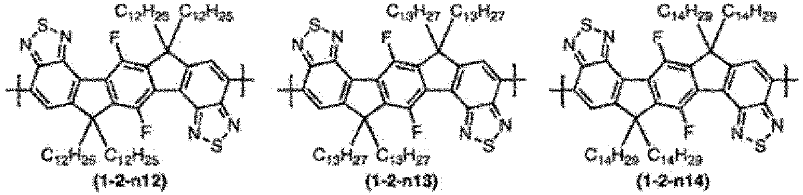

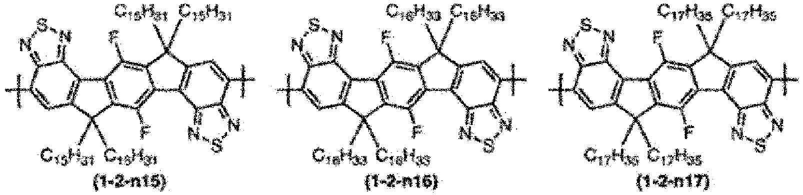

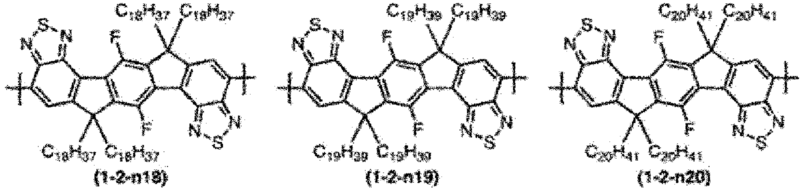

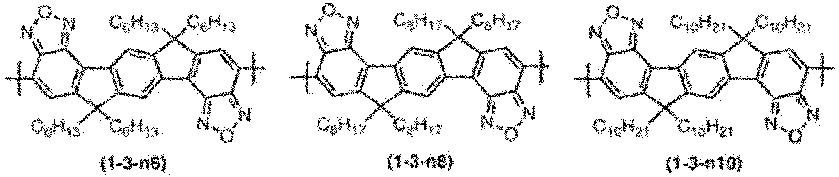

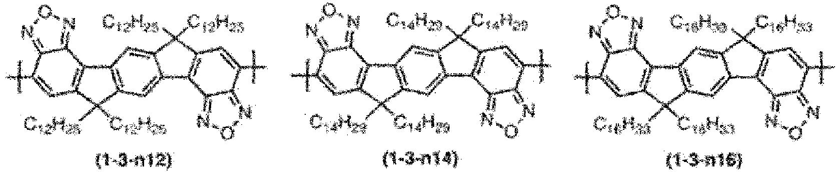

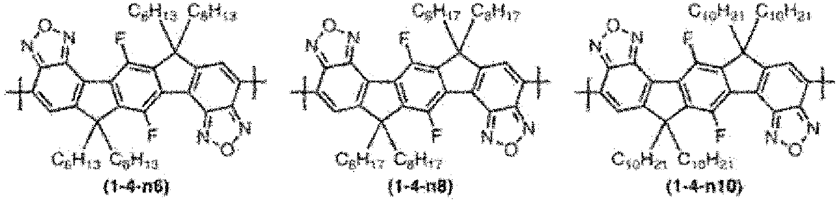

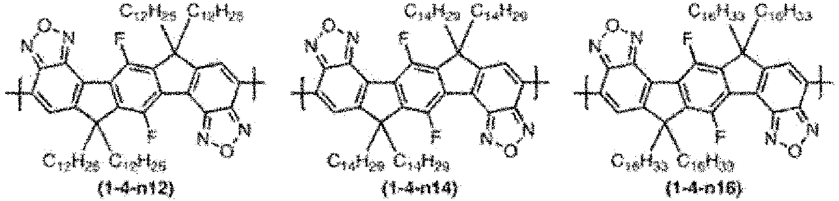

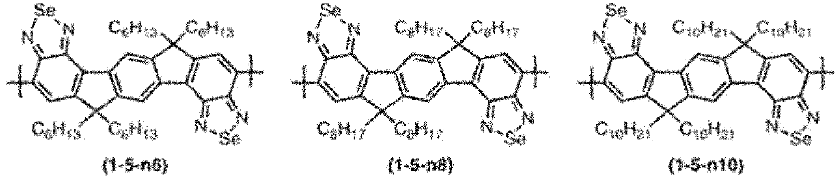

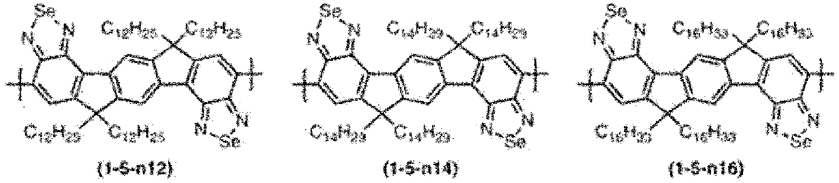

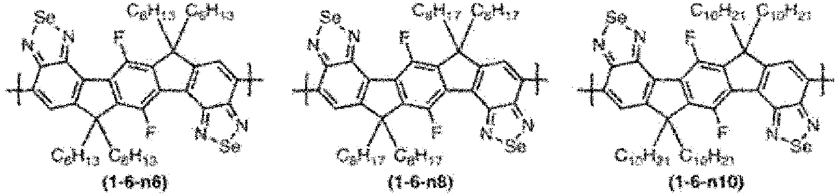

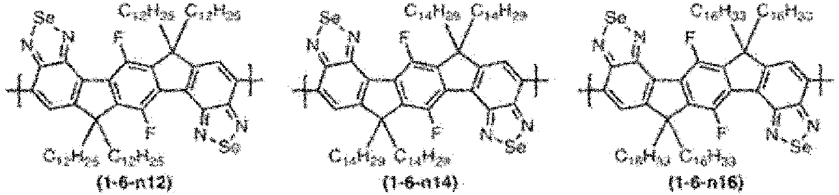

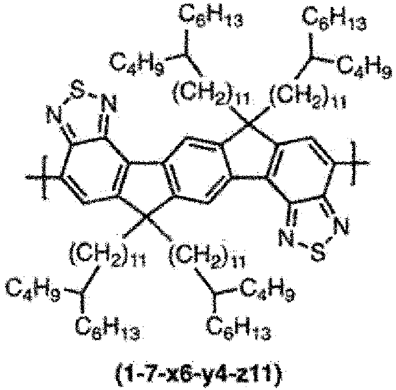

In the formulae, CₙH₂ₙ₊₁, CₓH₂ₓ₊₁, and C_{y}H_{2y+1} each represent a linear alkyl group.

From the viewpoint of improving film formability of the conjugated polymer and increasing carrier mobility of the organic transistor, the structural unit represented by general formula (2) is preferably any structural unit represented by general formula (1-1), general formula (1-2), general formula (1-1-n6) to general formula (1-1-n20), or general formula (1-2-n6) to general formula (1-2-n20), more preferably any structural unit represented by general formula (1-1) or general formula (1-1-n6) to general formula (1-1-n20), further preferably any structural unit represented by general formula (1-1-n10) to general formula (1-1-n18), and especially preferably a structural unit represented by general formula (1-1-n16).

Examples of X include a divalent heteroaromatic ring linking group which may be substituted with an alkyl group having 1 to 50 carbon atoms or an alkoxy group having 1 to 50 carbon atoms, and specific examples include a divalent heteroaromatic ring linking group selected from the group consisting of linking groups represented by general formulae (4) through (23) below. 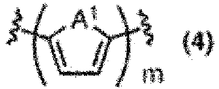 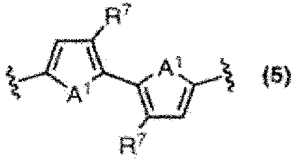 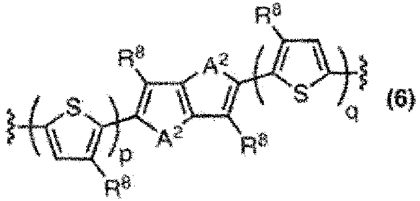 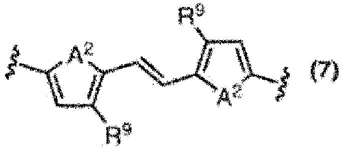 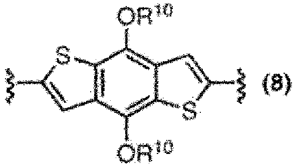 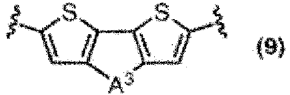 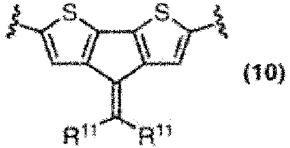 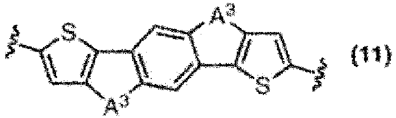 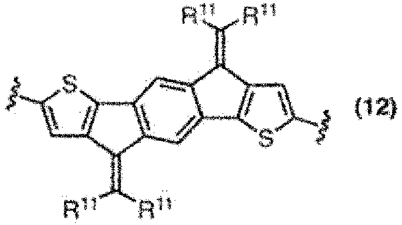 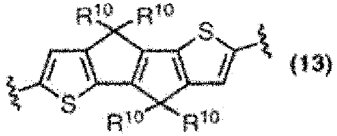 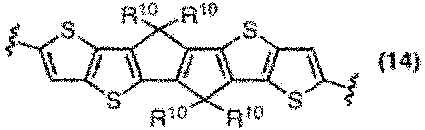 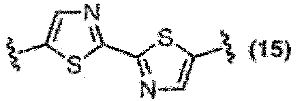 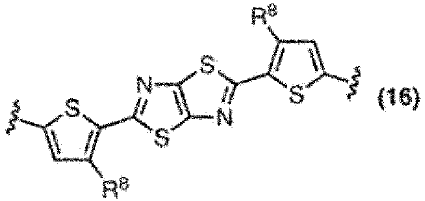 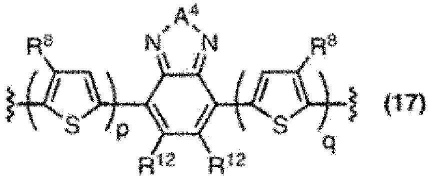 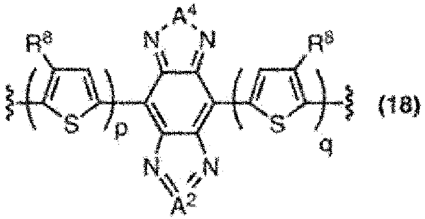 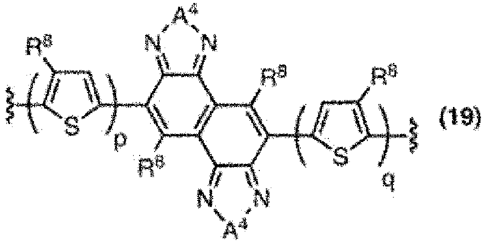 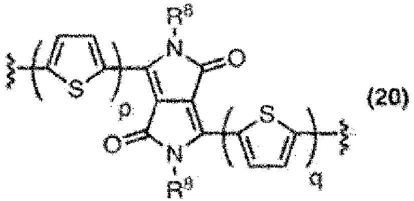 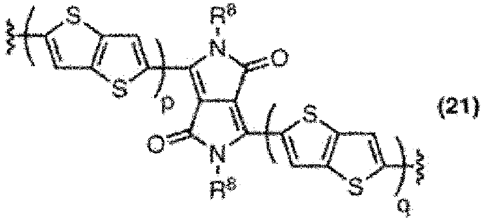 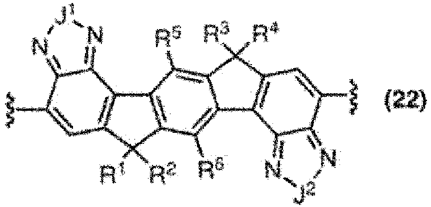 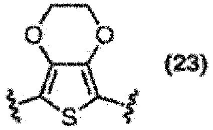 where R¹, R², R³, R⁴, R⁵, R⁶, J¹, and J² have the same meanings as described above. A¹ and A² each independently represent a chalcogen atom. Each A3 independently represents a chalcogen atom, C(R¹⁰)₂, C(H)(R¹⁰), Si(R¹⁰)₂, or NR¹⁰. Each A⁴ independently represents a chalcogen atom or NR¹⁰. R⁷ represents a hydrogen atom, a fluorine atom, or an alkyl group having 1 to 50 carbon atoms. The plurality of R⁷ may be the same or different. R⁸ represents a hydrogen atom or an alkyl group having 1 to 50 carbon atoms. The plurality of R⁸ may be the same or different. R⁹ represents a hydrogen atom, a fluorine atom, or an alkyl group having 1 to 50 carbon atoms. The plurality of R⁹ may be the same or different. R¹⁰ represents an alkyl group having 1 to 50 carbon atoms. The plurality of R¹⁰ may be the same or different. R¹¹ represents an alkyl group having 1 to 50 carbon atoms or a thioalkyl group having 1 to 50 carbon atoms. R¹² represents a hydrogen atom, a fluorine atom, an alkyl group having 1 to 50 carbon atoms, or an alkoxy group having 1 to 50 carbon atoms. m represents 1 to 3. p and q each independently represent 0 or 1.

From the viewpoint of improving film formability of the conjugated polymer and increasing carrier mobility of the organic transistor, the structural unit represented by general formula (3) is preferably a structural unit represented by general formula (4) to general formula (14) or general formula (20), more preferably a structural unit represented by general formula (5) to general formula (11) or general formula (20), and especially preferably a structural unit represented by general formula (6).

The alkyl group having 1 to 50 carbon atoms represented by R⁷, R⁸, R⁹, R¹⁰, R¹¹, and R¹² may be linear or branched, and examples thereof include linear alkyl groups such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a henicosyl group, a docosyl group, a tricosyl group, a tetracosyl group, a pentacosyl group, a hexacosyl group, a heptacosyl group, an octacosyl group, a nonacosyl group, a triacontyl group, a hentriacontyl group, a dotriacontyl group, a tritriacontyl group, a tetratriacontyl group, a pentatriacontyl group, a hexatriacontyl group, a tetracontyl group, a hentetracontyl group, a dotetracontyl group, a tritetracontyl group, a tetratetracontyl group, and a pentacontyl group; and branched alkyl groups such as an isopropyl group, an isobutyl group, a sec-butyl group, a tert-butyl group, a 2-ethylhexyl group, a 3,7-dimethyloctyl group, a 2-hexyloctyl group, a 2-hexyldecyl group, a 2-octyldodecyl group, a 2-decyltetradecyl group, a 2-dodecyltetradecyl group, a 2-dodecylhexadecyl group, a 2-tetradecylhexadecyl group, a 3-decylpentadecyl group, a 3-dodecylheptadecyl group, a 3-tetradecylnonacosyl group, a 4-decylhexadecyl group, a 4-dodecyloctadecyl group, and a 4-tetradecylicosyl group.

The alkoxy group having 1 to 50 carbon atoms represented by R⁷, R⁸, R⁹, R¹⁰, R¹¹, and R¹² may be either linear or branched, and examples thereof include linear alkoxy groups such as a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, a henicosyloxy group, a docosyloxy group, a tricosyloxy group, a tetracosyloxy group, a pentacosyloxy group, a hexacosyloxy group, a heptacosyloxy group, an octacosyloxy group, a nonacosyloxy group, a triacontyloxy group, a hentriacontyloxy group, a dotriacontyloxy group, a tritriacontyloxy group, a tetratriacontyloxy group, a pentatriacontyloxy group, a hexatriacontyloxy group, a tetracontyloxy group, a hentetracontyloxy group, a dotetracontyloxy group, a tritetracontyloxy group, a tetratetracontyloxy group, and a pentacontyloxy group; and branched alkoxy groups such as an isopropyloxy group, a 1-(2-methylpropyl)oxy group, a 2-butyloxy group, a tert-butoxy group, a 2-ethylhexyloxy group, a 3,7-dimethyloctyloxy group, a 2-decyltetradecyloxy group, a 2-dodecyltetradecyloxy group, a 2-dodecylhexadecyloxy group, and a 2-tetradecylhexadecyloxy group. From the viewpoint of increasing carrier mobility of the organic transistor, an alkoxy group having 1 to 34 carbon atoms is preferable, and an alkoxy group having 1 to 20 carbon atoms is more preferable, and a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, an isopropyloxy group, a 1-(2-methylpropyl)oxy group, a 2-butyloxy group, a tert-butoxy group, a 2-ethylhexyloxy group, or a 3,7-dimethyloctyloxy group is further preferable, and a methoxy group is especially preferable.

From the viewpoint of increasing carrier mobility of the organic transistor, R⁷ is preferably a hydrogen atom, a fluorine atom, or an alkyl group having 1 to 34 carbon atoms, more preferably a hydrogen atom, a fluorine atom, or an alkyl group having 1 to 20 carbon atoms, further preferably a hydrogen atom, a fluorine atom, or a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group, or a 2-octyldodecyl group, especially preferably a hydrogen atom, a fluorine atom, or a methyl group, an octyl group, or a 2-octyldodecyl group, and particularly preferably a hydrogen atom.

From the viewpoint of increasing carrier mobility of the organic transistor, R⁸ is preferably a hydrogen atom or an alkyl group having 1 to 34 carbon atoms, more preferably a hydrogen atom or an alkyl group having 1 to 20 carbon atoms, further preferably a hydrogen atom, a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group, or a 2-octyldodecyl group, especially preferably a hydrogen atom, a methyl group, an octyl group, or a 2-octyldodecyl group, and particularly preferably a hydrogen atom.

From the viewpoint of increasing carrier mobility of the organic transistor, R⁹ is preferably a hydrogen atom, a fluorine atom, or an alkyl group having 1 to 34 carbon atoms, more preferably a hydrogen atom, a fluorine atom, or an alkyl group having 1 to 20 carbon atoms, further preferably a hydrogen atom, a fluorine atom, a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group, or a 2-octyldodecyl group, especially preferably a hydrogen atom, a fluorine atom, a methyl group, or a hexyl group, and particularly preferably a hydrogen atom.

From the viewpoint of increasing carrier mobility of the organic transistor, R¹⁰ is preferably an alkyl group having 1 to 34 carbon atoms, more preferably an alkyl group having 1 to 20 carbon atoms, further preferably a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group, or a 2-octyldodecyl group, and especially preferably a methyl group or an octyl group.

As the alkyl group represented by R¹¹, from the viewpoint of increasing carrier mobility of the organic transistor, an alkyl group having 1 to 34 carbon atoms is preferable, an alkyl group having 1 to 20 carbon atoms is more preferable, a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group, or a 2-octyldodecyl group is further preferable, and a methyl group, a propyl group, or a hexyl group is especially preferable.

Examples of the thioalkyl group having 1 to 50 carbon atoms represented by R¹¹ include a methylthio group, an ethylthio group, a propylthio group, a butylthio group, a pentylthio group, a hexylthio group, a heptylthio group, an octylthio group, a nonylthio group, a decylthio group, an undecylthio group, a dodecylthio group, a tridecylthio group, a tetradecylthio group, a pentadecylthio group, a hexadecylthio group, a heptadecylthio group, an octadecylthio group, a nonadecylthio group, an icosylthio group, a 2-ethylhexylthio group, a 2-methylheptylthio group, a 2-propylhexylthio group, a 2-ethylheptylthio group, a 2-methyloctylthio group, a 2-butylhexylthio group, a 2-ethyloctylthio group, a 2-methylnonylthio group, a 2-butylheptylthio group, a 2-propyloctylthio group, a 2-ethylnonylthio group, a 2-pentylheptylthio group, a 2-butyloctylthio group, a 2-propylnonylthio group, a 2-pentyloctylthio group, a 2-butylnonylthio group, a 2-methyldecylthio group, a 2-hexyloctylthio group, a 2-pentylnonylthio group, a 2-hexylnonylthio group, a 2-hexyldecylthio group, a 2-heptylnonylthio group, a 2-octyldodecylthio group, and the like. From the viewpoint of increasing carrier mobility of the organic transistor, a thioalkyl group having 1 to 34 carbon atoms is preferable, a thioalkyl group having 1 to 20 carbon atoms is more preferable, a methylthio group, an ethylthio group, a propylthio group, a butylthio group, a pentylthio group, a hexylthio group, a heptylthio group, an octylthio group, a nonylthio group, a decylthio group, an undecylthio group, a dodecylthio group, a tridecylthio group, a tetradecylthio group, a pentadecylthio group, a hexadecylthio group, a heptadecylthio group, an octadecylthio group, a nonadecylthio group, an icosylthio group, a 2-ethylhexylthio group, a 2-methylheptylthio group, a 2-propylhexylthio group, a 2-ethylheptylthio group, a 2-methyloctylthio group, a 2-butylhexylthio group, a 2-ethyloctylthio group, a 2-methylnonylthio group, a 2-butylheptylthio group, a 2-propyloctylthio group, a 2-ethylnonylthio group, a 2-pentylheptylthio group, a 2-butyloctylthio group, a 2-propylnonylthio group, a 2-pentyloctylthio group, a 2-butylnonylthio group, a 2-methyldecylthio group, a 2-hexyloctylthio group, a 2-pentylnonylthio group, a 2-hexylnonylthio group, a 2-hexyldecylthio group, a 2-heptylnonylthio group, or a 2-octyldodecylthio group is further preferable, and a methylthio group or a propylthio group is especially preferable.

From the viewpoint of increasing carrier mobility of the organic transistor, R¹¹ is preferably an alkyl group having 1 to 34 carbon atoms or a thioalkyl group having 1 to 34 carbon atoms, more preferably an alkyl group having 1 to 20 carbon atoms or a thioalkyl group having 1 to 20 carbon atoms, further preferably a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a methylthio group, an ethylthio group, a propylthio group, a butylthio group, a pentylthio group, a hexylthio group, a heptylthio group, an octylthio group, a nonylthio group, a decylthio group, an undecylthio group, a dodecylthio group, a tridecylthio group, a tetradecylthio group, a pentadecylthio group, a hexadecylthio group, a heptadecylthio group, an octadecylthio group, a nonadecylthio group, or an icosylthio group, and especially preferably a methyl group, a propyl group, a hexyl group, a methylthio group, or a propylthio group.

As the alkyl group having 1 to 50 carbon atoms represented by R¹², from the viewpoint of increasing carrier mobility of the organic transistor, an alkyl group having 1 to 34 carbon atoms is preferable, an alkyl group having 1 to 20 carbon atoms is more preferable, a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, an isopropyl group, an isobutyl group, a sec-butyl group, a tert-butyl group, a 2-ethylhexyl group, a 3,7-dimethyloctyl group, a 2-hexyloctyl group, a 2-hexyldecyl group, or a 2-octyldodecyl group is further preferable, and a methyl group is especially preferable.

The alkoxy group having 1 to 50 carbon atoms represented by R¹² may be either linear or branched, and examples thereof include linear alkoxy groups such as a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, a henicosyloxy group, a docosyloxy group, a tricosyloxy group, a tetracosyloxy group, a pentacosyloxy group, a hexacosyloxy group, a heptacosyloxy group, an octacosyloxy group, a nonacosyloxy group, a triacontyloxy group, a hentriacontyloxy group, a dotriacontyloxy group, a tritriacontyloxy group, a tetratriacontyloxy group, a pentatriacontyloxy group, a hexatriacontyloxy group, a tetracontyloxy group, a hentetracontyloxy group, a dotetracontyloxy group, a tritetracontyloxy group, a tetratetracontyloxy group, and a pentacontyloxy group; and branched alkoxy groups such as an isopropyloxy group, a 1-(2-methylpropyl)oxy group, a 2-butyloxy group, a tert-butoxy group, a 2-ethylhexyloxy group, a 3,7-dimethyloctyloxy group, a 2-decyltetradecyloxy group, a 2-dodecyltetradecyloxy group, a 2-dodecylhexadecyloxy group, and a 2-tetradecylhexadecyloxy group. From the viewpoint of increasing carrier mobility of the organic transistor, an alkoxy group having 1 to 34 carbon atoms is preferable, and an alkoxy group having 1 to 20 carbon atoms is more preferable, and a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, an isopropyloxy group, a 1-(2-methylpropyl)oxy group, a 2-butyloxy group, a tert-butoxy group, a 2-ethylhexyloxy group, or a 3,7-dimethyloctyloxy group is further preferable, and a methoxy group is especially preferable.

From the viewpoint of increasing carrier mobility of the organic transistor, R¹² is preferably a hydrogen atom, a fluorine atom, an alkyl group having 1 to 34 carbon atoms, or an alkoxy group having 1 to 34 carbon atoms, more preferably a hydrogen atom, a fluorine atom, an alkyl group having 1 to 20 carbon atoms, or an alkoxy group having 1 to 20 carbon atoms, further preferably a hydrogen atom, a fluorine atom, a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an icosyl group, a 2-ethylhexyl group, a 2-methylheptyl group, a 2-propylhexyl group, a 2-ethylheptyl group, a 2-methyloctyl group, a 2-butylhexyl group, a 2-ethyloctyl group, a 2-methylnonyl group, a 2-butylheptyl group, a 2-propyloctyl group, a 2-ethylnonyl group, a 2-pentylheptyl group, a 2-butyloctyl group, a 2-propylnonyl group, a 2-pentyloctyl group, a 2-butylnonyl group, a 2-methyldecyl group, a 2-hexyloctyl group, a 2-pentylnonyl group, a 2-hexylnonyl group, a 2-hexyldecyl group, a 2-heptylnonyl group, a 2-octyldodecyl group, a methoxy group, an ethoxy group, a propoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a nonadecyloxy group, an icosyloxy group, an isopropyloxy group, a 1-(2-methylpropyl)oxy group, a 2-butyloxy group, a tert-butoxy group, a 2-ethylhexyloxy group, or a 3,7-dimethyloctyloxy group is further preferable, and a hydrogen atom, a fluorine atom, a methyl group, or a methoxy group is especially preferable.

Examples of the chalcogen atom represented by A¹, A², A³, and A⁴ include an oxygen atom, a sulfur atom, and a selenium atom.

As the chalcogen atom represented by A¹, from the viewpoint of increasing carrier mobility of the organic transistor, an oxygen atom, a sulfur atom, or a selenium atom is preferable, an oxygen atom or a sulfur atom is more preferable, and a sulfur atom is further preferable.

As the chalcogen atom represented by A², from the viewpoint of increasing carrier mobility of the organic transistor, an oxygen atom, a sulfur atom, or a selenium atom is preferable, an oxygen atom or a sulfur atom is more preferable, and a sulfur atom is further preferable.

As A³, from the viewpoint of increasing carrier mobility of the organic transistor, a sulfur atom, a selenium atom, C(R¹⁰)₂, C(H)(R¹⁰), Si(R¹⁰)₂, or NR¹⁰ is preferable, C(R¹⁰)₂, C(H)(R¹⁰), Si(R¹⁰)₂, or NR¹⁰ is more preferable, and C(R¹⁰)₂ or C(H)(R¹⁰) is further preferable.

As A⁴, from the viewpoint of increasing carrier mobility of the organic transistor, an oxygen atom, a sulfur atom, a selenium atom, or NR¹⁰ is preferable, an oxygen atom or a sulfur atom is more preferable, and a sulfur atom is further preferable.
m represents 1 to 3, and from the viewpoint of improving film formability of the conjugated polymer and increasing carrier mobility of the organic transistor, 2 is preferable.
p and q each represent 0 or 1, and from the viewpoint of improving film formability of the conjugated polymer and increasing carrier mobility of the organic transistor, 0 is preferable.

From the viewpoint of increasing solubility of the conjugated polymer, more specific examples of the structural unit represented by general formula (3) can be structural units shown below. Note, however, that the present invention is not limited to those examples. In the formulae, k represents a natural number of 1 to 50, which may be the same or different. x represents a natural number of 2 to 24, which may be the same or different. y represents a natural number of 1 to 24, which may be the same or different. Specific examples of structural units encompassed therein are also indicated below.

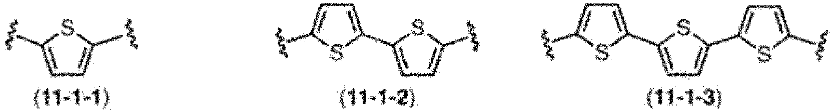

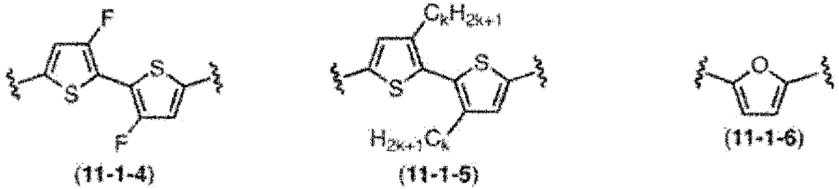

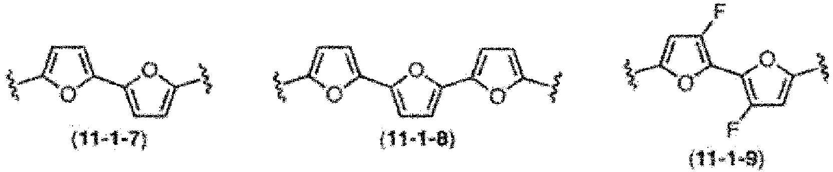

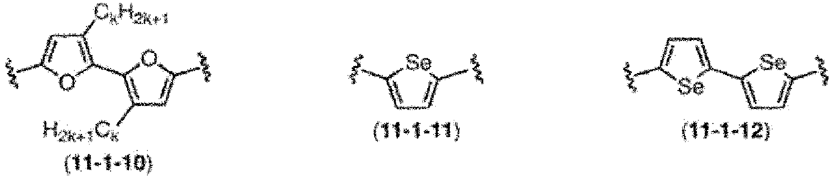

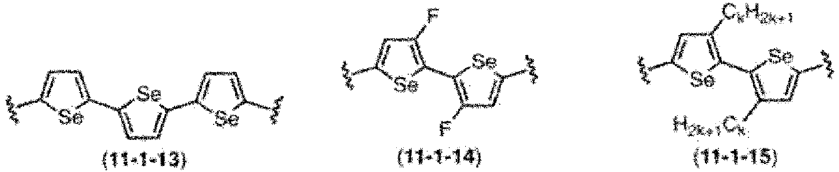

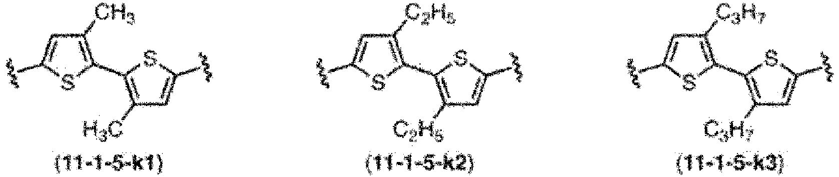

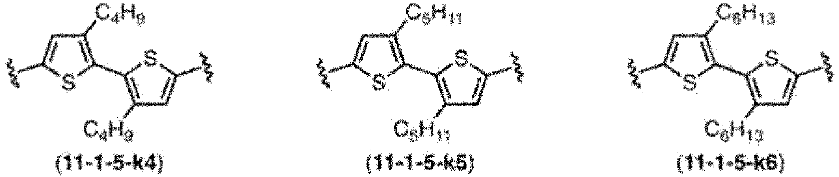

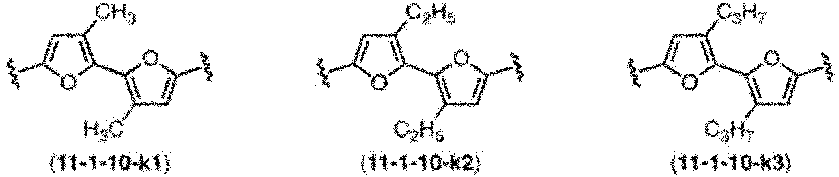

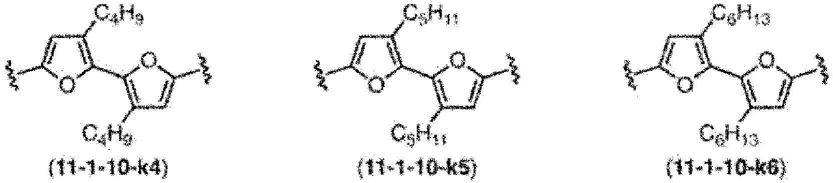

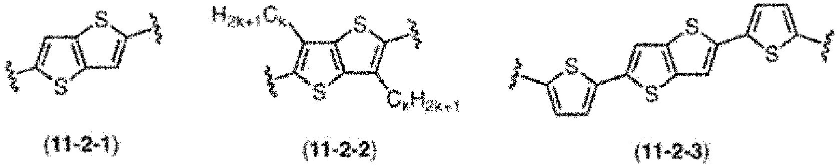

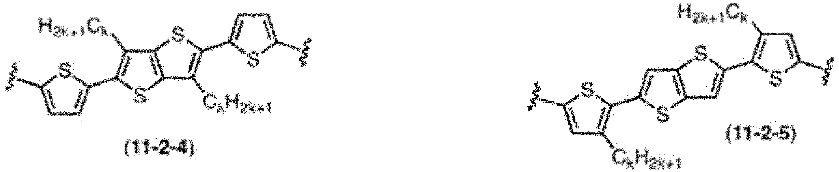

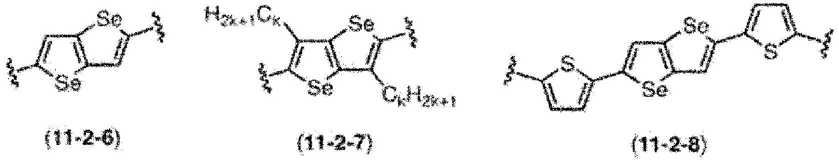

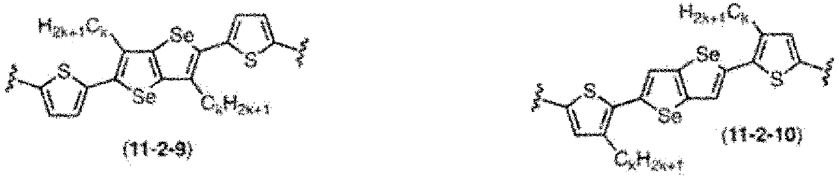

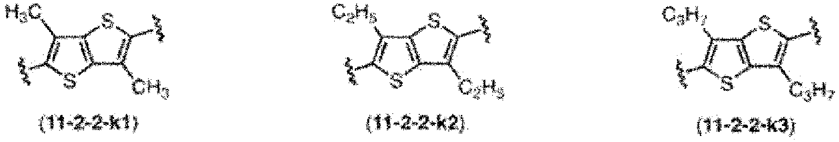

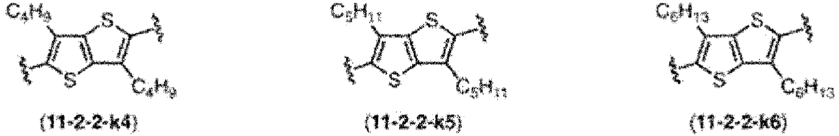

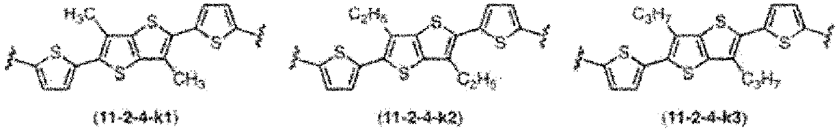

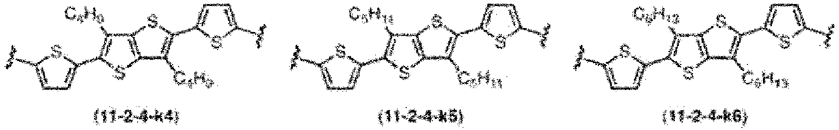

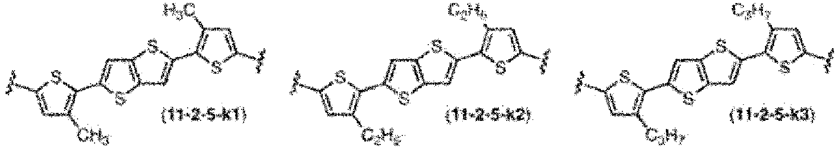

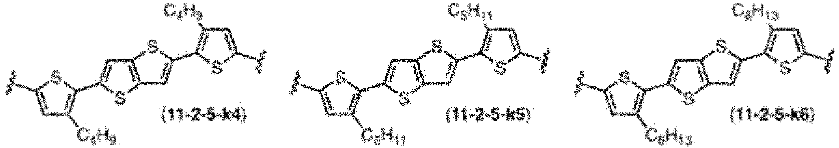

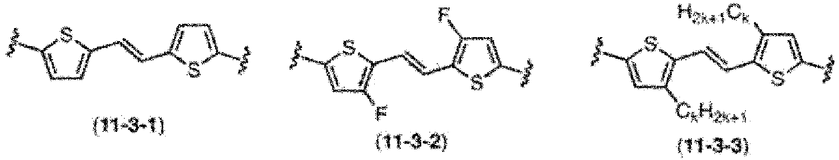

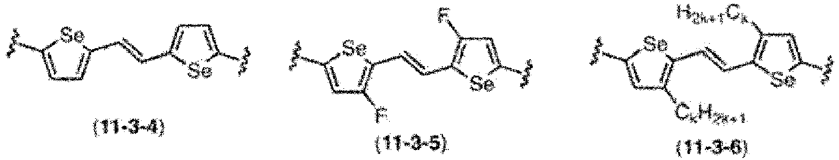

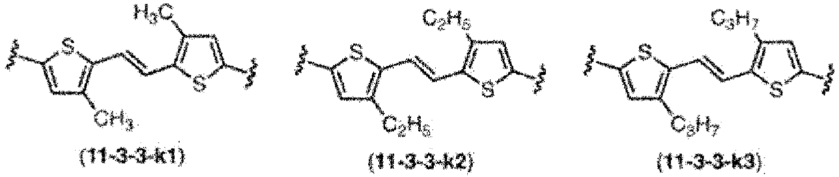

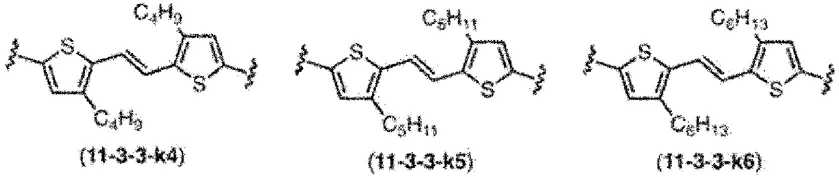

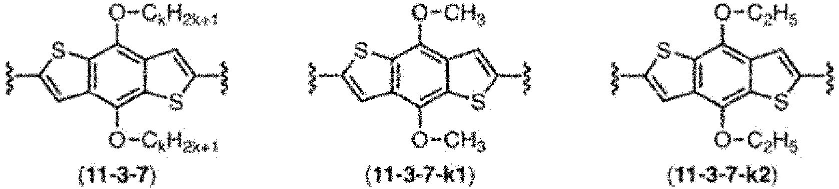

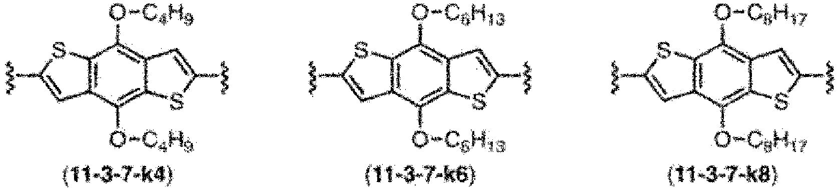

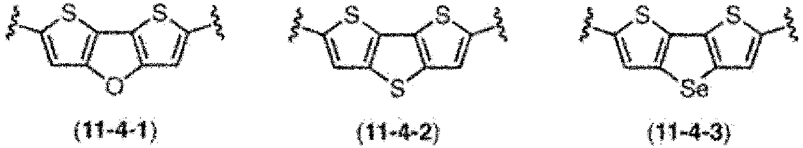

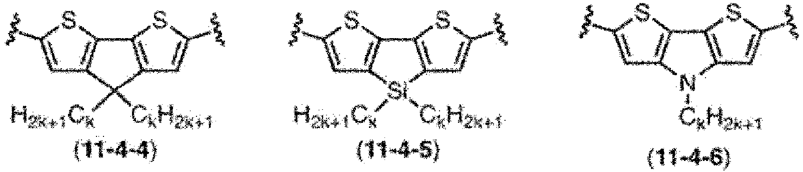

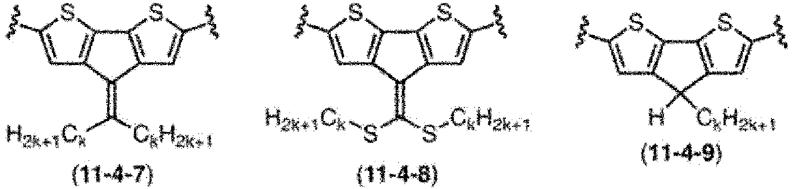

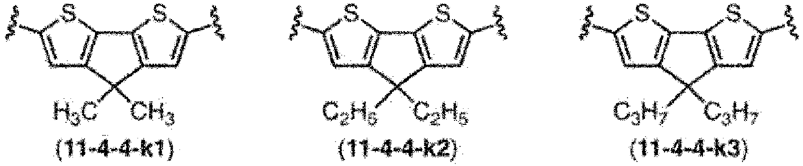

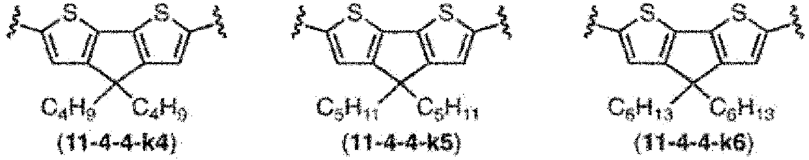

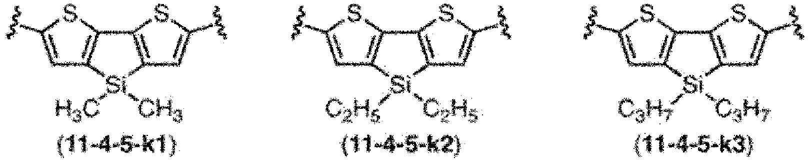

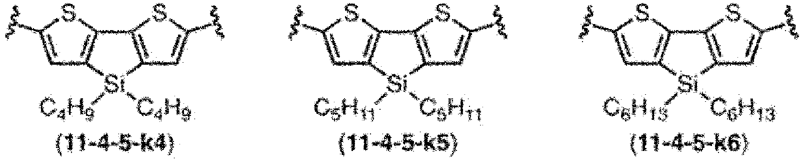

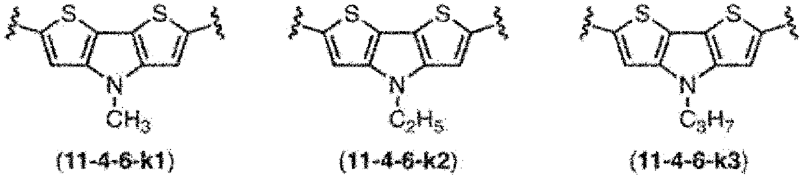

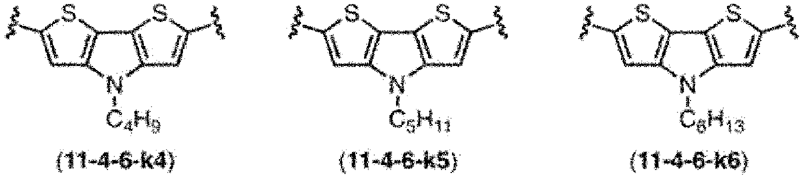

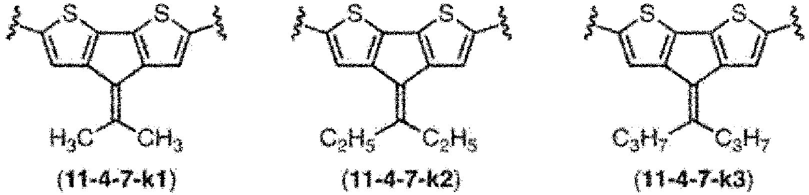

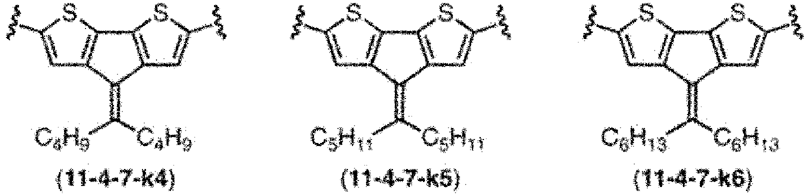

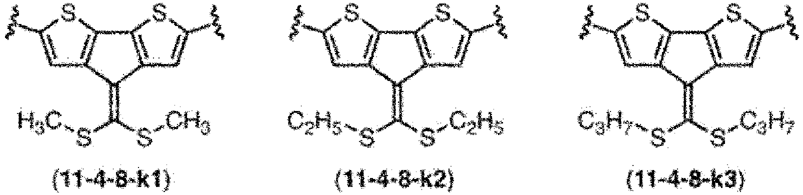

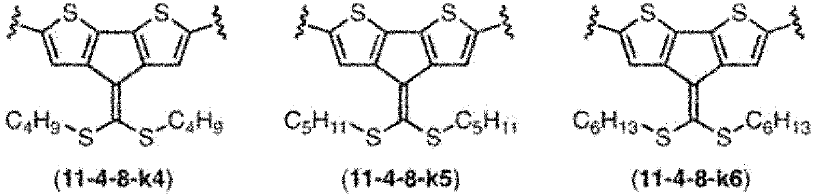

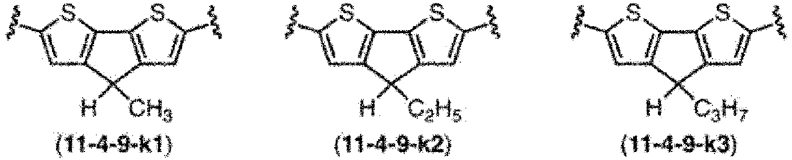

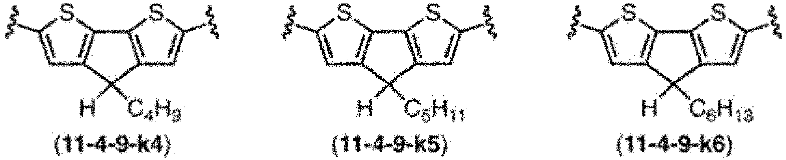

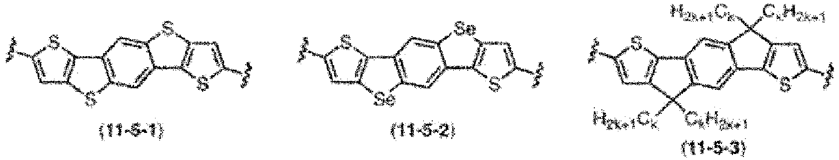

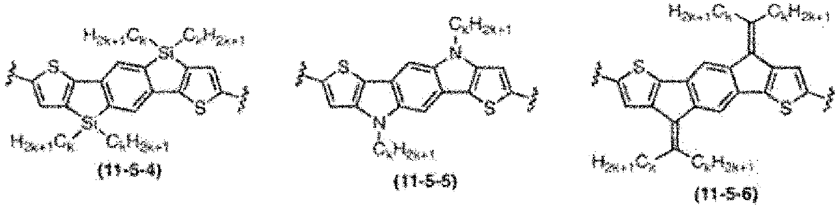

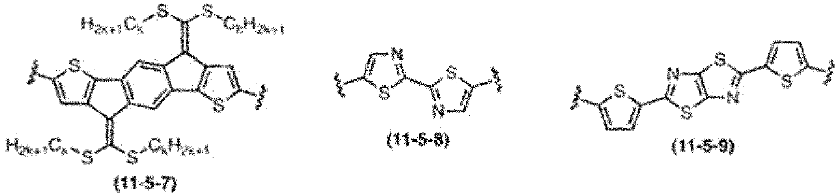

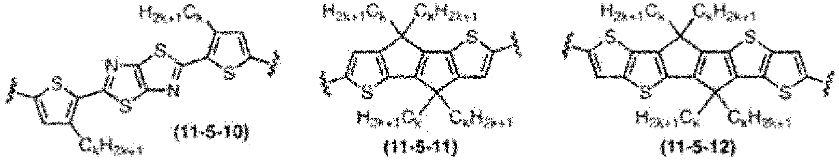

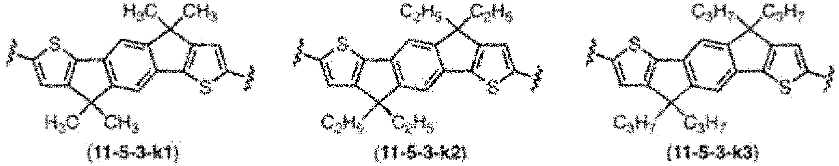

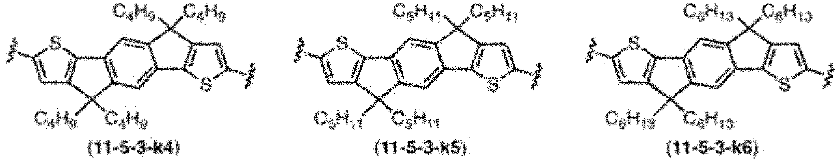

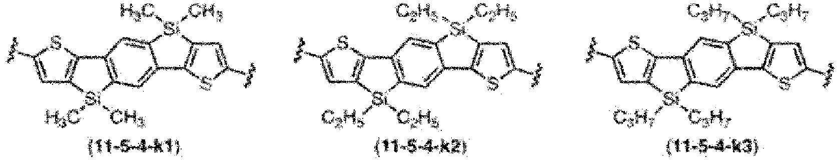

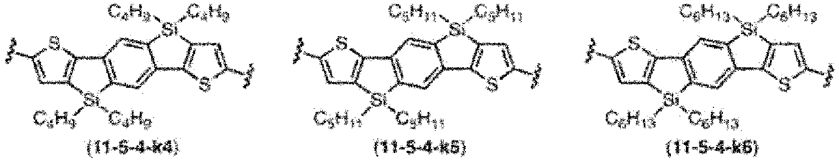

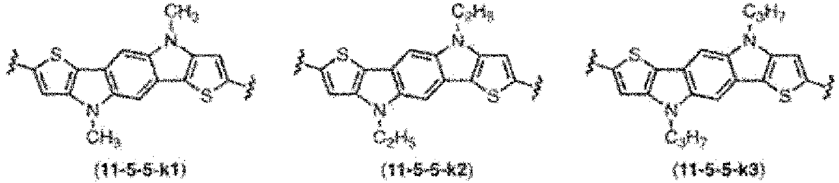

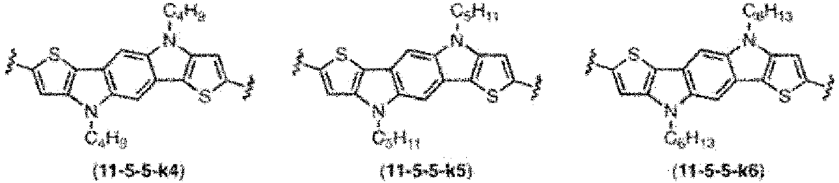

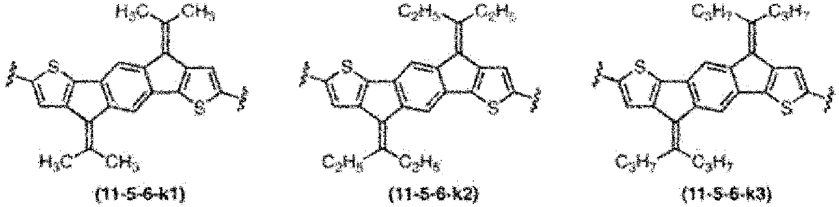

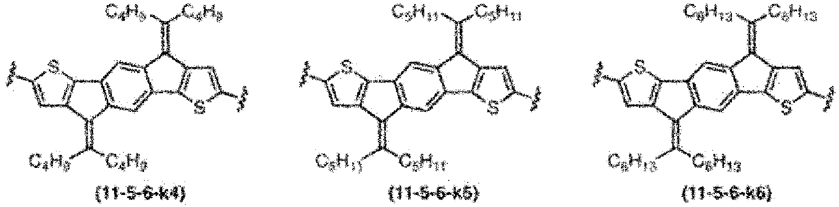

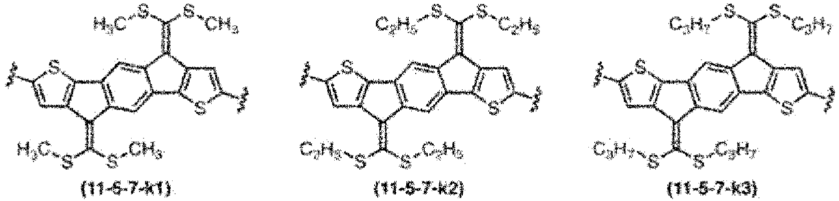

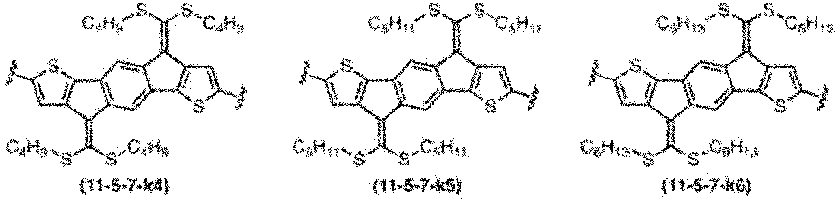

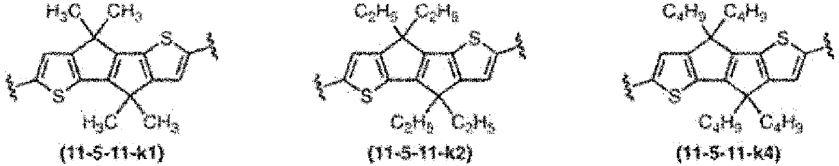

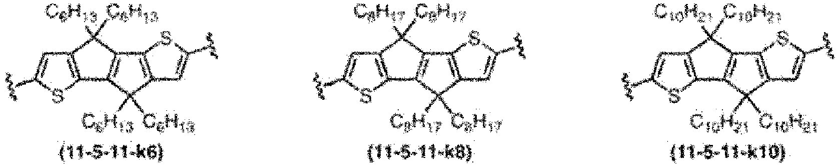

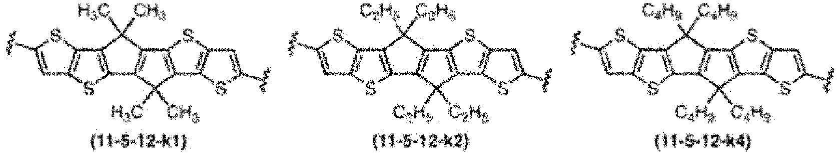

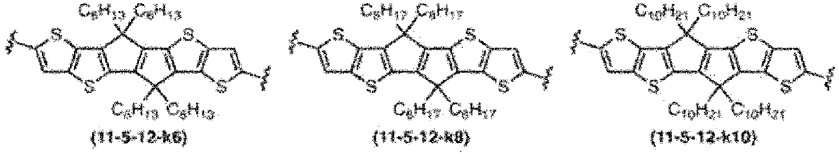

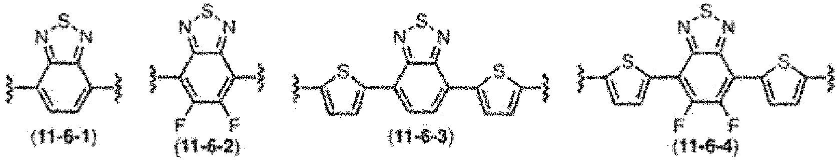

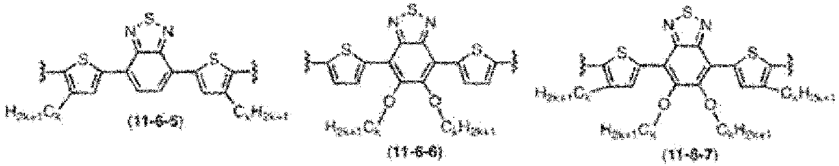

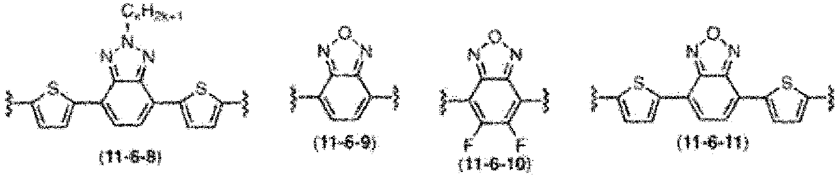

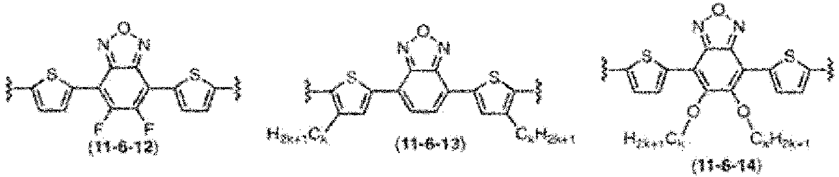

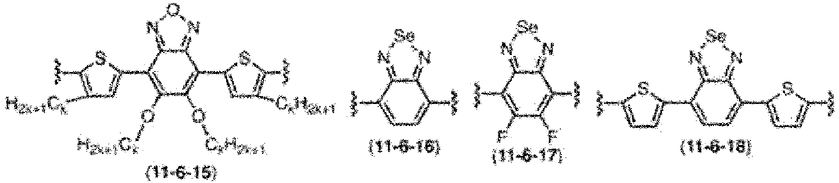

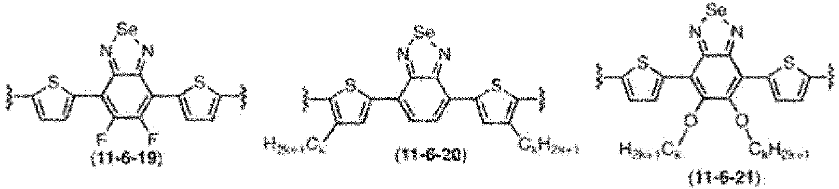

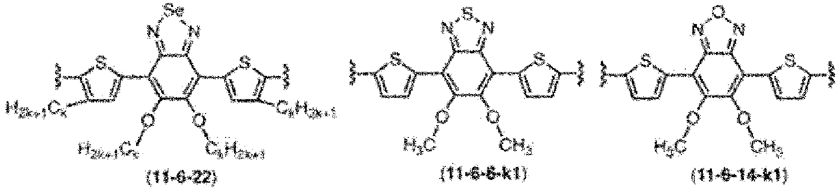

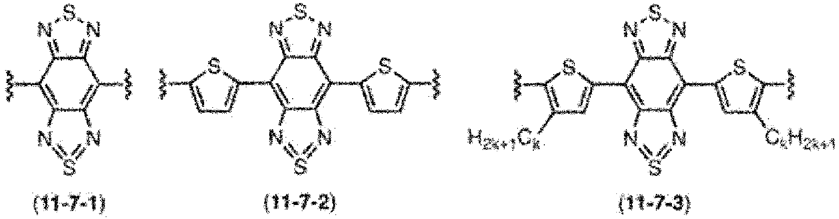

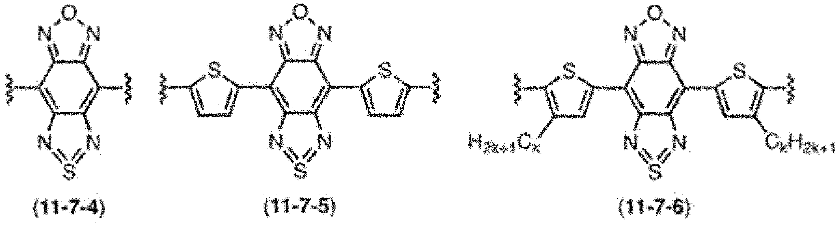

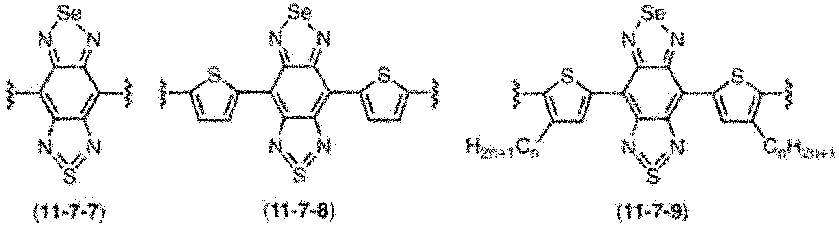

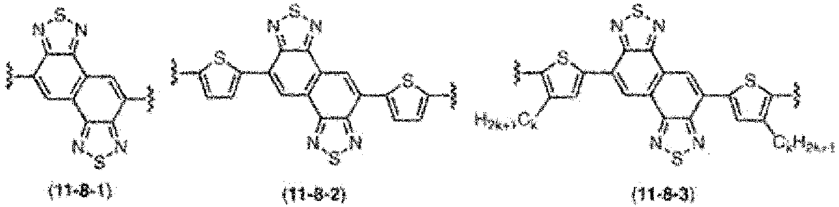

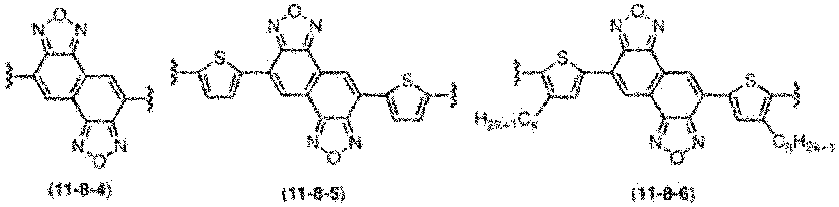

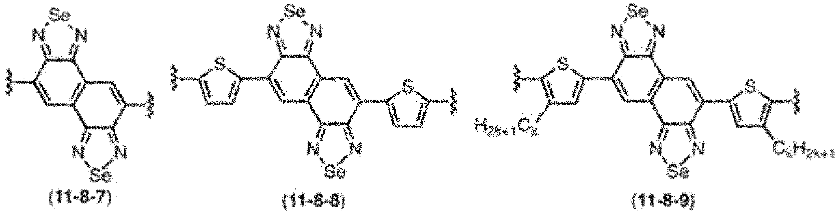

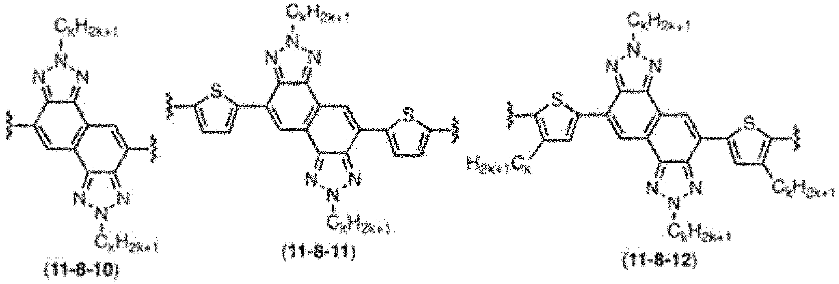

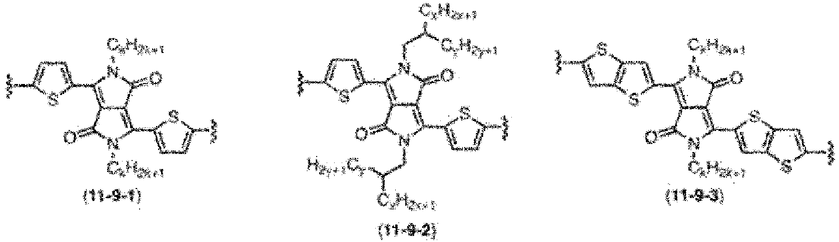

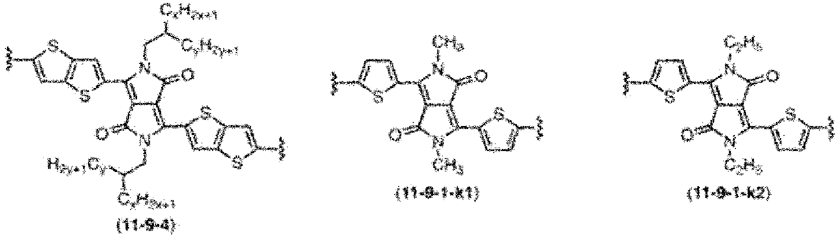

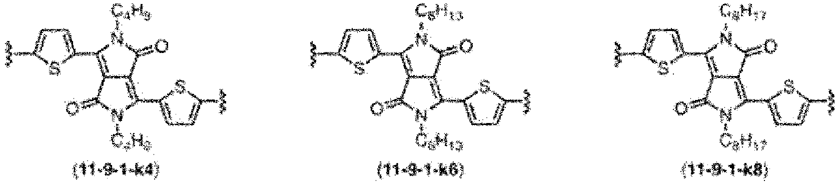

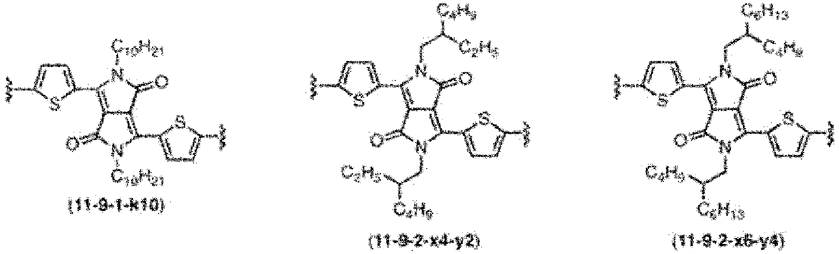

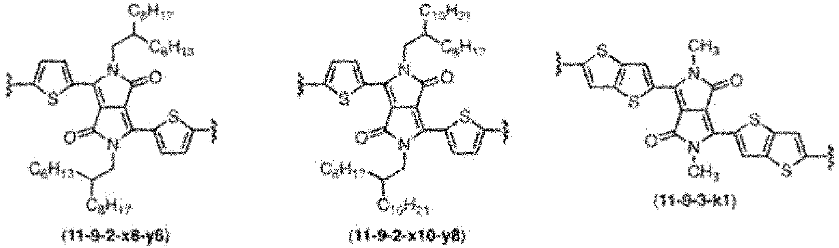

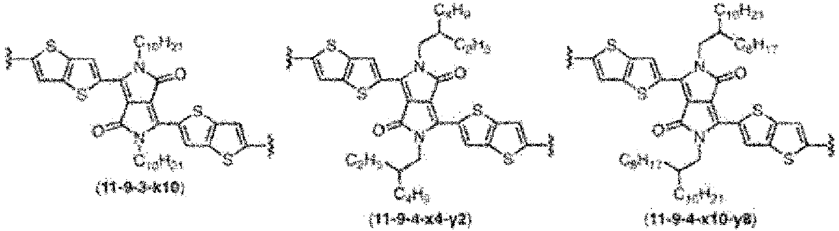

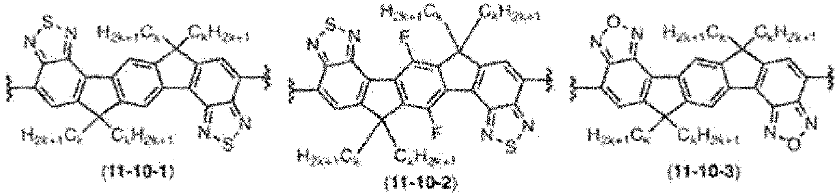

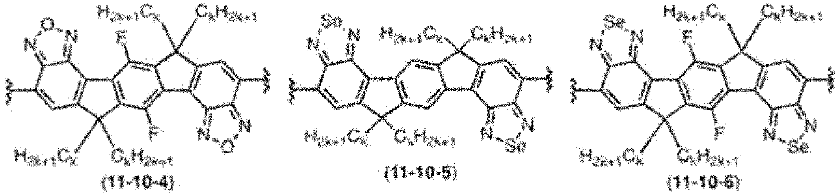

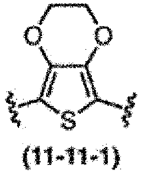

In the formulae, CₖH₂ₖ₊₁, CₓH₂ₓ₊₁, and C_{y}H_{2y+1} each represent a linear alkyl group.

As the structural unit represented by general formula (3), from the viewpoint of improving film formability of the conjugated polymer and increasing carrier mobility of the organic transistor, any structural unit represented by formula (11-1-1) to formula (11-1-5), formula (11-2-1) to formula (11-2-5), formula (11-3-1) to formula (11-3-3), formula (11-3-7), formula (11-4-1) to formula (11-4-9), formula (11-5-1) to formula (11-5-7), formula (11-5-11), formula (11-5-12), formula (11-6-1) to formula (11-6-6), formula (11-7-1), formula (11-7-2), formula (11-8-1), formula (11-8-2), formula (11-9-1) to formula (11-9-4), formula (11-1-5-k1) to formula (11-1-5-k6), formula (11-2-4-k1) to formula (11-2-4-k6), formula (11-2-5-k1) to formula (11-2-5-k6), formula (11-3-3-k1) to formula (11-3-3-k6), formula (11-3-7-k1) to formula (11-3-7-k8), formula (11-4-4-k1) to formula (11-4-4-k6), formula (11-4-5-k1) to formula (11-4-5-k6), formula (11-4-6-k1) to formula (11-4-6-k6), formula (11-4-7-k1) to formula (11-4-7-k6), formula (11-4-8-k1) to formula (11-4-8-k6), formula (11-4-9-k1) to formula (11-4-9-k6), formula (11-5-3-k1) to formula (11-5-3-k6), formula (11-5-4-k1) to formula (11-5-4-k6), formula (11-5-5-k1) to formula (11-5-5-k6), formula (11-5-6-k1) to formula (11-5-6-k6), formula (11-5-7-k1) to formula (11-5-7-k6), formula (11-5-11-k1) to formula (11-5-11-k10), formula (11-5-12-k1) to formula (11-5-12-k10), formula (11-6-6-k1), formula (11-9-1-k1) to formula (11-9-1-k10), formula (11-9-2-x4-y2) to formula (11-9-2-x10-y8), formula (11-9-3-k1), formula (11-9-3-k10), formula (11-9-4-x4-y2), or formula (11-9-4-x10-y8) is preferable, more preferably any structural unit represented by formula (11-1-2), formula (11-1-4), formula (11-2-1), formula (11-2-3), formula (11-2-5), formula (11-3-1), formula (11-3-2), formula (11-3-7), formula (11-4-4), formula (11-4-5), formula (11-4-7), formula (11-4-8), formula (11-4-9), formula (11-5-3), formula (11-5-4), formula (11-5-6), formula (11-5-7), formula (11-5-11), formula (11-5-12), formula (11-6-3), formula (11-6-4), formula (11-7-2), formula (11-8-2), formula (11-9-1), (11-9-2), formula (11-2-5-k1) to formula (11-2-5-k6), formula (11-3-3-k1) to formula (11-3-3-k6), formula (11-3-7-k1) to formula (11-3-7-k8), formula (11-4-4-k1) to formula (11-4-4-k6), formula (11-4-5-k1) to formula (11-4-5-k6), formula (11-4-7-k1) to formula (11-4-7-k6), formula (11-4-8-k1) to formula (11-4-8-k6), formula (11-4-9-k1) to formula (11-4-9-k6), formula (11-5-3-k1) to formula (11-5-3-k6), formula (11-5-4-k1) to formula (11-5-4-k6), formula (11-5-6-k1) to formula (11-5-6-k6), formula (11-5-7-k1) to formula (11-5-7-k6), formula (11-5-11-k1) to formula (11-5-11-k10), formula (11-5-12-k1) to formula (11-5-12-k10), formula (11-9-1-k1) to formula (11-9-1-k10), or formula (11-9-2-x4-y2) to formula (11-9-2-x10-y8), further preferably any structural unit represented by formula (11-1-2), formula (11-2-1), formula (11-3-1), formula (11-3-7), formula (11-4-4), formula (11-4-7), formula (11-4-8), formula (11-4-9), formula (11-5-3), formula (11-9-1), formula (11-9-2), formula (11-3-3-k1) to formula (11-3-3-k6), formula (11-3-7-k1) to formula (11-3-7-k8), formula (11-4-4-k1) to formula (11-4-4-k6), formula (11-4-7-k1) to formula (11-4-7-k6), formula (11-4-8-k1) to formula (11-4-8-k6), formula (11-4-9-k1) to formula (11-4-9-k6), formula (11-5-3-k1) to formula (11-5-3-k6), formula (11-9-1-k1) to formula (11-9-1-k10), or formula (11-9-2-x4-y2) to formula (11-9-2-x10-y8), and especially preferably any structural unit represented by formula (11-1-2), formula (11-2-1), formula (11-3-1), formula (11-3-3-k4), formula (11-3-3-k6), formula (11-3-7-k8), formula (11-4-4-k1), formula (11-4-7-k1), formula (11-4-8-k1), formula (11-4-8-k3), formula (11-4-9-k1), formula (11-5-3-k1), formula (11-9-1-k1), formula (11-9-1-k8), or formula (11-9-2-x10-y8).

The conjugated polymer is not particularly limited in the order of structural units as long as the conjugated polymer includes the structural unit represented by general formula (2) and the structural unit represented by general formula (3), and examples thereof include copolymerization modes such as alternating, random, block, and gradient. From the viewpoint of improving film formability of the conjugated polymer and mobility, a conjugated polymer having a structure in which the structural unit represented by general formula (2) and the structural unit represented by general formula (3) are alternately repeated is preferable, this structure is represented by general formula (24), and it is further preferable that X in general formula (24) is a linking group selected from the group consisting of general formulae (3-1) through (3-3) below. It is still more preferable that the conjugated polymer is composed of a structural unit represented by general formula (24), X is a linking group represented by general formulae (3-1) through (3-3) below, and further at least one of A and B is a linking group represented by general formulae (4-1) through (4-8). Moreover, it is particularly preferable that the conjugated polymer is composed of a structural unit represented by general formula (24), A and B in general formula (24) are each a linking group represented by general formula (4-1), and X is a linking group selected from the group consisting of general formulae (3-1) through (3-3). 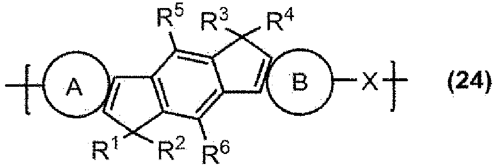 where R¹, R², R³, R⁴, R⁵, R⁶, J¹, J², and X have the same meanings as described above.

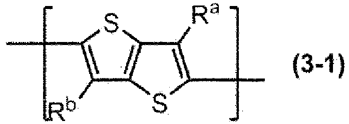

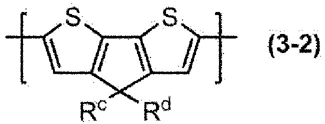

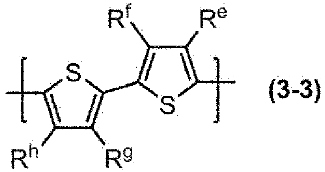

R^{a}, R^{b}, R^{c}, R^{d}, R^{e}, R^{f}, R^{g}, and R^{h} each independently represent hydrogen or an alkyl group having 1 to 50 carbon atoms or an alkoxy group having 1 to 50 carbon atoms.

Examples of the alkyl group having 1 to 50 carbon atoms and the alkoxy group having 1 to 50 carbon atoms as R^{a}, R^{b}, R^{c}, R^{d}, R^{e}, R^{f}, R^{g}, and R^{h} include groups similar to the alkyl group having 1 to 50 carbon atoms and the alkoxy group having 1 to 50 carbon atoms as A and B in general formula (2).

As R^{a}, R^{b}, R^{e}, R^{f}, R^{g}, and R^{h}, hydrogen or an alkyl group having 1 to 50 carbon atoms is preferable, and hydrogen is more preferable.

As R^{c} and R^{d}, hydrogen or an alkyl group having 1 to 50 carbon atoms is preferable, hydrogen or a methyl group is more preferable, and a methyl group is especially preferable.

Examples of the structural unit represented by general formula (24) can be structural units represented below. Note, however, that the present invention is not limited to those examples. In the formulae, n represents a natural number of 1 to 50, which may be the same or different. k represents a natural number of 1 to 50, which may be the same or different. x represents a natural number of 2 to 24, which may be the same or different. y represents a natural number of 1 to 24, which may be the same or different. z represents a natural number of 1 to 20, which may be the same or different. Specific examples of structural units encompassed therein are also indicated below.

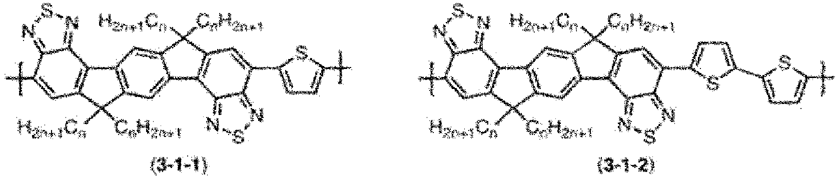

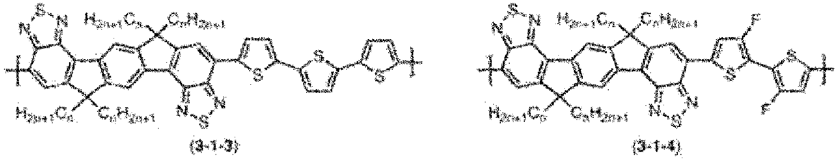

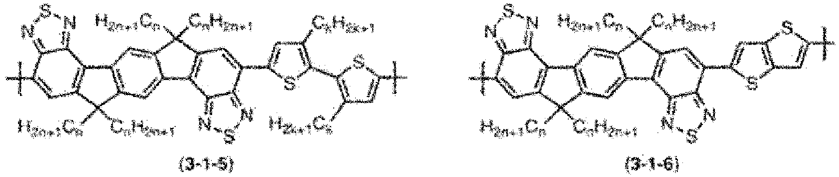

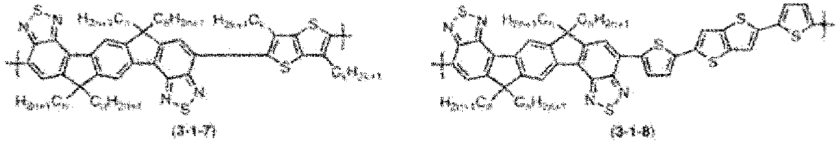

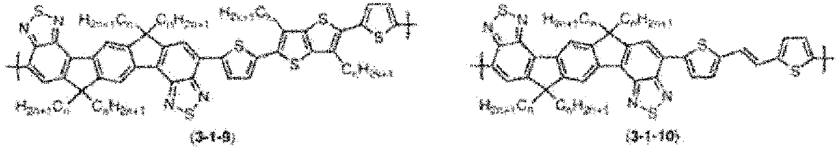

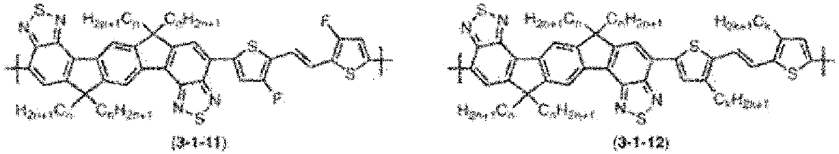

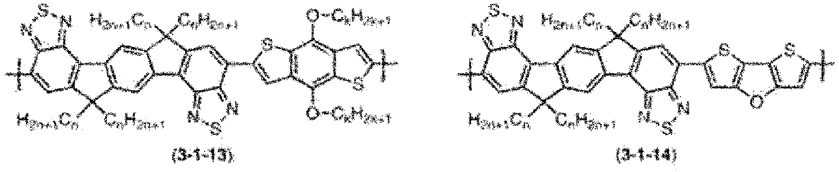

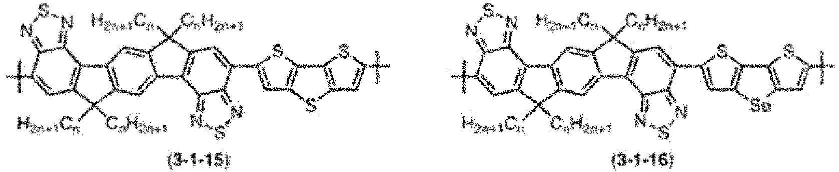

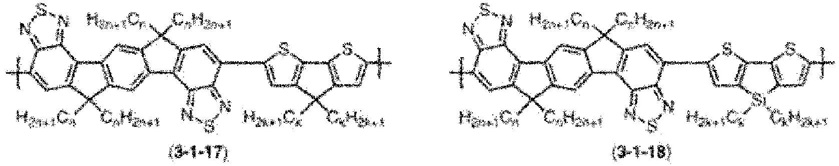

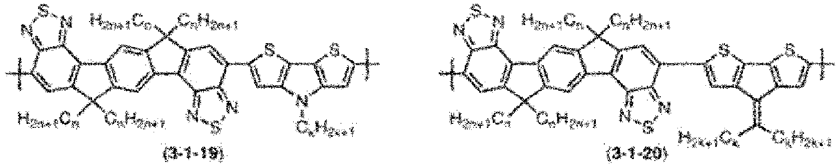

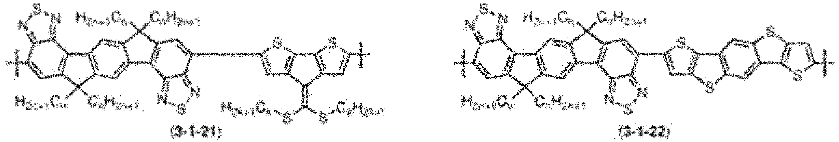

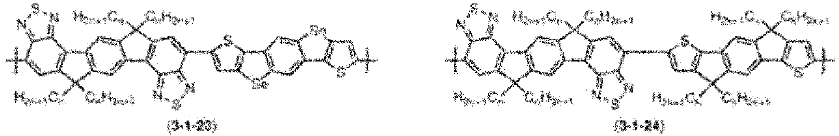

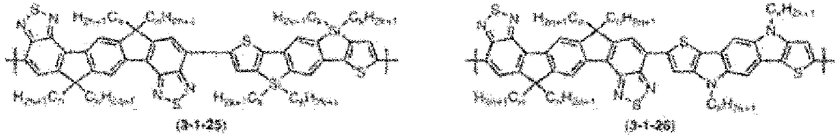

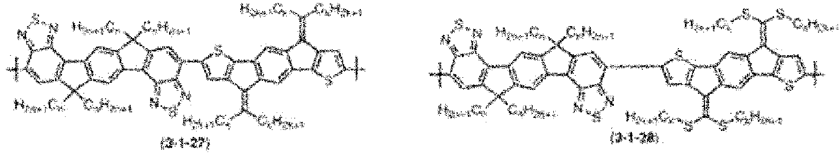

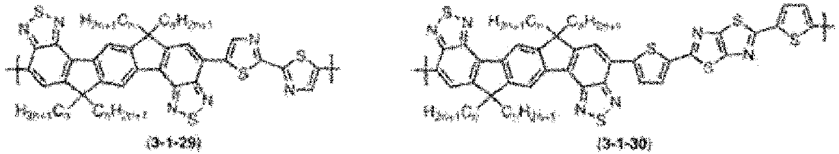

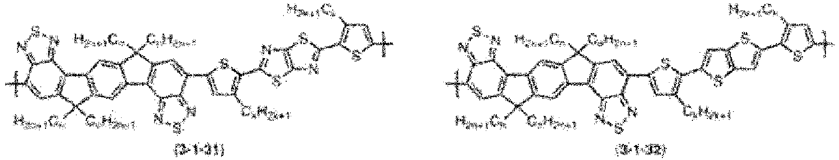

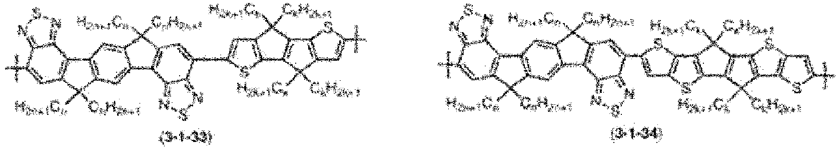

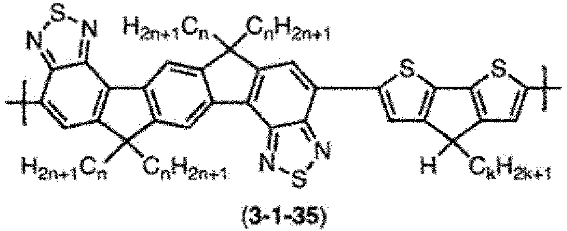

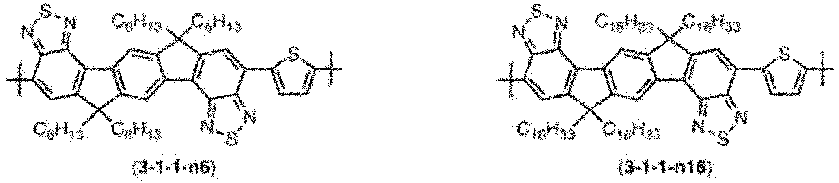

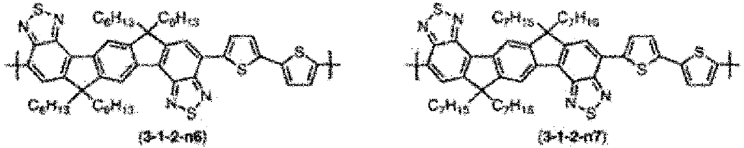

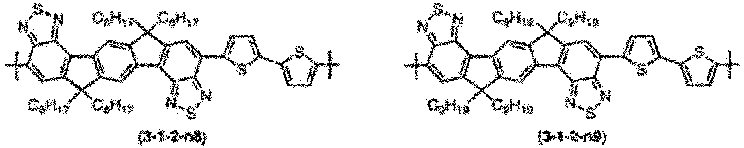

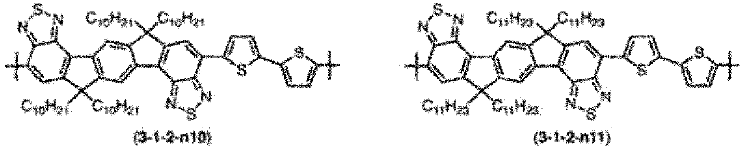

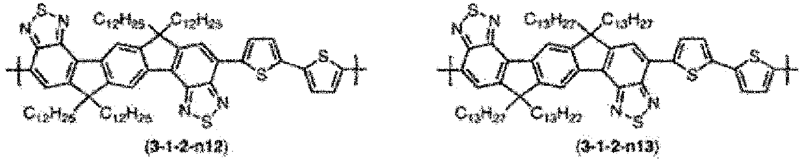

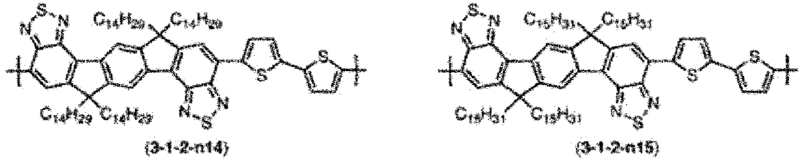

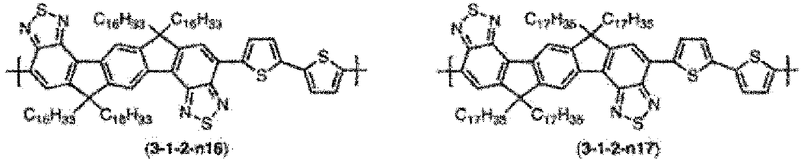

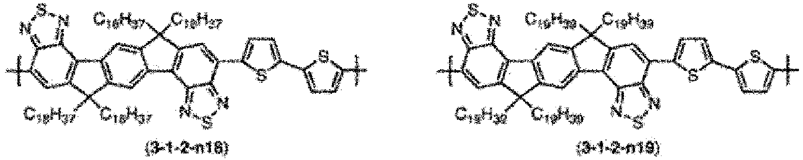

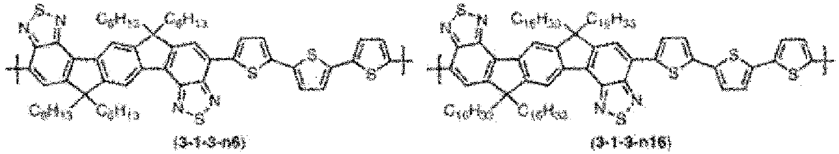

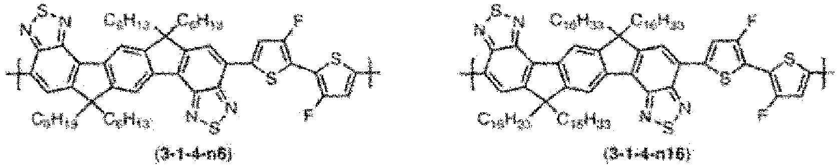

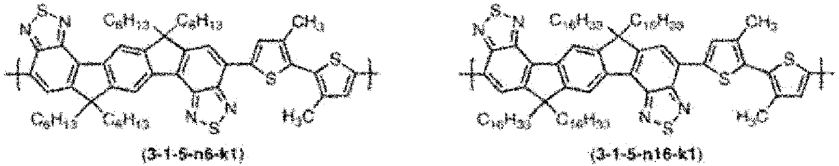

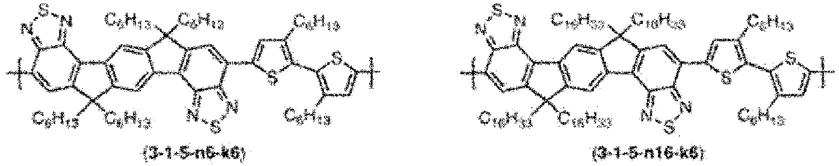

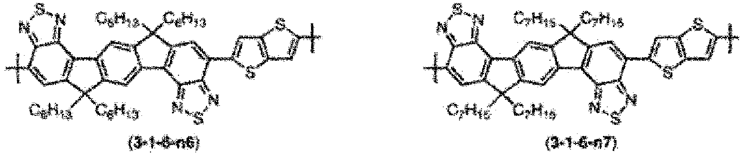

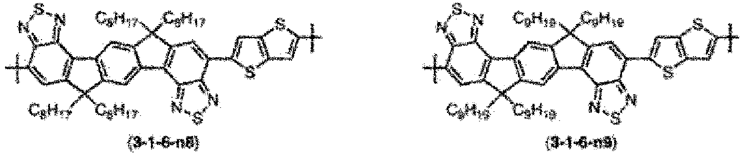

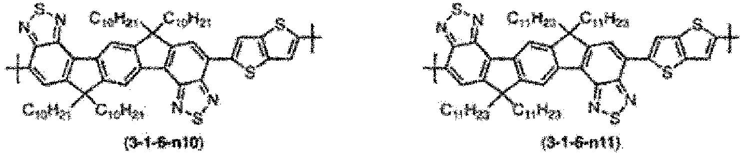

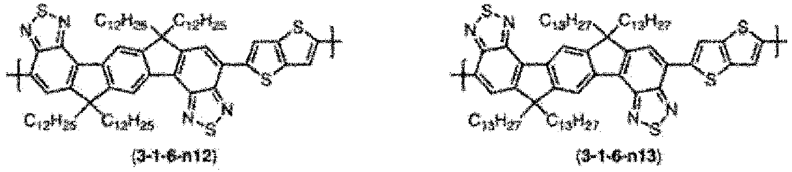

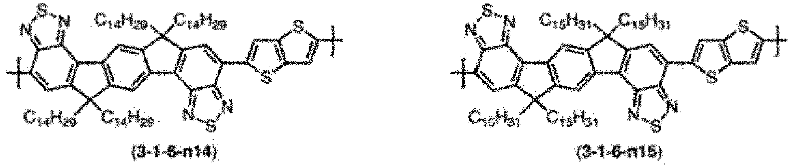

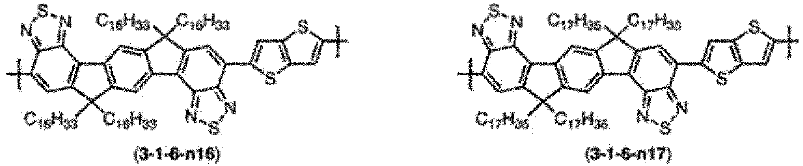

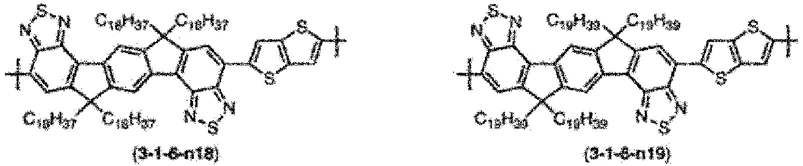

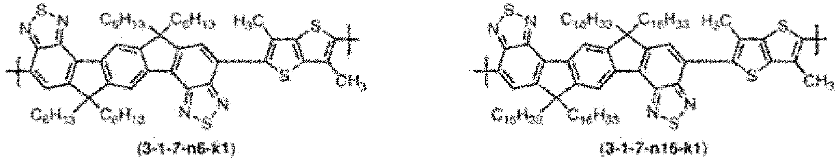

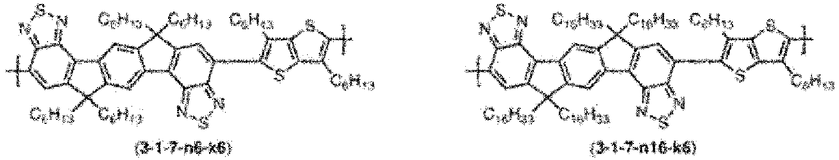

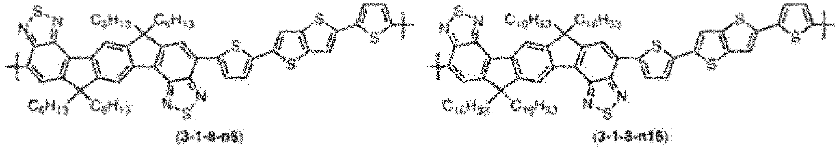

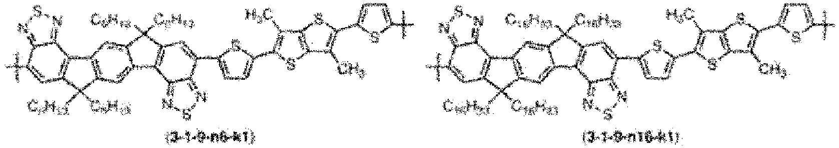

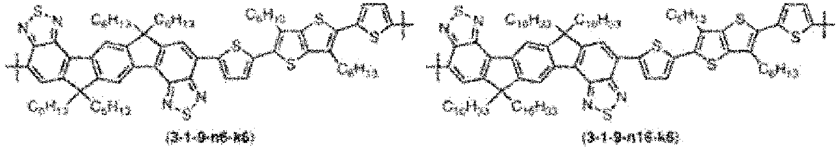

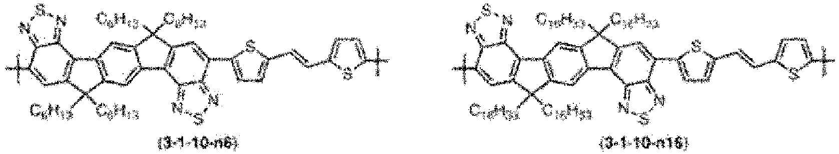

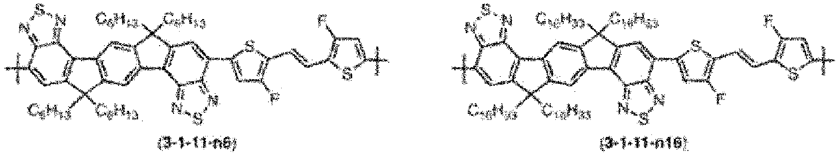

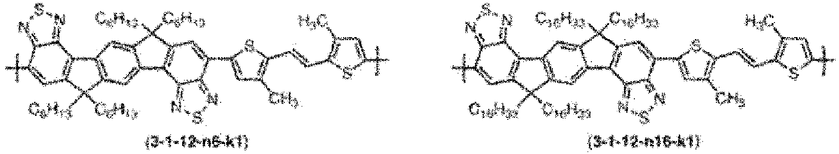

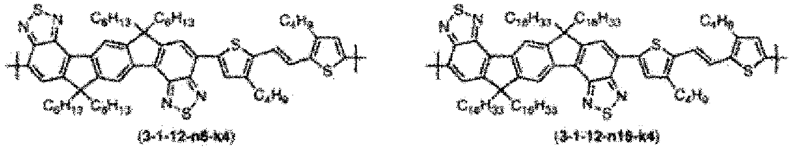

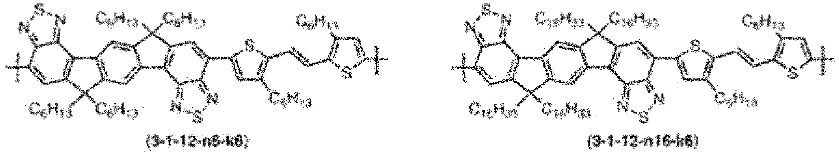

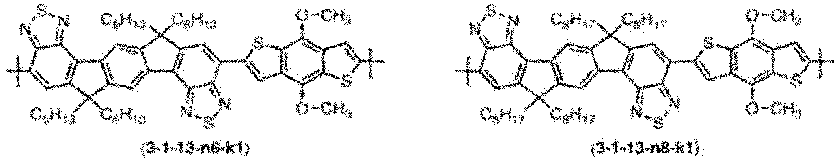

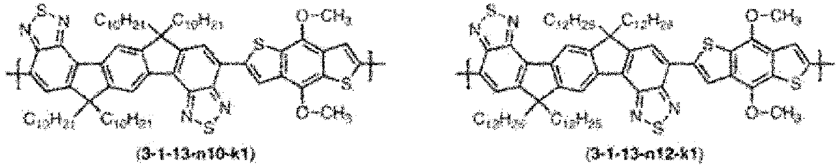

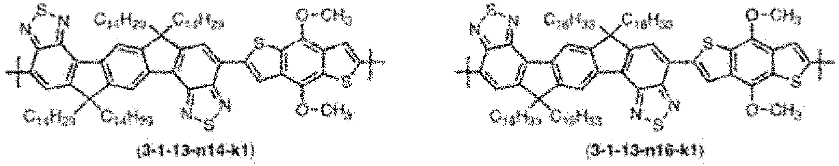

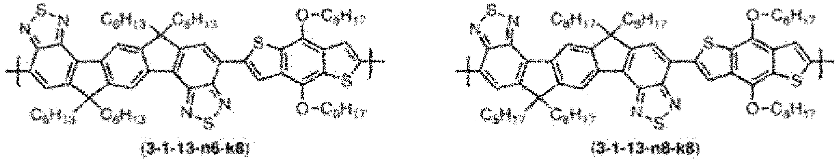

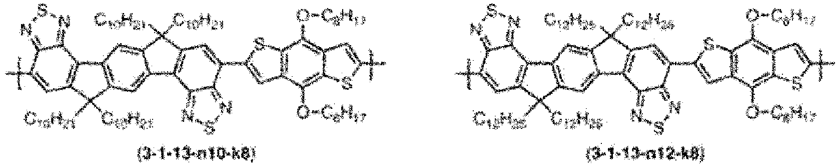

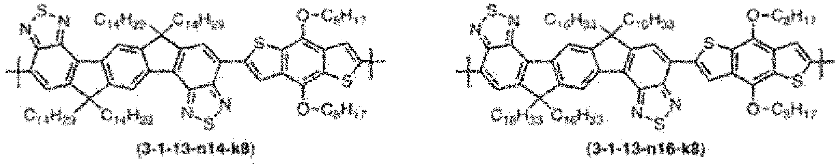

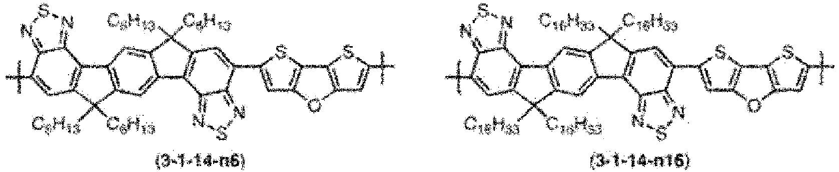

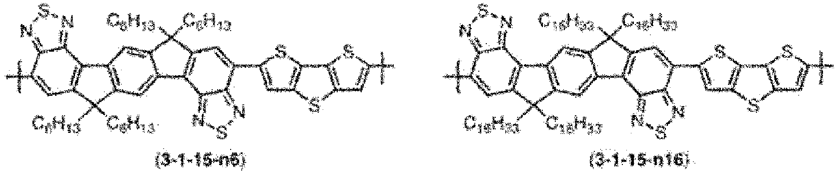

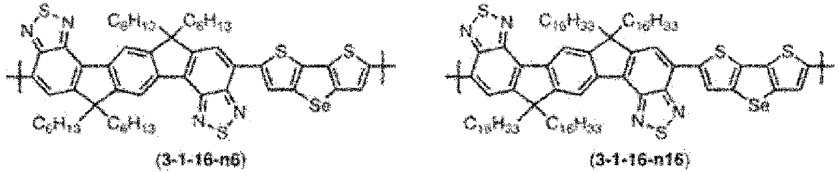

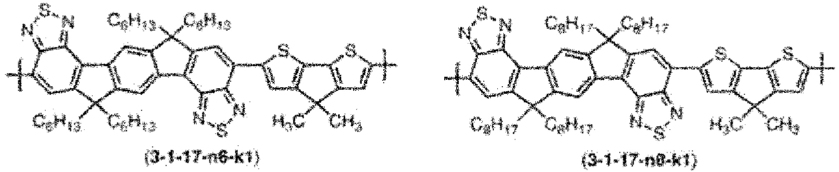

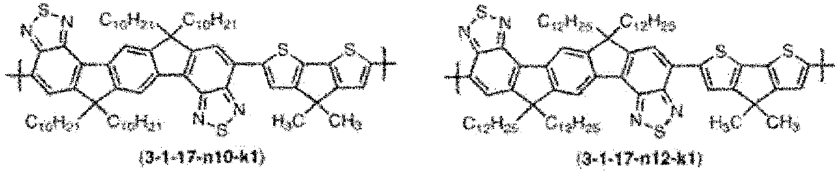

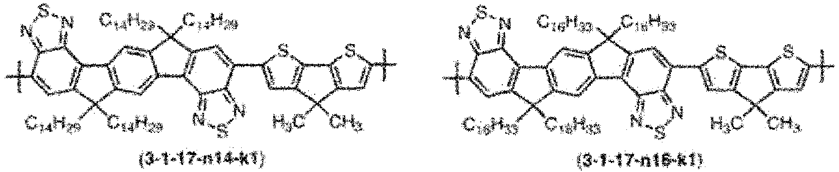

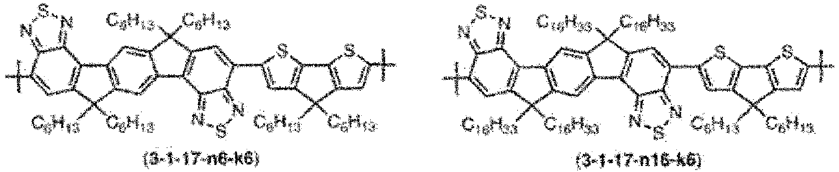

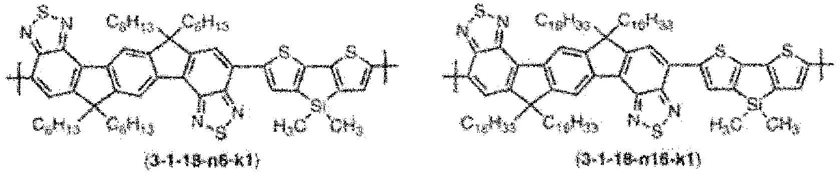

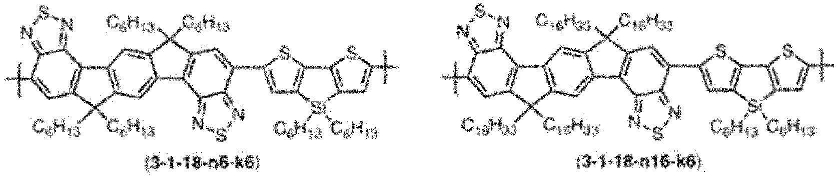

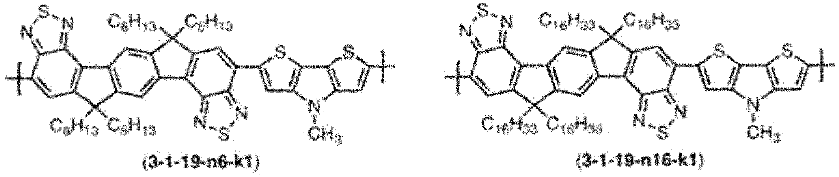

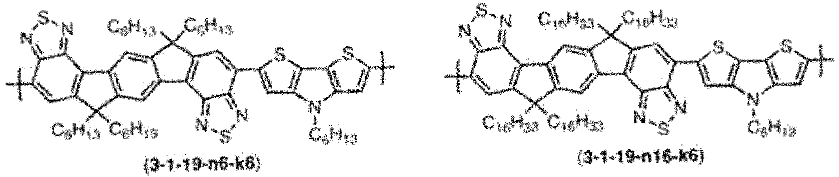

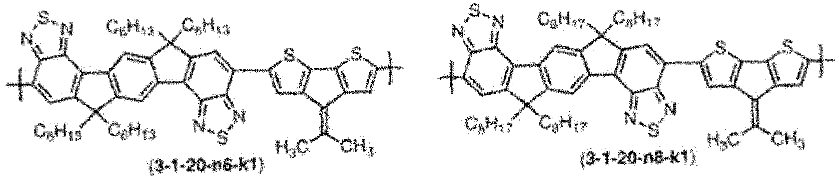

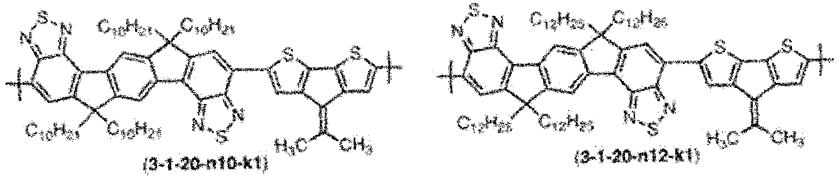

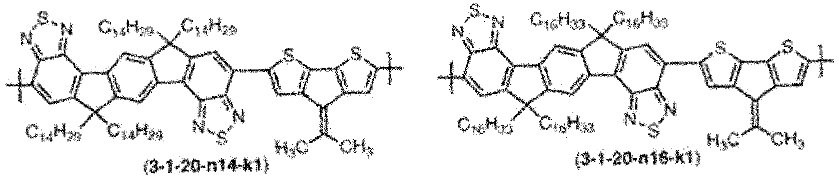

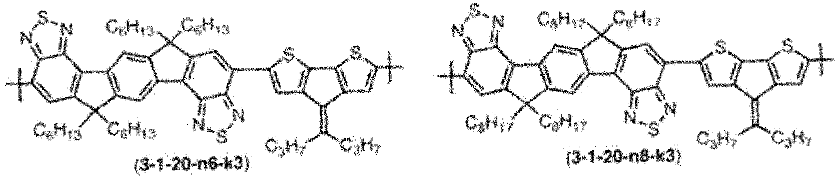

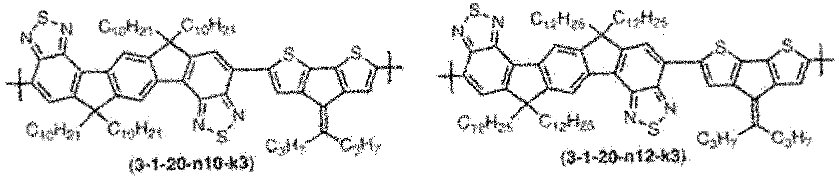

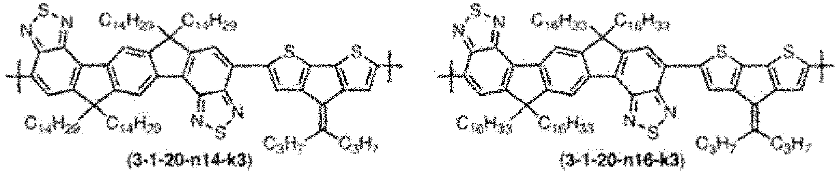

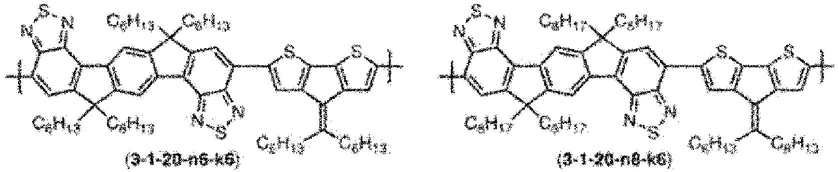

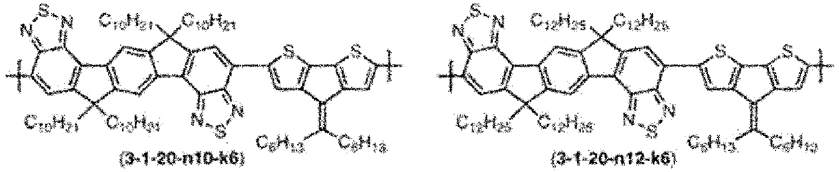

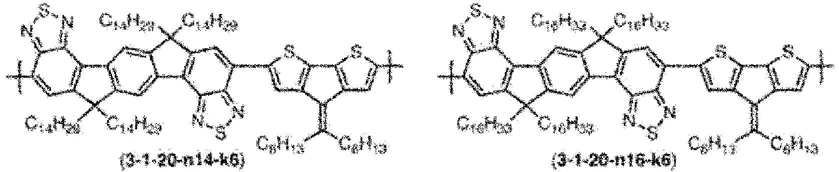

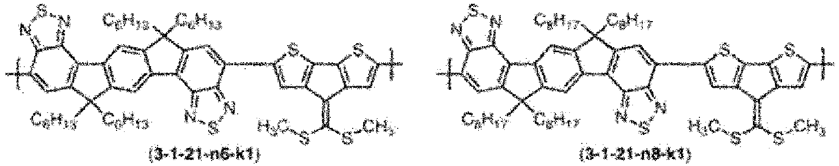

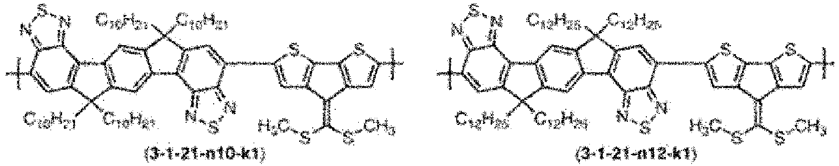

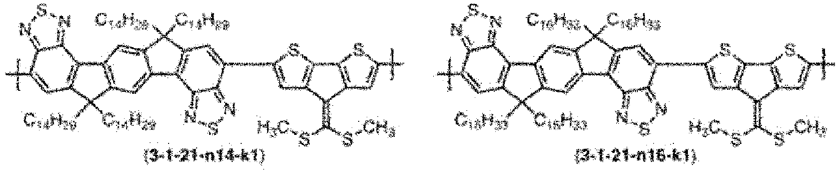

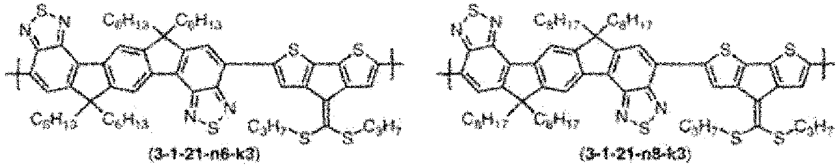

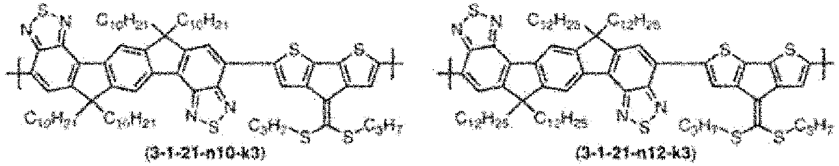

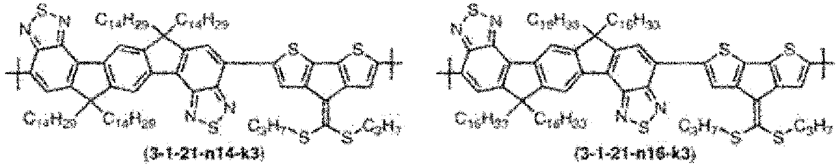

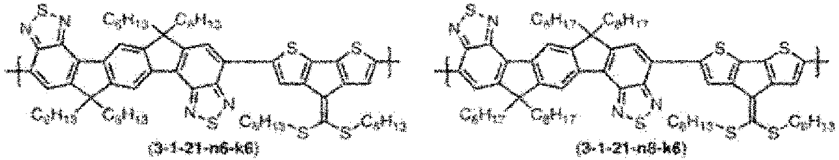

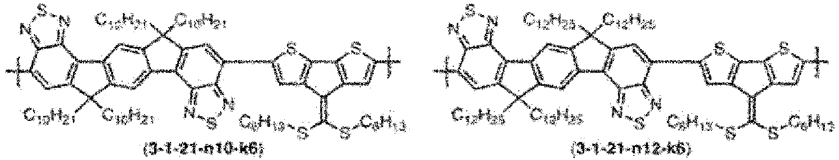

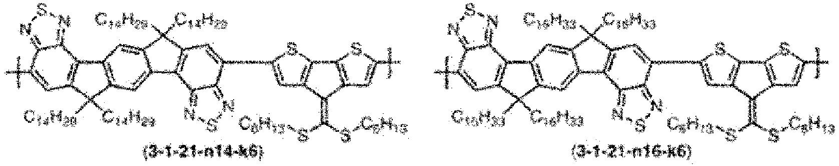

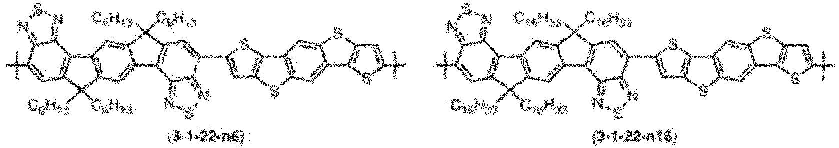

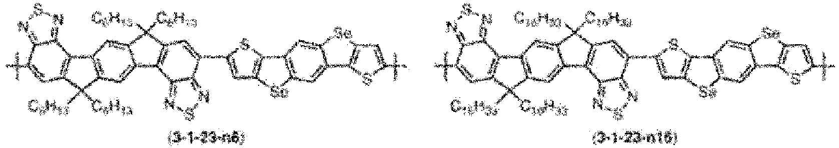

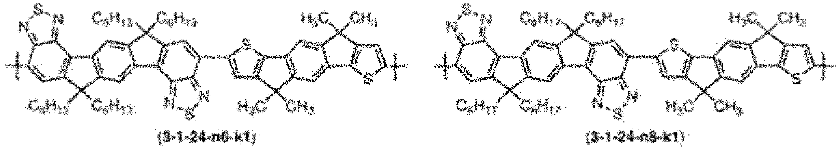

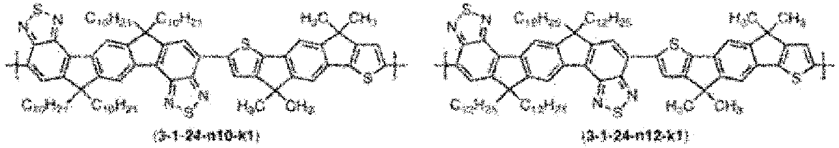

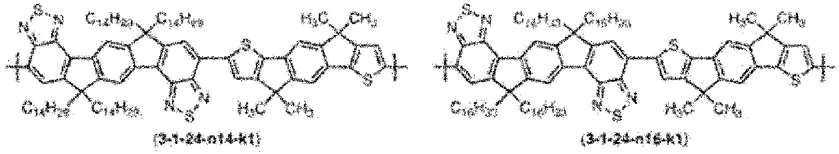

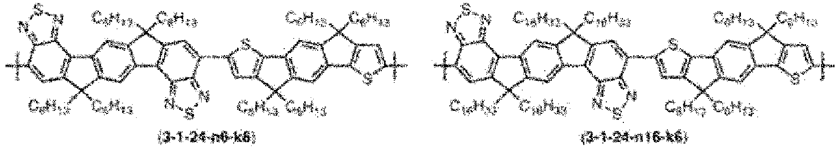

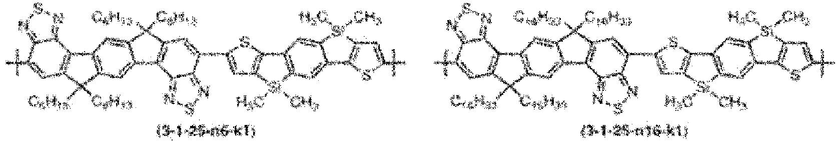

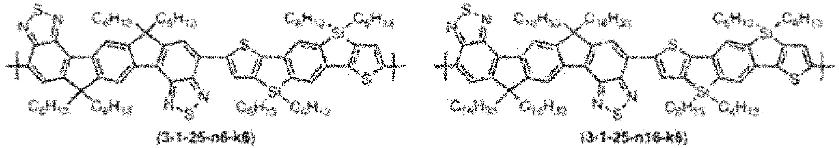

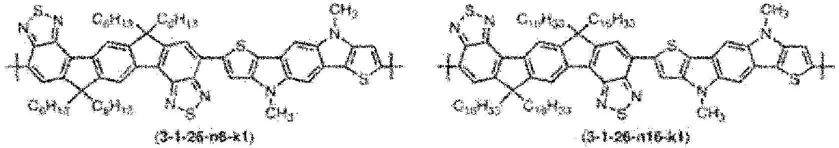

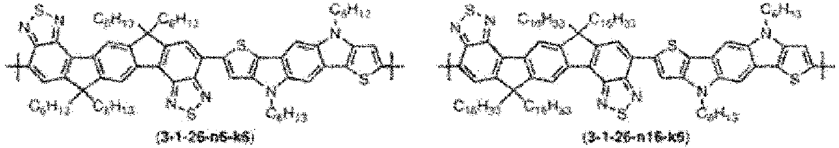

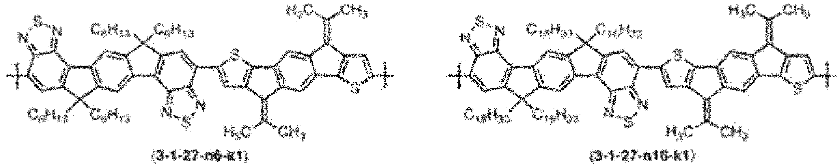

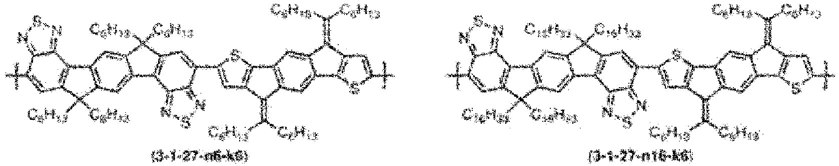

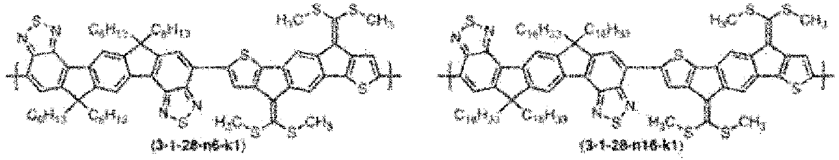

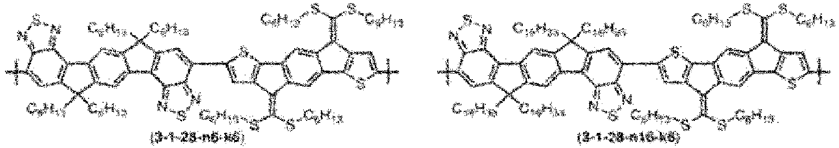

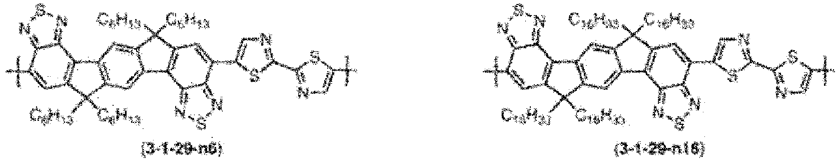

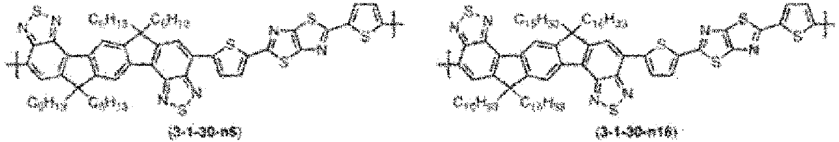

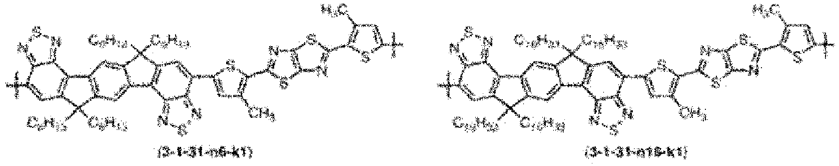

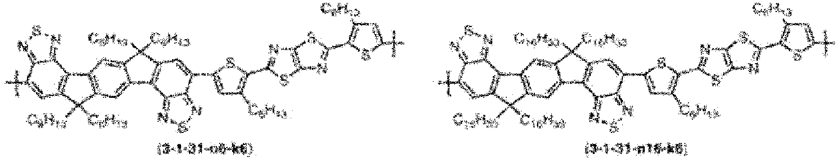

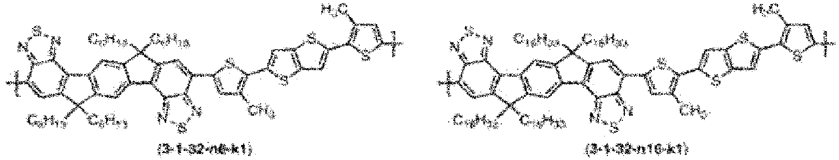

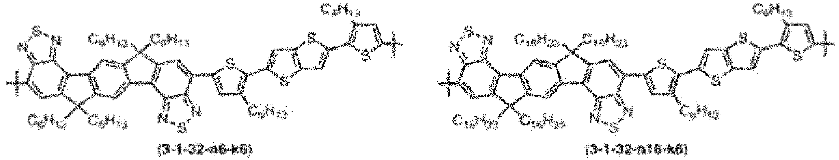

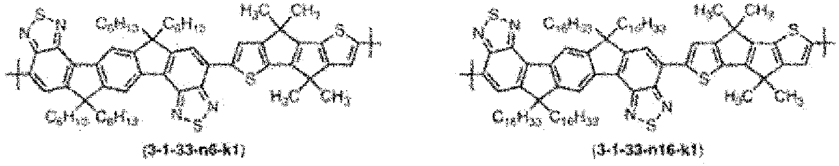

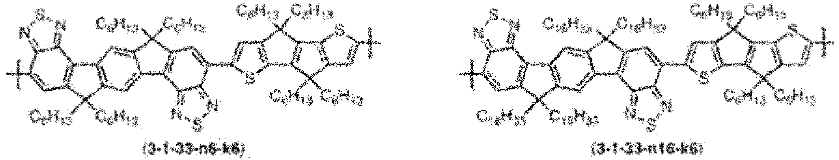

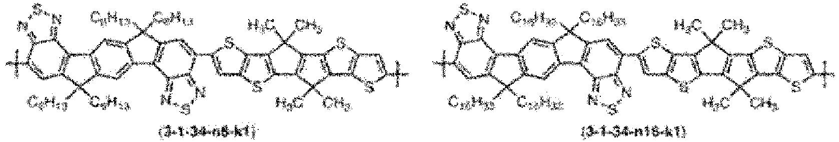

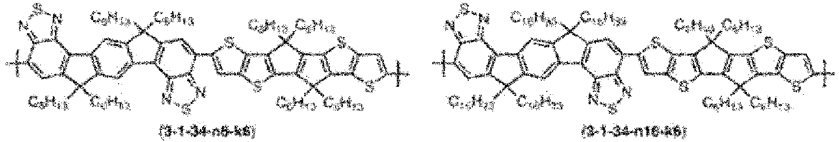

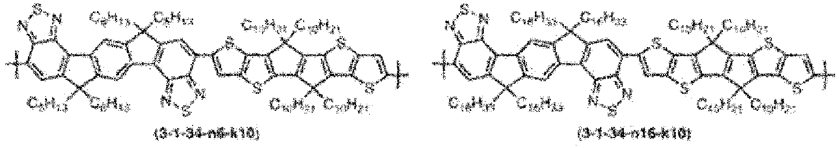

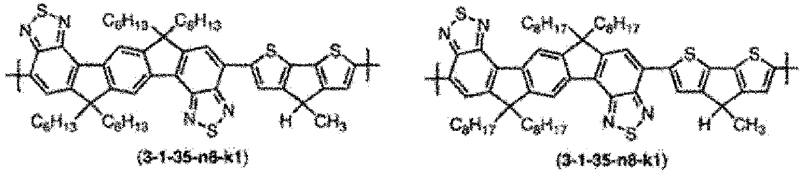

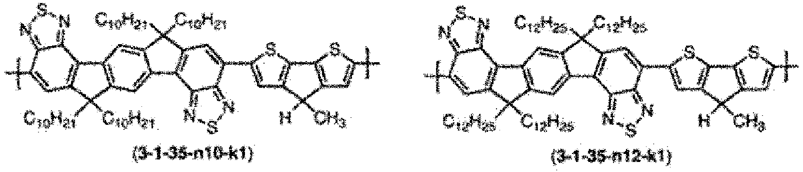

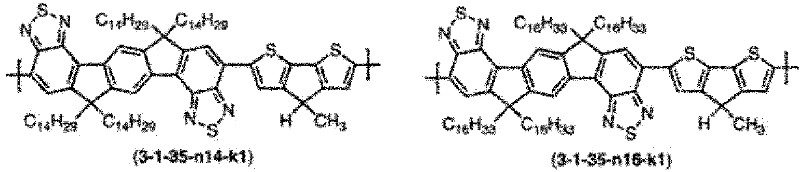

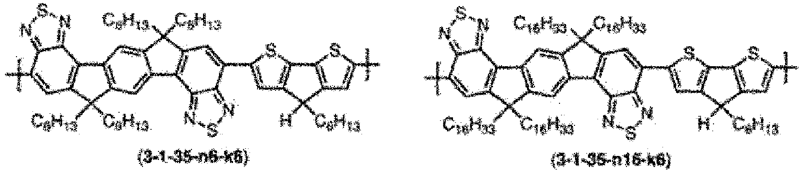

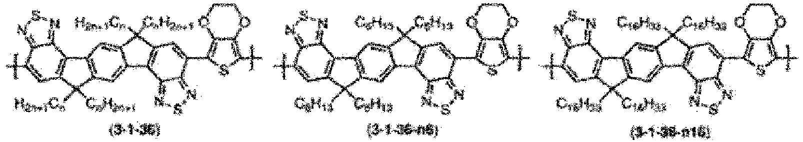

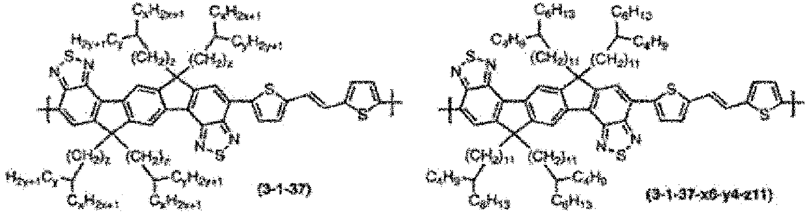

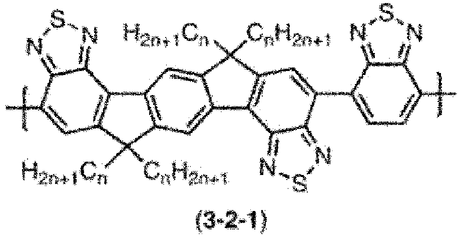

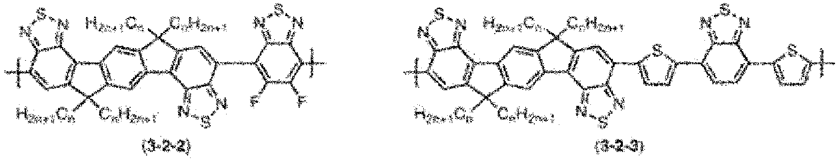

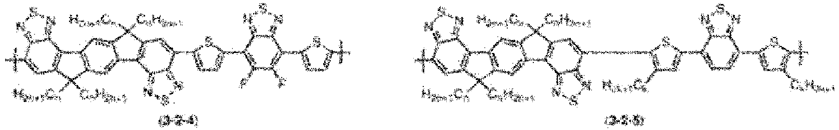

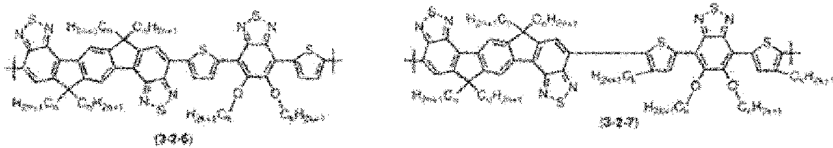

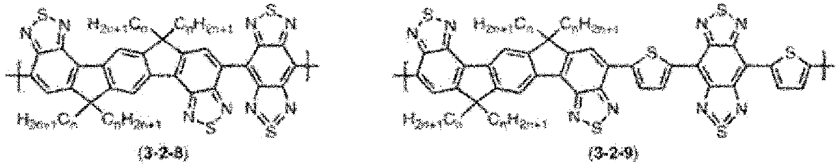

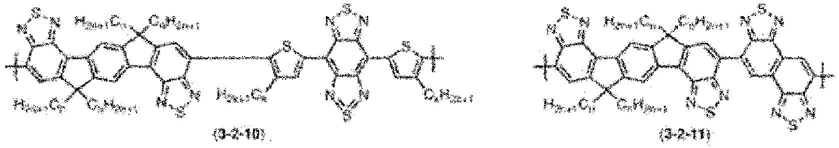

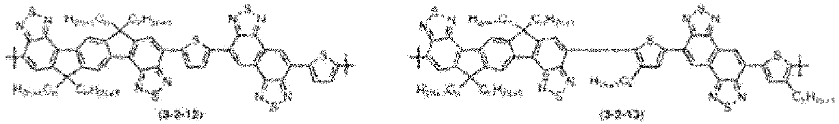

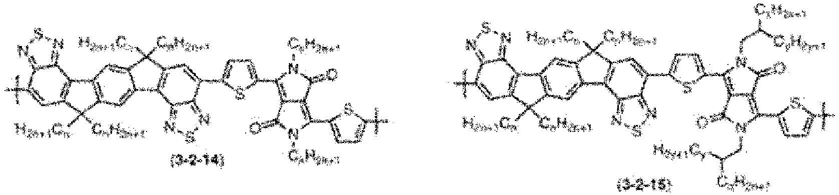

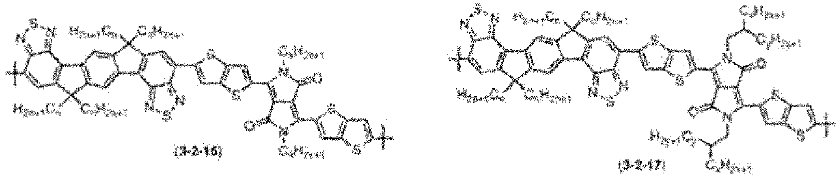

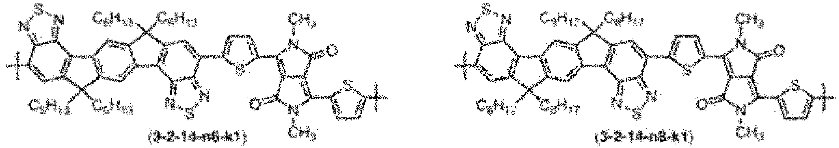

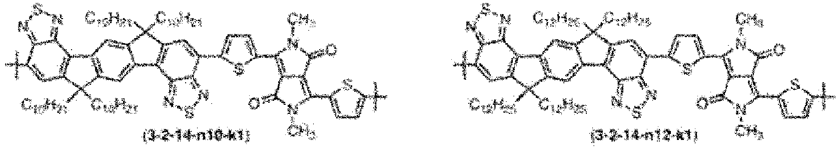

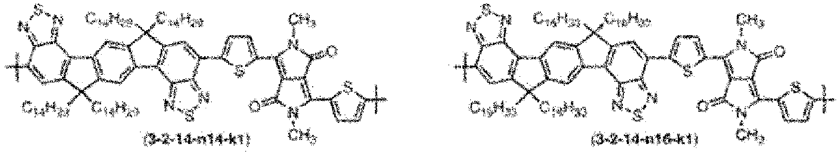

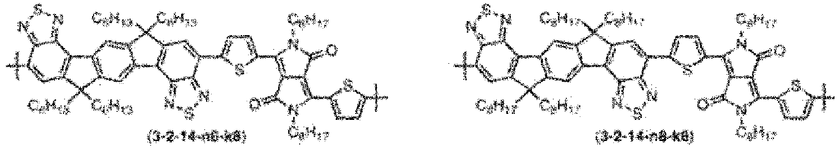

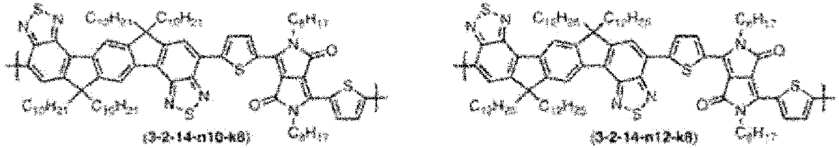

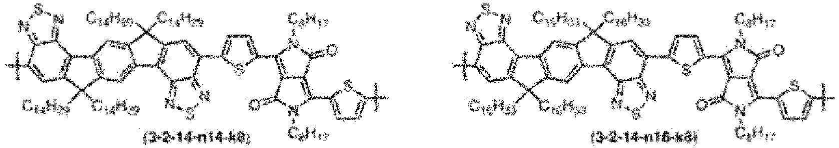

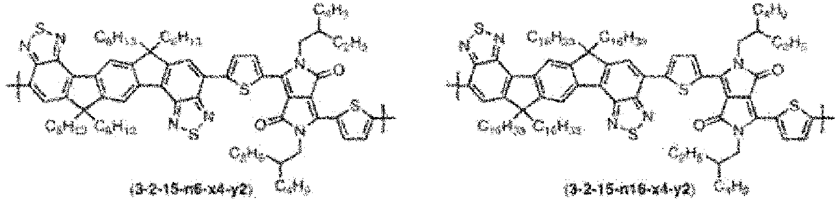

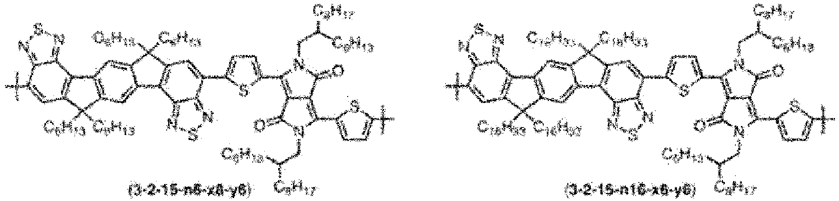

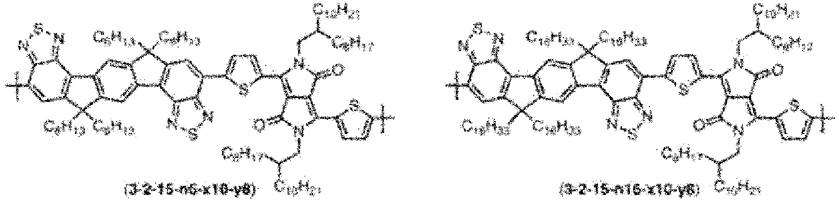

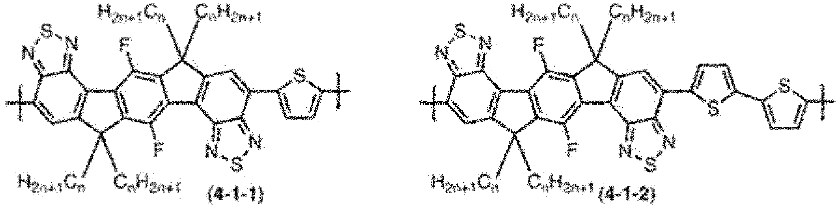

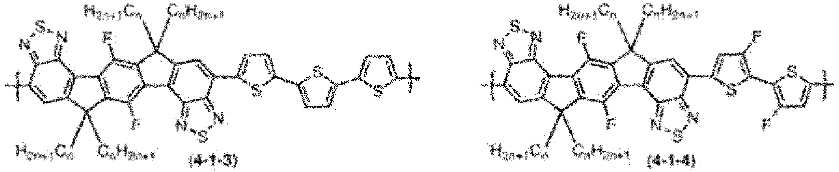

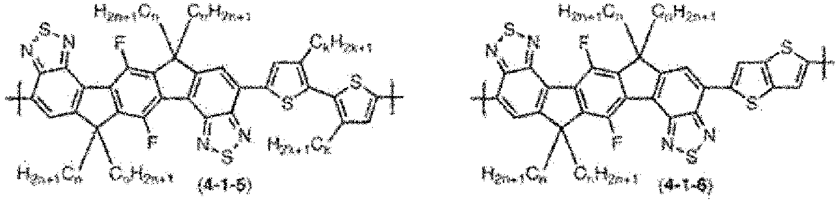

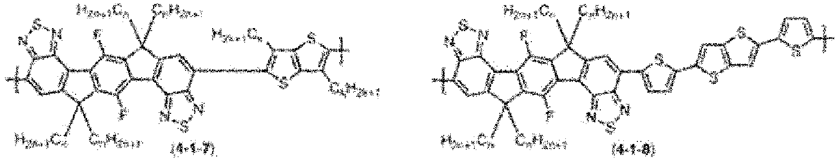

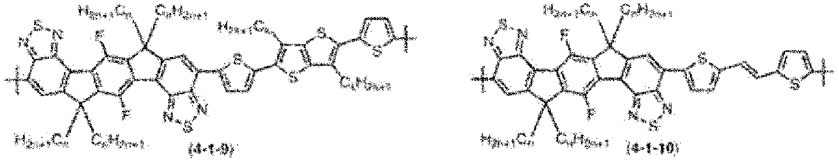

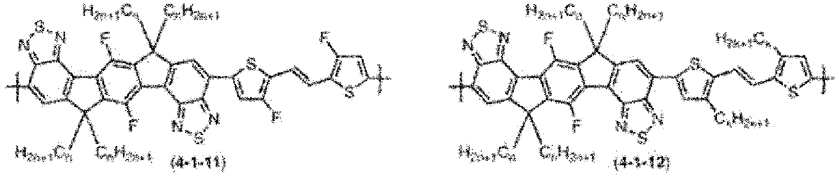

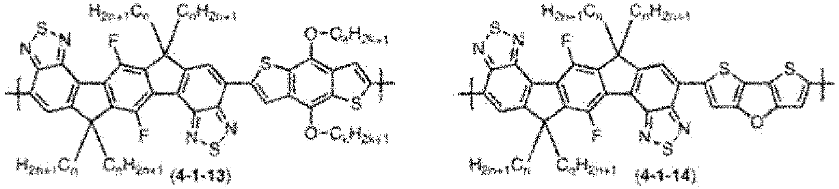

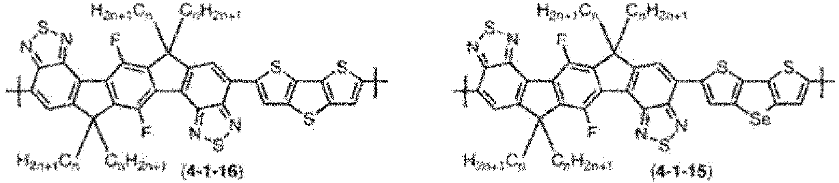

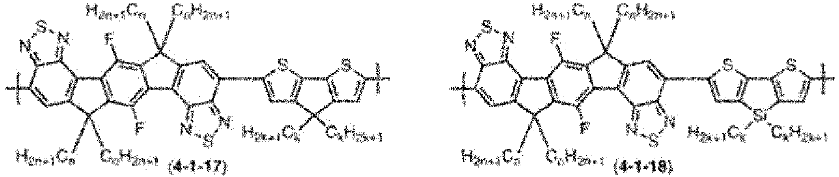

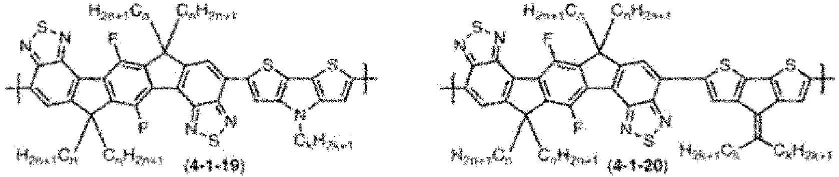

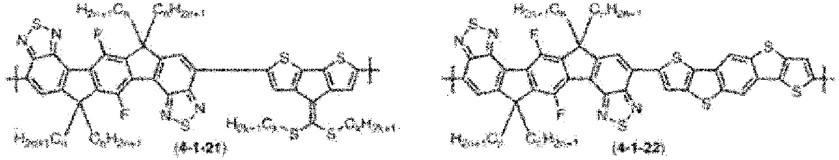

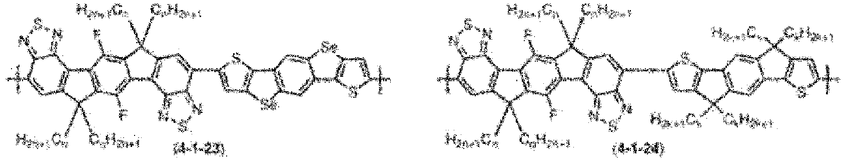

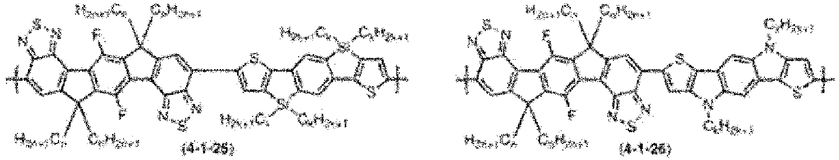

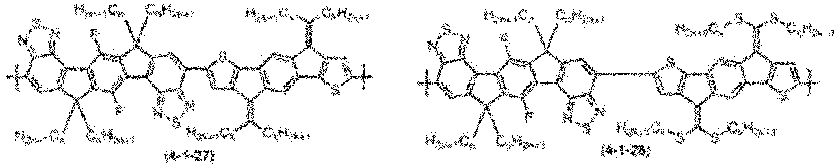

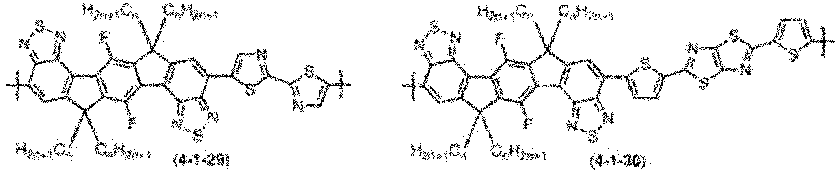

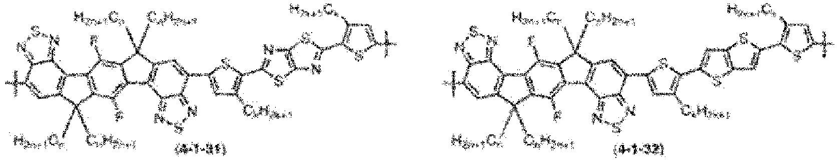

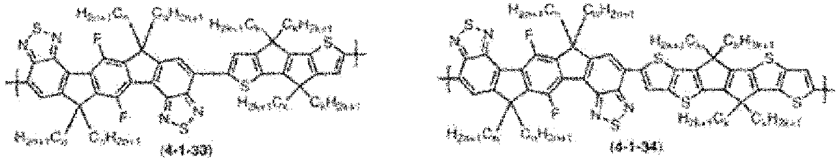

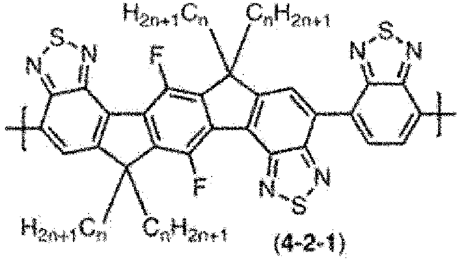

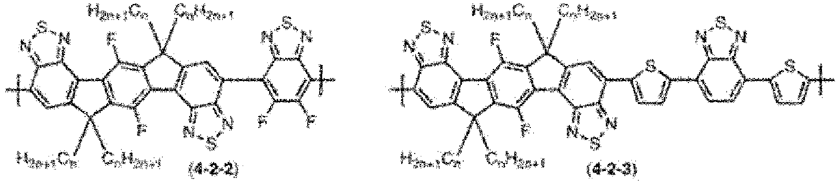

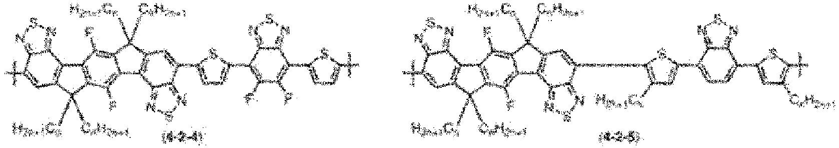

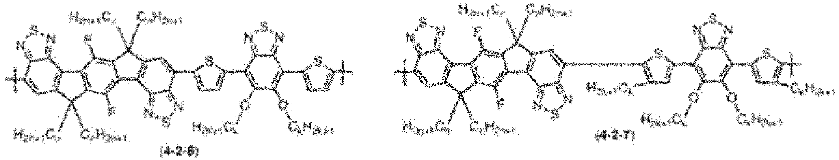

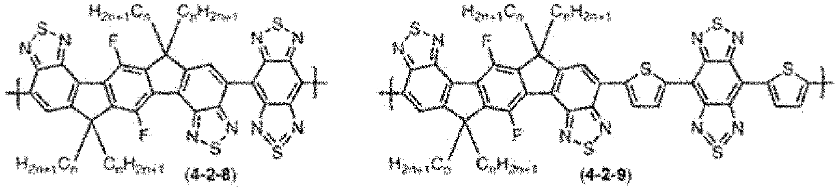

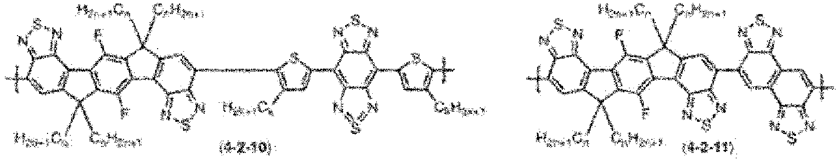

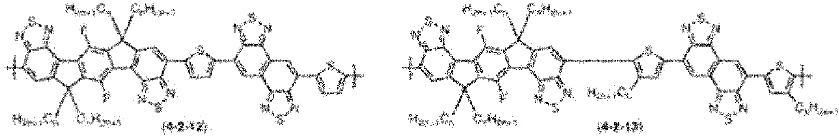

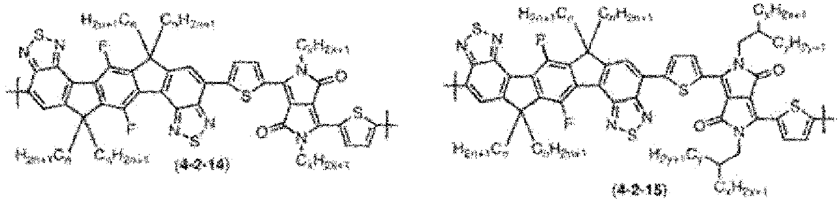

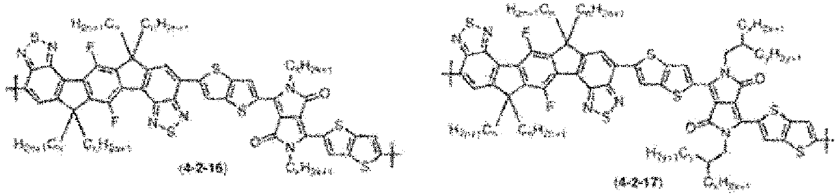

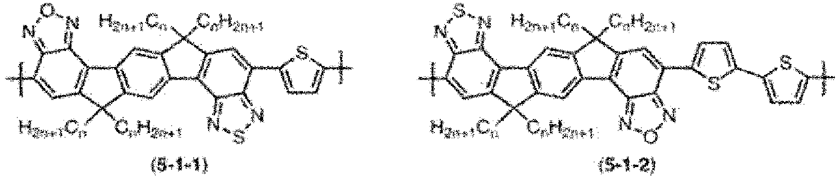

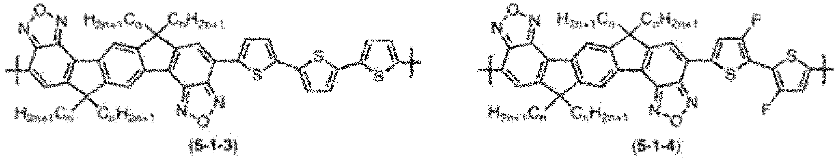

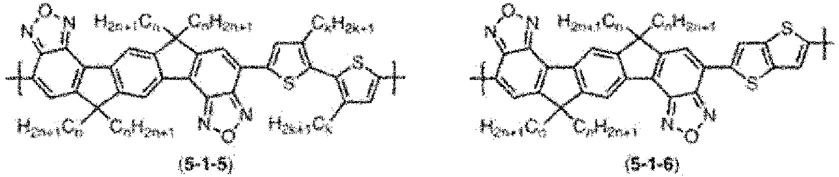

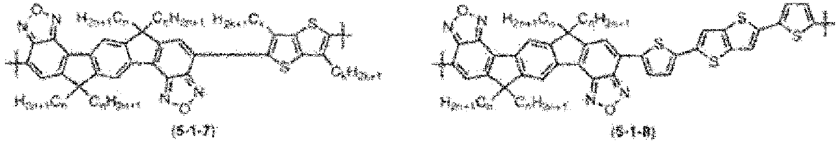

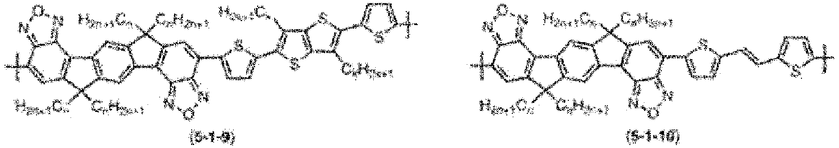

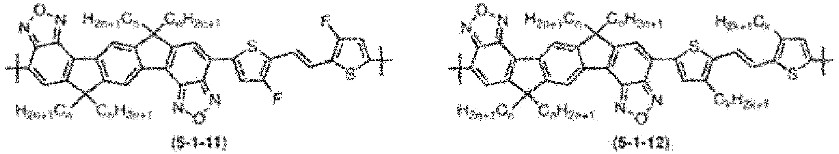

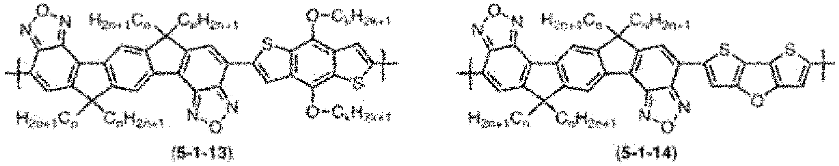

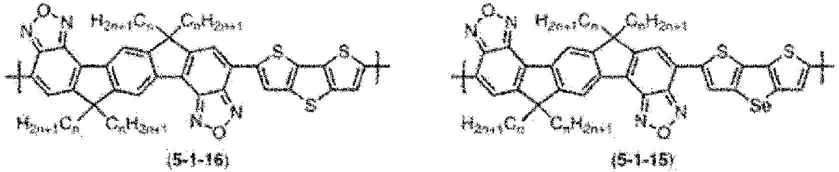

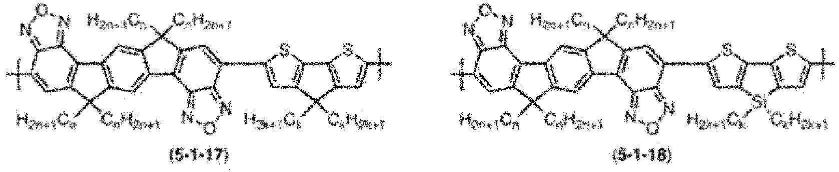

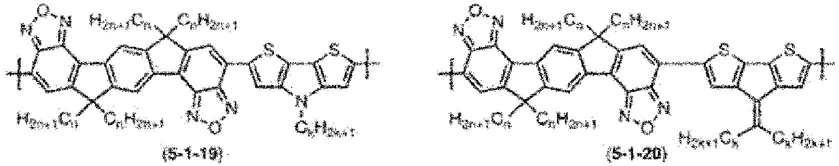

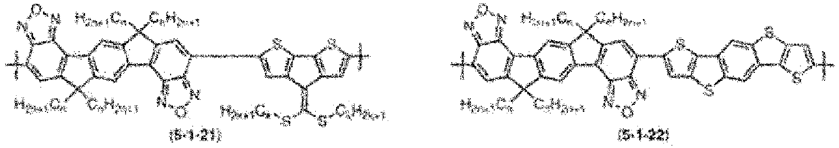

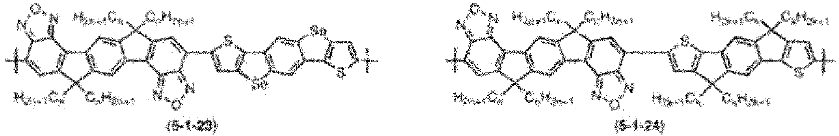

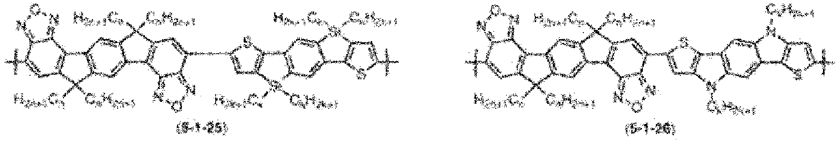

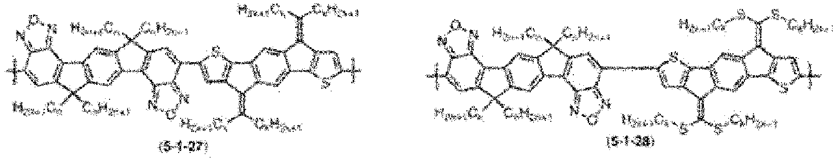

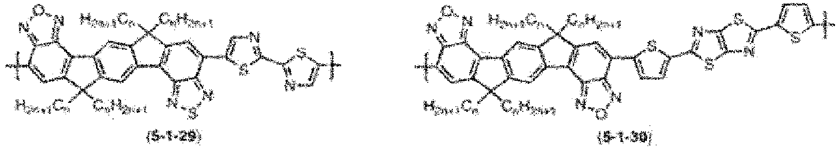

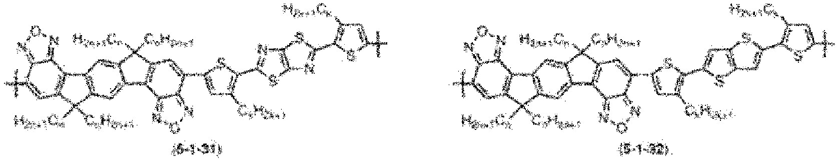

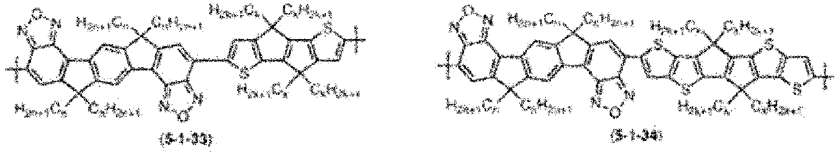

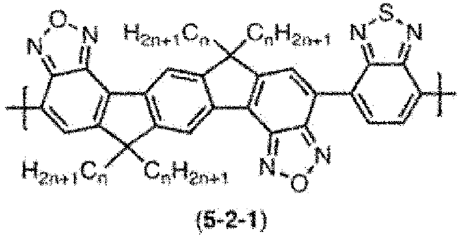

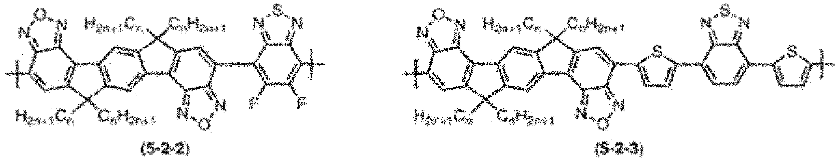

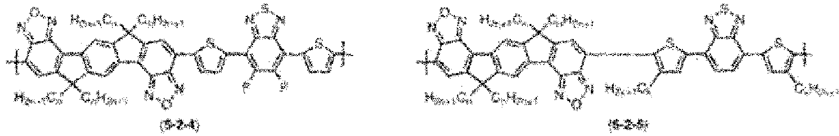

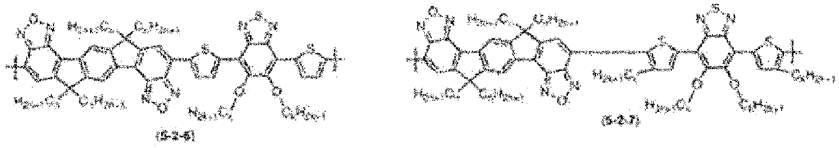

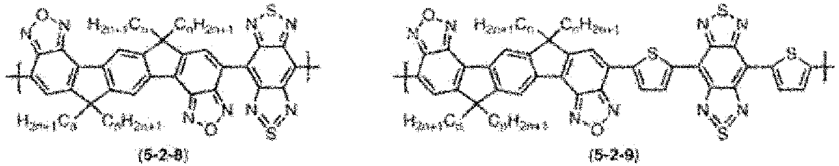

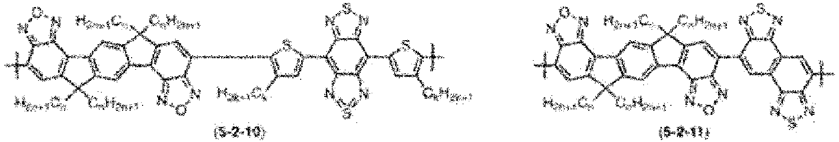

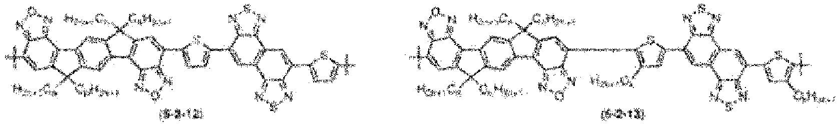

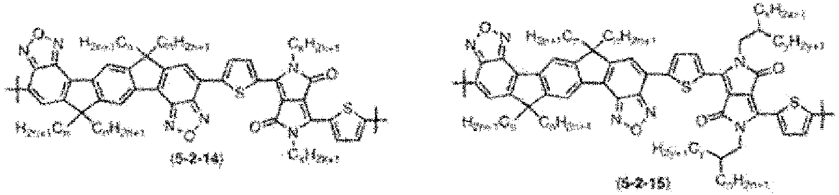

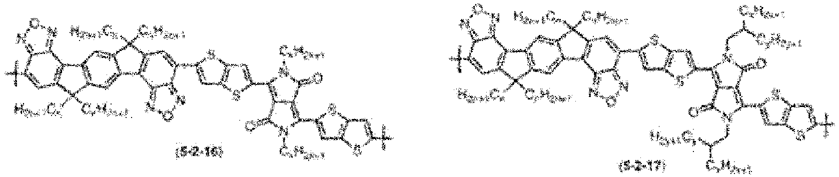

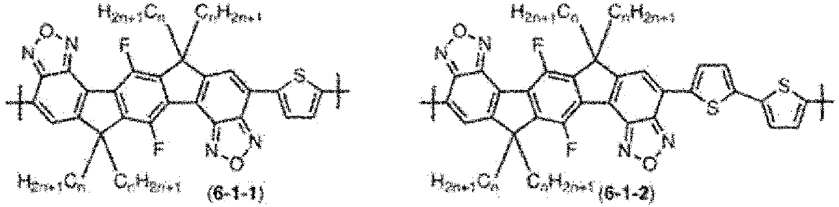

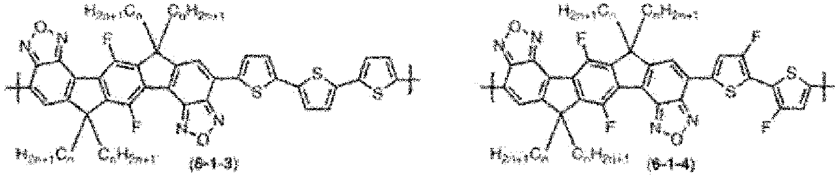

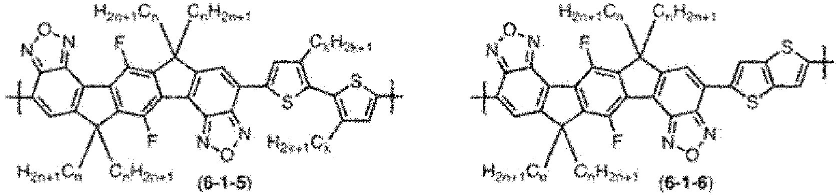

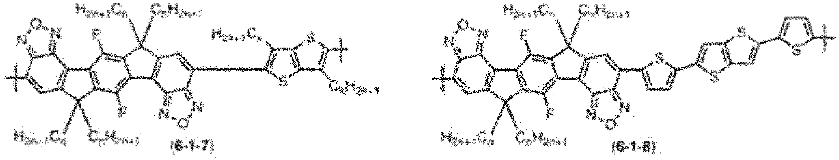

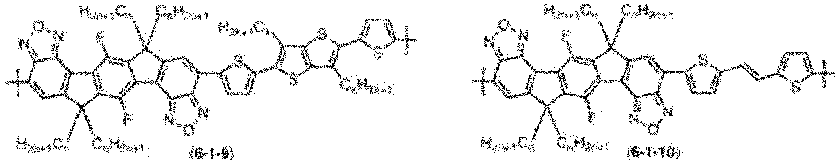

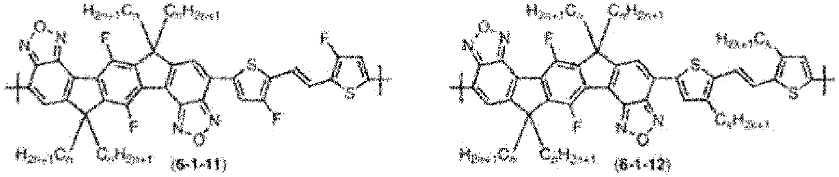

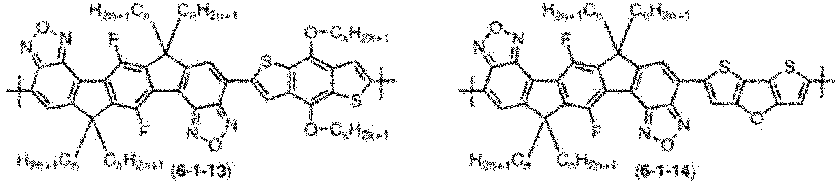

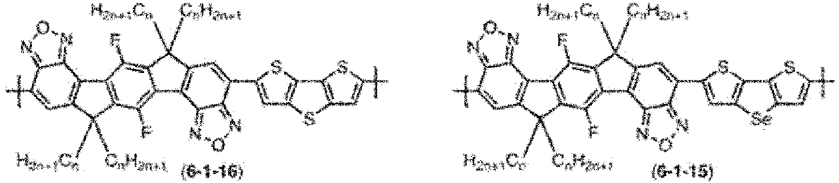

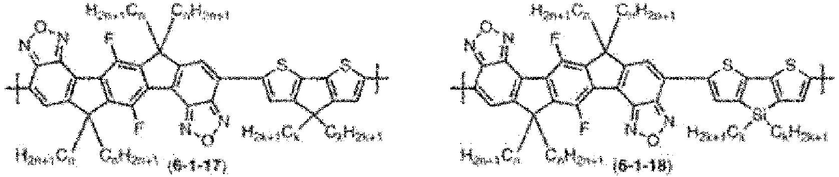

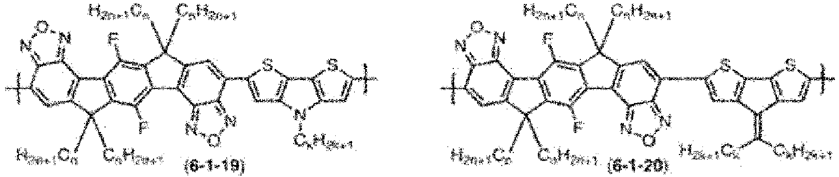

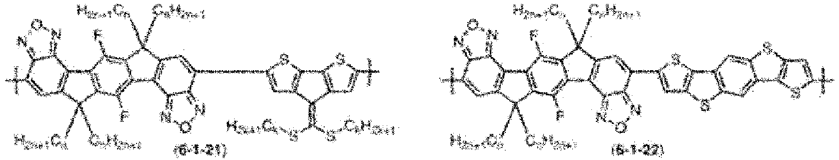

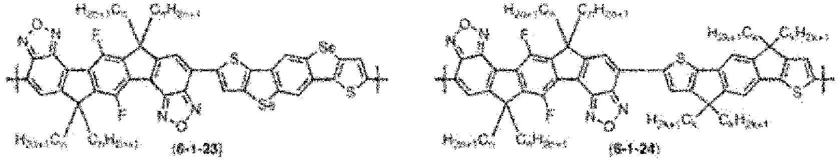

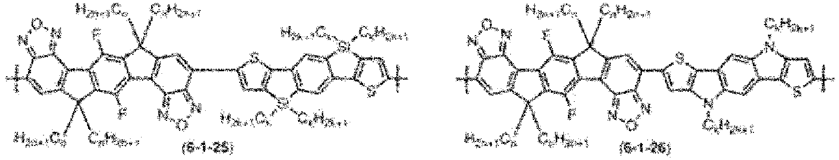

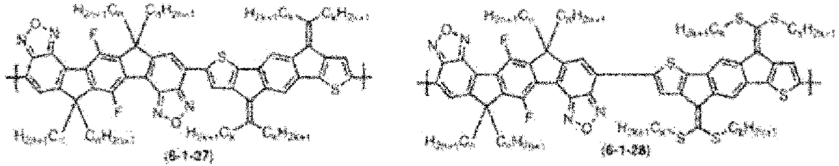

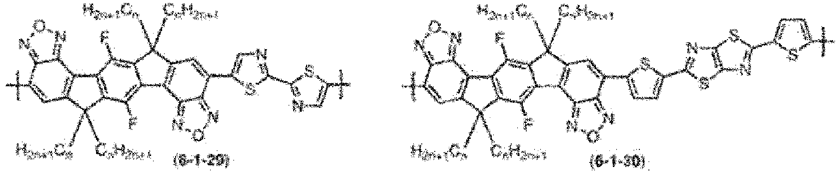

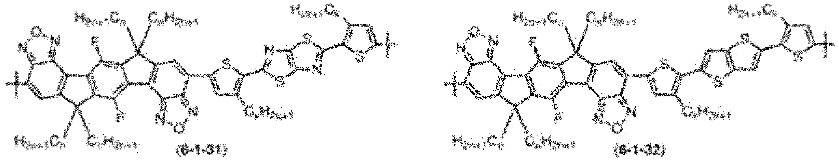

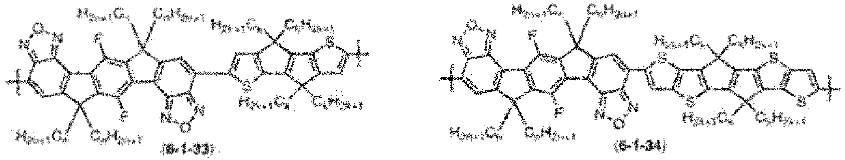

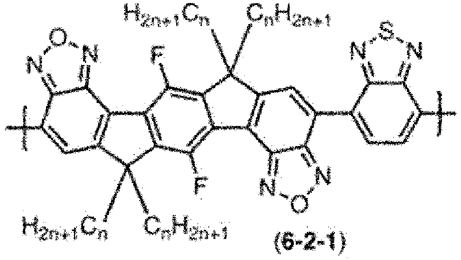

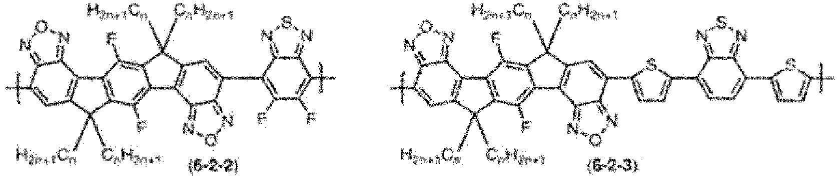

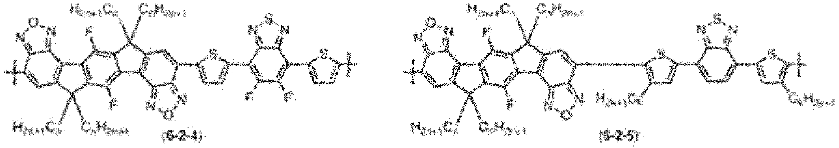

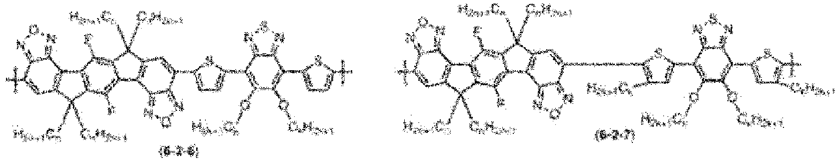

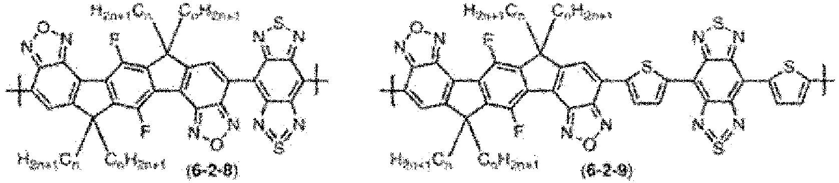

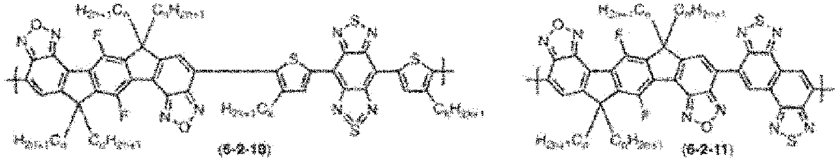

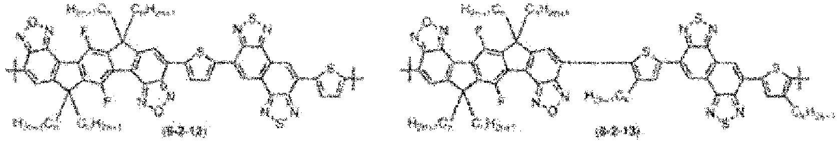

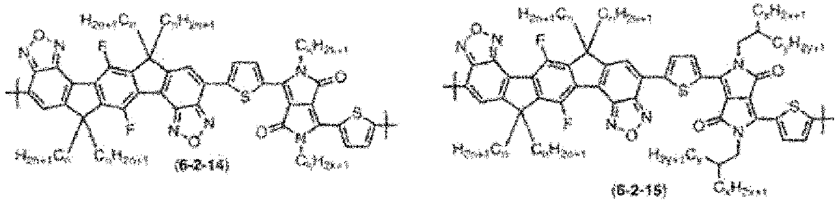

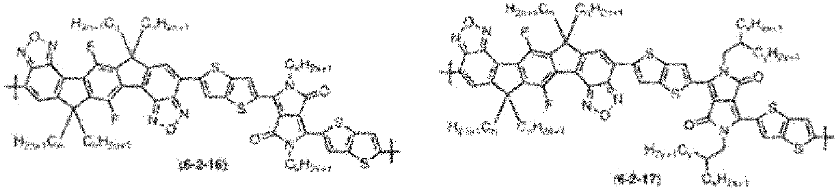

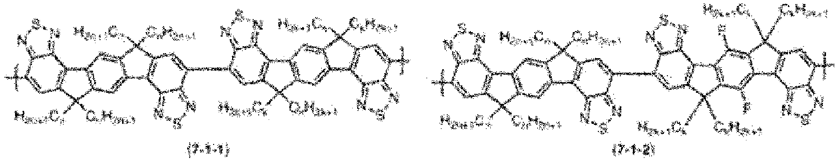

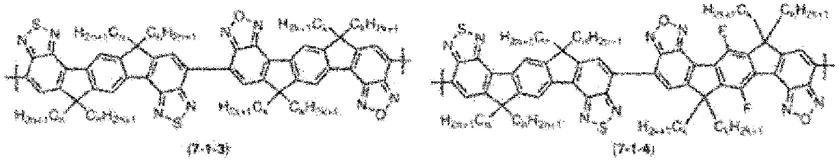

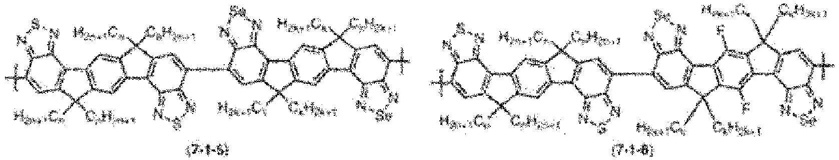

In the formulae, CₙH₂ₙ₊₁, CₖH₂ₖ₊₁, CₓH₂ₓ₊₁, and C_{y}H_{2y+1} each represent a linear alkyl group.

From the viewpoint of improving film formability of the conjugated polymer and increasing carrier mobility of the organic transistor, the structural unit represented by general formula (24) is preferably any structural unit represented by formula (3-1-2), formula (3-1-4), formula (3-1-6), formula (3-1-8), formula (3-1-10), formula (3-1-11), formula (3-1-13), formula (3-1-17), formula (3-1-18), formula (3-1-20), formula (3-1-21), formula (3-1-24), formula (3-1-25), formula (3-1-27), formula (3-1-28), formula (3-1-32), formula (3-1-33), formula (3-1-34), formula (3-1-35), formula (3-1-2-n6) to formula (3-1-2-n19), formula (3-1-4-n6), formula (3-1-4-n16), formula (3-1-6-n6) to formula (3-1-6-n19), formula (3-1-8-n6), formula (3-1-8-n16), formula (3-1-10-n6), formula (3-1-10-n16), formula (3-1-11-n6), formula (3-1-11-n16), formula (3-1-12-n6-k1), formula (3-1-12-n16-k1), formula (3-1-12-n16-k4), formula (3-1-12-n16-k4), formula (3-1-12-n1-k6), formula (3-1-12-n16-k6), formula (3-1-13-n6-k1) to formula (3-1-13-n16-k1), formula (3-1-13-n6-k8) to formula (3-1-13-n16-k8), formula (3-1-17-n6-k1) to formula (3-1-17-n16-k1), formula (3-1-17-n6-k6) to formula (3-1-17-n16-k6), formula (3-1-18-n6-k1), formula (3-1-18-n16-k1), formula (3-1-18-n6-k6), formula (3-1-18-n16-k6), formula (3-1-20-n6-k1) to formula (3-1-20-n16-k1), formula (3-1-20-n6-k3) to formula (3-1-20-n16-k3), formula (3-1-20-n6-k6) to formula (3-1-20-n16-k6), formula (3-1-21-n6-k1) to formula (3-1-21-n16-k1), formula (3-1-21-n6-k3) to formula (3-1-21-n16-k3), formula (3-1-21-n6-k6) to formula (3-1-21-n16-k6), formula (3-1-24-n6-k1) to formula (3-1-24-n16-k1), formula (3-1-24-n6-k6) to formula (3-1-24-n16-k6), formula (3-1-25-n6-k1), formula (3-1-25-n16-k1), formula (3-1-25-n6-k6), formula (3-1-25-n16-k6), formula (3-1-27-n6-k1), formula (3-1-27-n16-k1), formula (3-1-27-n6-k6), formula (3-1-27-n16-k6), formula (3-1-28-n6-k1), formula (3-1-28-n16-k1), formula (3-1-28-n6-k6), formula (3-1-28-n16-k6), formula (3-1-32-n6-k1), formula (3-1-32-n16-k1), formula (3-1-32-n6-k6), formula (3-1-32-n16-k6), formula (3-1-33-n6-k1), formula (3-1-33-n16-k1), formula (3-1-33-n6-k6), formula (3-1-33-n16-k6), formula (3-1-34-n6-k1), formula (3-1-34-n16-k1), formula (3-1-34-n6-k6), formula (3-1-34-n16-k6), formula (3-1-34-n6-k10), formula (3-1-34-n16-k10), formula (3-2-3), formula (3-2-4), formula (3-2-9), formula (3-2-12), formula (3-2-14), formula (3-2-15), formula (3-2-14-n6-k1) to formula (3-2-14-n16-k1), formula (3-2-14-n6-k8) to formula (3-2-14-n16-k8), formula (3-2-15-n6-x4-y2), formula (3-2-15-n16-x4-y2), formula (3-2-15-n6-x8-y6), formula (3-2-15-n16-x8-y6), formula (3-2-15-n6-x10-y8), formula (3-2-15-n16-x10-y8), formula (4-1-2), formula (4-1-4), formula (4-1-6), formula (4-1-8), formula (4-1-10), formula (4-1-11), formula (4-1-13), formula (4-1-17), formula (4-1-18), formula (4-1-20), formula (4-1-21), formula (4-1-24), formula (4-1-25), formula (4-1-27), formula (4-1-28), formula (4-1-34), formula (4-2-3), formula (4-2-4), formula (4-2-9), formula (4-2-12), formula (4-2-14), or formula (4-2-15), more preferably any structural unit represented by formula (3-1-2), formula (3-1-6), formula (3-1-10), formula (3-1-13), formula (3-1-17), formula (3-1-20), formula (3-1-21), formula (3-1-24), formula (3-1-25), formula (3-1-2-n6) to formula (3-1-2-n19), formula (3-1-6-n6) to formula (3-1-6-n19), formula (3-1-10-n6), formula (3-1-10-n16), formula (3-1-12-n6-k1), formula (3-1-12-n16-k1), formula (3-1-12-n16-k4), formula (3-1-12-n16-k4), formula (3-1-12-n1-k6), formula (3-1-12-n16-k6), formula (3-1-13-n6-k1) to formula (3-1-13-n16-k1), formula (3-1-13-n6-k8) to formula (3-1-13-n16-k8), formula (3-1-17-n6-k1) to formula (3-1-17-n16-k1), formula (3-1-17-n6-k6) to formula (3-1-17-n16-k6), formula (3-1-20-n6-k1) to formula (3-1-20-n16-k1), formula (3-1-20-n6-k3) to formula (3-1-20-n16-k3), formula (3-1-20-n6-k6) to formula (3-1-20-n16-k6), formula (3-1-21-n6-k1) to formula (3-1-21-n16-k1), formula (3-1-21-n6-k3) to formula (3-1-21-n16-k3), formula (3-1-21-n6-k6) to formula (3-1-21-n16-k6), formula (3-1-24-n6-k1) to formula (3-1-24-n16-k1), formula (3-1-24-n6-k6) to formula (3-1-24-n16-k6), formula (3-1-35-n6-k1), formula (3-1-35-n8-k1), formula (3-1-35-n10-k1), formula (3-1-35-n12-k1), formula (3-1-35-n14-k1), formula (3-1-35-n16-k1), formula (3-2-14), formula (3-2-15), formula (3-2-14-n6-k1) to formula (3-2-14-n16-k1), formula (3-2-14-n6-k8) to formula (3-2-14-n16-k8), formula (3-2-15-n6-x4-y2), formula (3-2-15-n16-x4-y2), formula (3-2-15-n6-x8-y6), formula (3-2-15-n16-x8-y6), formula (3-2-15-n6-x10-y8), or formula (3-2-15-n16-x10-y8), further preferably any structural unit represented by formula (3-1-2-n6) to formula (3-1-2-n19), formula (3-1-6-n6) to formula (3-1-6-n19), formula (3-1-10-n6), formula (3-1-10-n16), formula (3-1-12-n6-k1), formula (3-1-12-n16-k1), formula (3-1-12-n1-k4), formula (3-1-12-n16-k4), formula (3-1-12-n16-k6), formula (3-1-12-n16-k6), formula (3-1-13-n6-k8) to formula (3-1-13-n16-k8), formula (3-1-17-n6-k1) to formula (3-1-17-n16-k1), formula (3-1-20-n6-k1) to formula (3-1-20-n16-k1), formula (3-1-21-n6-k1) to formula (3-1-21-n16-k1), formula (3-1-21-n6-k3) to formula (3-1-21-n16-k3), formula (3-1-24-n6-k1) to formula (3-1-24-n16-k1), formula (3-1-35-n6-k1), formula (3-1-35-n8-k1), formula (3-1-35-n10-k1), formula (3-1-35-n12-k1), formula (3-1-35-n14-k1), formula (3-1-35-n16-k1), formula (3-2-14-n6-k1) to formula (3-2-14-n16-k1), formula (3-2-14-n6-k8) to formula (3-2-14-n16-k8), formula (3-2-15-n6-x10-y8), or formula (3-2-15-n16-x10-y8), and especially preferably any structural unit represented by formula (3-1-2-n9) to formula (3-1-2-n12), formula (3-1-2-n16), formula (3-1-6-n10), formula (3-1-6-n16), formula (3-1-6-n18), formula (3-1-10-n16), formula (3-1-13-n16-k4), formula (3-1-13-n16-k6), formula (3-1-13-n10-k8), formula (3-1-13-n16-k8), formula (3-1-17-n10-k1), formula (3-1-17-n16-k1), formula (3-1-20-n16-k1), formula (3-1-21-n16-k1), formula (3-1-21-n16-k3), formula (3-1-24-n16-k1), formula (3-1-35-n16-k1), formula (3-2-14-n6-k1), formula (3-2-14-n6-k8), or formula (3-2-15-n16-x10-y8).

The conjugated polymer may include other structural units in addition to the structural unit represented by general formula (2) and the structural unit represented by general formula (3), within a range that does not impair the effects of the present invention. A total content of the structural unit represented by general formula (2) and the structural unit represented by general formula (3) in the conjugated polymer is preferably not less than 90% by mass, and more preferably not less than 95% by mass.

A terminal structure of the conjugated polymer is not particularly limited, and examples thereof include a hydrogen atom, boron-containing groups such as a dihydroxyboryl group and a dialkoxyboryl group, tin-containing groups such as a trimethylstannyl group and a tributylstannyl group, halogen atoms such as a chlorine atom, a bromine atom, and an iodine atom, and aromatic groups such as a phenyl group and a thienyl group, and structures at both terminals may be the same or different.

A weight-average molecular weight (Mw) of the conjugated polymer is preferably 3000 to 10000000, more preferably 3000 to 1000000, and further preferably 3000 to 500000.

A molecular weight distribution (PDI) of the conjugated polymer is preferably 1.05 to 20.0, more preferably 1.2 to 10.0, further preferably 1.2 to 9.0, and especially preferably 1.2 to 8.0.

A molar ratio of the structural unit represented by general formula (2) and the structural unit represented by general formula (3) in the conjugated polymer (structural unit represented by general formula (2) : structural unit represented by general formula (3)) is not particularly limited, and is preferably in a range of 10:1 to 1:10, more preferably in a range of 5:1 to 1:5, further preferably in a range of 2:1 to 1:2, especially preferably in a range of 1.2:1 to 1:1.2, and particularly preferably 1:1.

Next, the following description will discuss a method for producing a conjugated polymer. The method for producing a conjugated polymer is as described below.

The production method is a method for producing a conjugated polymer by coupling a compound (monomer) represented by general formula (mono-hal) with a compound (monomer) represented by general formula (mono-X-Sn) in the presence of a transition metal catalyst. 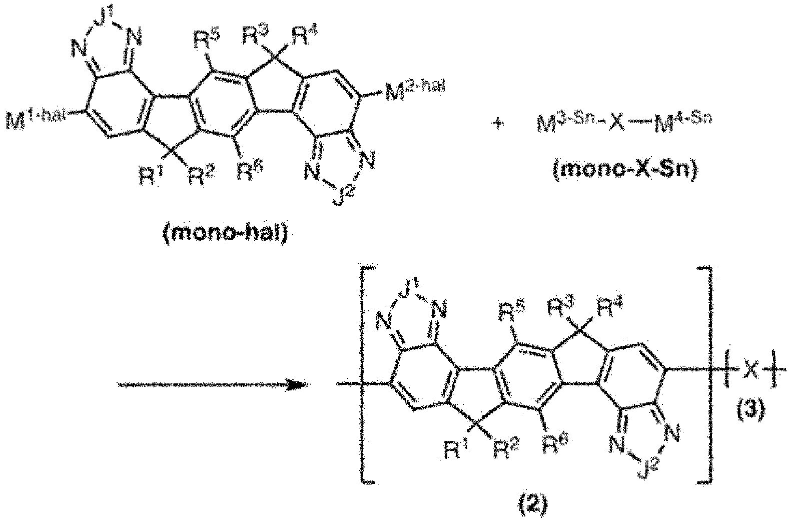 where R¹, R², R³, R⁴, R⁵, R⁶, J¹, J², and X have the same meanings as described above. M^{1-hal} and M^{2-hal} each independently represent a halogen atom, and M^{3-Sn} and M^{4-Sn} each independently represent a tin-containing group.

As the halogen atom represented by M^{1-hal} and M^{2-hal}, from the viewpoint of improving production efficiency of the conjugated polymer, a chlorine atom, a bromine atom, or an iodine atom is preferable, a bromine atom or an iodine atom is more preferable, and a bromine atom is further preferable.

Examples of the tin-containing group represented by M^{3-Sn} and M^{4-Sn} include a trialkylstannyl group, a dialkylarylstannyl group, an alkyldiarylstannyl group, a triarylstannyl group, and the like. From the viewpoint of improving production efficiency of the conjugated polymer, a trialkylstannyl group or a triarylstannyl group is preferable, any group represented by general formula (13-1) to general formula (13-5) is more preferable, and a group represented by general formula (13-1) is further preferable. In this specification, Me, Et, Pr, Bu, and Ph represent a methyl group, an ethyl group, a propyl group, a butyl group, and a phenyl group, respectively.

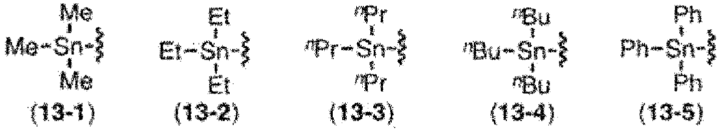

It is essential that the production method is carried out in the presence of a transition metal catalyst, and examples of the transition metal catalyst that can be used include a palladium catalyst, a nickel catalyst, a platinum catalyst, and the like. As the transition metal catalyst, "metal", "supported metal", "metal salts such as chlorides, bromides, iodides, nitrates, sulfates, carbonates, oxalates, acetates, or oxides of a metal", or "complex compounds such as olefin complexes, phosphine complexes, amide complexes, amine complexes, carbene complexes, or acetylacetonato complexes" can be used. From the viewpoint of good reaction yield, use of a palladium catalyst or a nickel catalyst is preferable, and use of a palladium catalyst is more preferable. Further, a combination of these metals, supported metals, metal salts, and complex compounds with a tertiary phosphorus compound, a carbene compound, or the like can also be used.

The palladium catalyst is not particularly limited, and examples thereof include palladium metals such as palladium black and palladium sponge, and supported palladium metals such as palladium/alumina, palladium/carbon, palladium/silica, and palladium/Y-type zeolite. Examples of the palladium catalyst also include metal salts such as palladium chloride, palladium bromide, palladium iodide, palladium acetate, palladium trifluoroacetate, and palladium nitrate, π-allylpalladium chloride dimer, palladium acetylacetonate, dichlorobis(acetonitrile)palladium, dichlorobis(benzonitrile)palladium, bis(dibenzylideneacetone)palladium, tris(dibenzylideneacetone)dipalladium, dichlorodiamminepalladium, dichlorobis(triphenylphosphine)palladium, dichlorobis(tricyclohexylphosphine)palladium, tetrakis(triphenylphosphine)palladium, dichloro[1,2-bis(diphenylphosphino)ethane]palladium, dichloro[1,3-bis(diphenylphosphino)propane]palladium, dichloro[1,4-bis(diphenylphosphino)butane]palladium, dichloro[1,1'-bis(diphenylphosphino)ferrocene]palladium, bis(tri-tert-butylphosphine)palladium, bis(tricyclohexylphosphine)palladium, [1,3-bis(2,6-diisopropylphenyl)imidazole-2-ylidene](3-chloropyridyl)palladium(II) dichloride (Pd-PEPPSI-IPent), [1,3-bis(2,6-di-3-pentylphenyl)imidazole-2-ylidene](3-chloropyridyl)palladium(II) dichloride (Pd-PEPPSI-IPr), [1,3-bis(2,6-diisopropylphenyl)imidazolidine-2-ylidene](3-chloropyridyl)palladium(II) dichloride (Pd-PEPPSI-SIPr), and the like.

Among these palladium catalysts, from the viewpoint of good reaction yield, it is preferable to use palladium acetate, palladium acetylacetonate, bis(dibenzylideneacetone)palladium, tris(dibenzylideneacetone)dipalladium, tetrakis(triphenylphosphine)palladium (Pd(PPh₃)₄), or bis(tri-tert-butylphosphine)palladium as the palladium catalyst.

The nickel catalyst is not particularly limited, and specific examples thereof include nickel(II) chloride, bis(triphenylphosphine)nickel(II) dichloride, bis(2,4-pentanedionato)nickel(II) hydrate, bis(1,5-cyclooctadiene)nickel(0), dichloro(1,1'-bis(diphenylphosphino)ethane)nickel, dichloro(1,1'-bis(diphenylphosphino)propane)nickel, [1,3-bis(2,6-diisopropylphenyl)imidazole-2-ylidene]triphenylphosphinenickel(II) dichloride, and the like.

These palladium catalysts or nickel catalysts may be used alone, or may be used in combination with a tertiary phosphorus compound or a carbene compound. Examples of the tertiary phosphorus compound that can be used include triphenylphosphine, trimethylphosphine, triethylphosphine, tributylphosphine, tri(tert-butyl)phosphine, tri-tert-butylphosphonium tetrafluoroborate, tricyclohexylphosphine, tri(o-tolyl)phosphine, tris(2-methoxyphenyl)phosphine, trioctylphosphine, 9,9-dimethyl-4,5-bis(diphenylphosphino)xanthene, 2-(di-tert-butylphosphino)biphenyl, 2-(dicyclohexylphosphino)biphenyl, 1,2-bis(diphenylphosphino)ethane, 1,3-bis(diphenylphosphino)propane, 1,4-bis(diphenylphosphino)butane, 1,1'-bis(diphenylphosphino)ferrocene, tert-butyldiphenylphosphine, 2-(diphenylphosphino)-2'-(N,N-dimethylamino)biphenyl, bis(diphenylphosphino)methane, 1,4-bis(diphenylphosphino)butane, tri(2-furyl)phosphine, tris(2,5-xylyl)phosphine, (R)-(+)-2,2'-bis(diphenylphosphino)-1,1'-binaphthyl, (S)-(-)-2,2'-bis(diphenylphosphino)-1,1'-binaphthyl, (±)-2,2'-bis(diphenylphosphino)-1,1'-binaphthyl, and the like. Examples of the carbene compound that can be used include 1,3-bis(2,6-diisopropylphenyl)imidazole-2-ylidene, 1,3-bis(2,6-diisopropylphenyl)imidazolidine-2-ylidene, 1,3-di-tert-butylimidazole-2-ylidene, 1,3-dimesitylimidazole-2-ylidene, and the like.

From the viewpoint of good reaction yield, as the tertiary phosphorus compound, triphenylphosphine, tri(tert-butyl)phosphine, tricyclohexylphosphine, or tri(o-tolyl)phosphine is preferably used.

The molar ratio of the tertiary phosphorus compound and the transition metal catalyst (tertiary phosphorus compound : transition metal catalyst) is preferably in a range of 1:10 to 10:1, and more preferably in a range of 1:5 to 5:1 from the viewpoint of good reaction yield.

An amount of the transition metal catalyst used is not particularly limited, and from the viewpoint of good reaction yield, 0.001 to 50 mol% is preferable and 0.1 to 20 mol% is more preferable, relative to the compound represented by general formula (mono-hal).

The production method may also use a promoter. The promoter is not particularly limited, and specific examples thereof include monovalent or divalent copper salts such as copper fluoride, copper chloride, copper bromide, copper iodide, and copper oxide.

The production method can be carried out in a solvent. The solvent that can be used is not particularly limited as long as the solvent does not inhibit the reaction, and examples thereof include aliphatic hydrocarbon solvents such as hexane, heptane, decane, and tridecane; ether solvents such as diisopropyl ether, dibutyl ether, cyclopentyl methyl ether (CPME), tetrahydrofuran (THF), 2-methyltetrahydrofuran, 1,4-dioxane, and 1,2-dimethoxyethane; aromatic hydrocarbon solvents such as benzene, toluene, xylene, mesitylene, and tetralin; carbonate ester solvents such as ethylene carbonate, propylene carbonate, dimethyl carbonate, diethyl carbonate, ethyl methyl carbonate, and 4-fluoroethylene carbonate; ester solvents such as ethyl acetate, butyl acetate, methyl propionate, ethyl propionate, methyl butyrate, and γ-lactone; amide solvents such as N,N-dimethylformamide (DMF), dimethylacetamide (DMAc), and N-methylpyrrolidone (NMP); urea solvents such as N,N,N',N'-tetramethylurea (TMU) and N,N'-dimethylpropyleneurea (DMPU); sulfoxide solvents such as dimethyl sulfoxide (DMSO); alcohol solvents such as methanol, ethanol, 2-propanol, butanol, octanol, benzyl alcohol, ethylene glycol, propylene glycol, diethylene glycol, triethylene glycol, and 2,2,2-trifluoroethanol; halogen solvents such as chloroform, dichloromethane, carbon tetrachloride, 1,2-dichloroethane, chlorobenzene, and o-dichlorobenzene (DCB); fluorine solvents such as bis(2,2,2-trifluoroethyl)=N,N-diisopropylphosphoroamidate (PF-37) and tris(2,2,2-trifluoroethyl) phosphate (TFEP); nitromethane; water; and the like, and these may be used as a mixture at any ratio. From the viewpoint of good reaction yield, an aliphatic hydrocarbon solvent, an aromatic hydrocarbon solvent, a halogen solvent, an ether solvent, an amide solvent, a sulfoxide solvent, a fluorine solvent, a mixed solvent of an aromatic hydrocarbon solvent and water, a mixed solvent of a halogen solvent and water, a mixed solvent of an ether solvent and water, a mixed solvent of an aromatic hydrocarbon solvent and a sulfoxide solvent, a mixed solvent of a halogen solvent and a sulfoxide solvent, a mixed solvent of an ether solvent and a sulfoxide solvent, a mixed solvent of an aromatic hydrocarbon solvent and a fluorine solvent, a mixed solvent of a halogen solvent and a fluorine solvent, and a mixed solvent of an ether solvent and a fluorine solvent are preferable, and an aliphatic hydrocarbon solvent, an aromatic hydrocarbon solvent, a halogen solvent, an ether solvent, a mixed solvent of an aromatic hydrocarbon solvent and a fluorine solvent, a mixed solvent of a halogen solvent and a fluorine solvent, and a mixed solvent of an ether solvent and a fluorine solvent are more preferable, and tetralin, toluene, chlorobenzene, o-DCB, and THF are further preferable.

An amount of the solvent used is not particularly limited, and is preferably in a range of 0.001 to 100 mL/mg relative to the weight of the compound represented by general formula (mono-hal).

The production method can be carried out at a temperature appropriately selected from 0°C to 240°C, and from the viewpoint of good reaction yield, preferably at a temperature appropriately selected from 70°C to 220°C, and more preferably at a temperature appropriately selected from 100°C to 200°C.

The production method can also be carried out using a microwave reaction device.

The production method is preferably carried out under an inert gas atmosphere such as argon gas or nitrogen gas, or under reduced pressure.

A reaction time varies depending on the compound used (the compound represented by general formula (mono-hal) or general formula (mono-X-Sn)), as well as the solvent and the reaction temperature, and is preferably 0.1 to 100 hours, and more preferably 1 to 90 hours.

The conjugated polymer can be obtained by carrying out an ordinary treatment after completion of the production method. If necessary, purification may be carried out using, as appropriate, general means used by a person skilled in the art for purification of polymer compounds, such as washing, precipitation, filtration, dialysis, column chromatography, preparative HPLC, and Soxhlet extraction.

For the purpose of improving carrier mobility or solubility in the resultant conjugated polymer, an organoboron compound or an organotin compound may be added during or after the reaction to produce a conjugated polymer in which a functional group such as a thienyl group or a phenyl group has been introduced into an end of the conjugated polymer. The functional group may be introduced by combining known methods, and for example, can be introduced according to a method disclosed in a Non-Patent Literature (Macromolecules, vol. 48, pp. 6994-7006, 2015, and the like).

The compound represented by general formula (mono-X-Sn) used in the production method is not limited in acquisition method thereof, and can be produced, for example, with reference to methods disclosed in Non-Patent Literatures (Journal of the American Chemical Society, vol. 134, pp. 3498-3507, 2012, and Nature Chem, vol. 11, pp. 271-277, 2019, and the like). A commercially available product may also be used.

The organic semiconductor film may also contain an additive in order to adjust a surface energy. Examples of the additive include a silicone-based surfactant, a fluorine-based surfactant, and a hydrocarbon-based surfactant, among which a silicone-based surfactant is preferable.

The silicone-based surfactant is not particularly limited, and examples thereof include polydimethylsiloxane, polymethylphenylsiloxane, polyether-modified polydimethylsiloxane, polyether ester-modified polydimethylsiloxane, hydroxyl group-containing polyether-modified polydimethylsiloxane, acrylic group-containing polyether-modified polydimethylsiloxane, acrylic group-containing polyester-modified polydimethylsiloxane, perfluoropolyether-modified polydimethylsiloxane, perfluoropolyester-modified polydimethylsiloxane, polyether-modified polymethylphenylsiloxane, polyether ester-modified polymethylphenylsiloxane, hydroxyl group-containing polyether-modified polymethylphenylsiloxane, acrylic group-containing polyether-modified polymethylphenylsiloxane, acrylic group-containing polyester-modified polymethylphenylsiloxane, perfluoropolyether-modified polymethylphenylsiloxane, perfluoropolyester-modified polymethylphenylsiloxane, silicone-modified acrylic compounds, and the like, and polydimethylsiloxane and polymethylphenylsiloxane are preferable, and polymethylphenylsiloxane is more preferable.

Examples of the fluorine-based surfactant include Surflon S-111, S-112, S-113, S-121, S-131, S-132, S-141, and S-145 (all manufactured by Asahi Glass Co., Ltd.); Fluorad FC-93, FC-95, FC-98, FC-129, FC-135, FC-170C, FC-430, FC-431, and FC-4430 (all manufactured by Sumitomo 3M Ltd.); MEGAFAC F-470, F-1405, and F-474 (all manufactured by Dainippon Ink and Chemicals, Inc.); Zonyl FS-300, FSN, FSN-100, and FSO (all manufactured by DuPont); and Eftop EF-351, EF-352, EF-801, and EF-802 (all manufactured by Jemco). Among these, Zonyl FS-300, FSN, FSN-100, and FSO (all manufactured by DuPont), which are favorable in terms of reliability and improvement in color development, are particularly suitable.

Examples of the silicone-based surfactant include modified silicones KF-351A, KF-353A, KF354L, KF355A, KF-615A, KF-640, KF-642, KF-643, and KF-6011 (all manufactured by Shin-Etsu Chemical Co., Ltd.); and silicones FZ-77, FZ-2104, FZ-2105, and L-7604 (all manufactured by Dow Corning Toray Co., Ltd.). Among these, KF-355A, KF-640, KF-642, and Kf-643 (all manufactured by Shin-Etsu Chemical Co., Ltd.), which are favorable in terms of reliability and improvement in color development, are particularly suitable.

The organic semiconductor film in the organic transistor in accordance with an aspect of the present invention is obtained by forming a film using a composition for film formation containing a conjugated polymer and a solvent.

The solvent is not particularly limited as long as the solvent can dissolve or disperse the conjugated polymer, and examples thereof include ether solvents such as diisopropyl ether, dibutyl ether, CPME, THF, 2-methyltetrahydrofuran, 1,4-dioxane, and 1,2-dimethoxyethane; aromatic hydrocarbon solvents such as benzene, toluene, xylene, mesitylene, and tetralin; carbonate ester solvents such as ethylene carbonate, propylene carbonate, dimethyl carbonate, diethyl carbonate, ethyl methyl carbonate, and 4-fluoroethylene carbonate; ester solvents such as ethyl acetate, butyl acetate, methyl propionate, ethyl propionate, methyl butyrate, and γ-lactone; amide solvents such as DMF, DMAc, and NMP; urea solvents such as TMU and DMPU; sulfoxide solvents such as DMSO; alcohol solvents such as methanol, ethanol, 2-propanol, butanol, octanol, benzyl alcohol, ethylene glycol, propylene glycol, diethylene glycol, triethylene glycol, and 2,2,2-trifluoroethanol; halogen solvents such as chloroform, dichloromethane, carbon tetrachloride, 1,2-dichloroethane, chlorobenzene, and o-DCB; nitromethane; and water, and these may be used as a mixture at any ratio. Among these, from the viewpoint of having high boiling points and volatilizing gently, aromatic hydrocarbons and halogen solvents are preferable, and toluene, xylene, mesitylene, cyclohexylbenzene, tetralin, 3,4-dimethylanisole, chlorobenzene, and o-DCB are more preferable.

An amount of the solvent used is not particularly limited, and it is more preferable to add the solvent so that a concentration of the conjugated polymer is 0.001 to 95% by weight and is a concentration appropriately selected from 0.01 to 30% by weight.

The composition for film formation is obtained by dissolving or dispersing the conjugated polymer in a solvent. As a method for dissolving or dispersing the conjugated polymer in a solvent, methods well known to a person skilled in the art, such as, for example, stirring, shaking, and ball milling, can be used. At this time, heating may be carried out.

The composition for film formation may contain a binder for improving film formability. Examples of such a binder include polymers such as polystyrene, poly-α-methylstyrene, polyvinylnaphthalene, poly(ethylene-co-norbornene), polymethyl methacrylate, polytriarylamine, and poly(9,9-dioctylfluorene-co-dimethyltriphenylamine). A concentration of the binder is not particularly limited, and is preferably 0.1 to 10.0% by weight from the viewpoint of good coatability.

The method for forming a film using the composition for film formation is not particularly limited, and examples thereof include: simple coating methods such as spin coating, drop casting, dip coating, and cast coating; printing methods such as dispenser printing, ink jet printing, slit coating, blade coating, flexographic printing, screen printing, gravure printing, and offset printing; and the like. Among these, spin coating, drop casting, and ink jet printing are preferable from the viewpoint of efficient film formation.

A thickness of the organic semiconductor film is not particularly limited, and is preferably 1 nm to 1000 nm, and more preferably 10 nm to 500 nm, from the viewpoint of increasing carrier mobility.

The organic transistor in accordance with an aspect of the present invention may include constituent units other than to the gate electrode, the gate insulating film, the source electrode, the drain electrode, and the organic semiconductor film, and examples of such constituent units include a substrate, a base material, a bank material, a protective film material, and the like.

Examples of the substrate include: plastic substrates such as polyethylene terephthalate, polyethylene naphthalate, polymethyl methacrylate, polymethyl acrylate, polyethylene, polypropylene, polystyrene, cyclic polyolefin, polyimide, polycarbonate, polyvinyl phenol, polyvinyl alcohol, poly(diisopropyl fumarate), poly(diethyl fumarate), poly(diisopropyl maleate), polyether sulfone, polyphenylene sulfide, cellulose triacetate, and the like; inorganic substrates such as glass, quartz, aluminum oxide, silicon, highly doped silicon, silicon oxide, tantalum dioxide, tantalum pentoxide, indium tin oxide, and the like; and metal substrates such as, for example, gold, copper, chromium, titanium, aluminum, and the like. Among these, from the viewpoint of good transistor performance, glass, silicon, and highly doped silicon are preferable, and glass is more preferable.

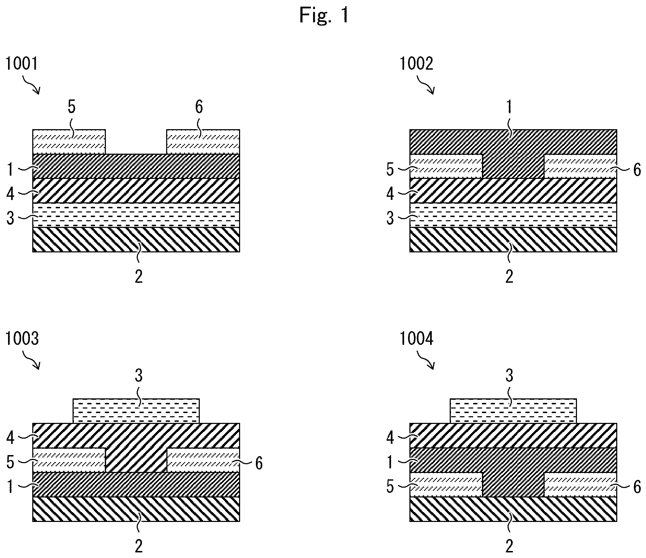

Fig. 1 illustrates a structure of elements included in the organic transistor of the present invention. The reference numeral 1001 indicates a transistor element of a bottom gate-top contact type. The reference numeral 1002 indicates a transistor element of a bottom gate-bottom contact type. The reference numeral 1003 indicates a transistor element of a top gate-top contact type. The reference numeral 1004 indicates a transistor element of a top gate-bottom contact type. The reference numeral 1 indicates an active layer (organic semiconductor film), the reference numeral 2 indicates a substrate, the reference numeral 3 indicates a gate electrode, the reference numeral 4 indicates a first gate insulating film, the reference numeral 5 indicates a source electrode, and the reference numeral 6 indicates a drain electrode.

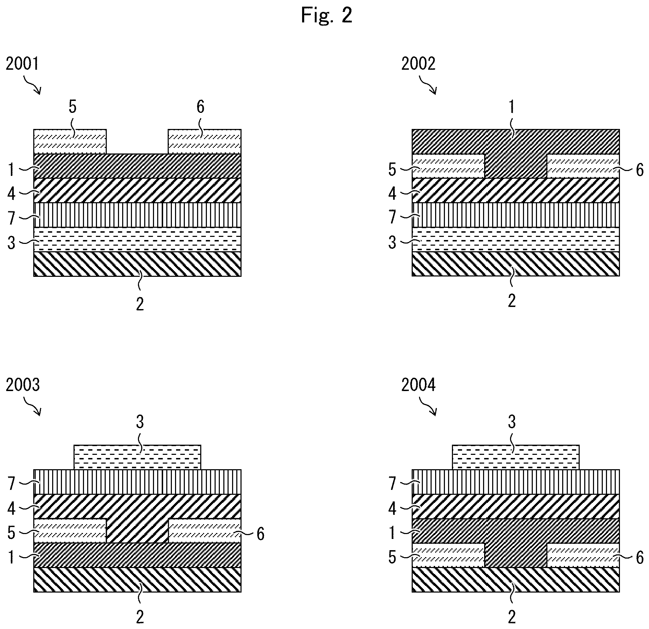

Fig. 2 illustrates a structure of elements included in the organic transistor of the present invention. The reference numeral 2001 indicates a transistor element of a bottom gate-top contact type. The reference numeral 2002 indicates a transistor element of a bottom gate-bottom contact type. The reference numeral 2003 indicates a transistor element of a top gate-top contact type. The reference numeral 2004 indicates a transistor element of a top gate-bottom contact type. The reference numeral 1 indicates an active layer (organic semiconductor film), the reference numeral 2 indicates a substrate, the reference numeral 3 indicates a gate electrode, the reference numeral 4 indicates a first gate insulating film, the reference numeral 5 indicates a source electrode, the reference numeral 6 indicates a drain electrode, and the reference numeral 7 indicates a second gate insulating film.

The organic transistor in accordance with an aspect of the present invention can be produced by a known production method. Examples of the known production method include a production method using a film-forming technique such as vacuum deposition, chemical vapor deposition (CVD), or spin coating.

### [Summary]

<1> An organic transistor including: a gate electrode, a source electrode, a drain electrode, and an organic semiconductor film; and
   a first gate insulating film in contact with the organic semiconductor film, or a first gate insulating film in contact with the organic semiconductor film and a second gate insulating film not in contact with the organic semiconductor film,
   the organic semiconductor film containing a conjugated polymer composed of a structural unit represented by general formula (2) below and a structural unit represented by general formula (3) below, and
   an interfacial energy between the first gate insulating film and the organic semiconductor film being 2.0 mJ/m² or less, 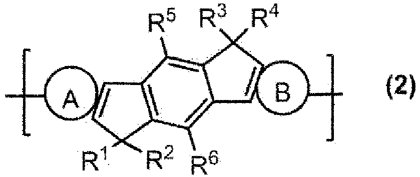
   where A and B each independently represent a monovalent aromatic ring linking group which may be substituted with an alkyl group having 1 to 50 carbon atoms or an alkoxy group having 1 to 50 carbon atoms, and R¹, R², R³, and R⁴ each independently represent an alkyl group having 1 to 50 carbon atoms, R¹ and R² may be bonded to each other to form a ring together with a carbon atom to which R¹ and R² are bonded, R³ and R⁴ may be bonded to each other to form a ring together with a carbon atom to which R³ and R⁴ are bonded, and R⁵ and R⁶ each independently represent a hydrogen atom, a fluorine atom, or an alkyl group having 1 to 50 carbon atoms, 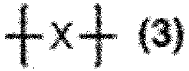
   where X represents a divalent heteroaromatic ring linking group which may be substituted with an alkyl group having 1 to 50 carbon atoms or an alkoxy group having 1 to 50 carbon atoms.
<2> The organic transistor described in <1>, in which the organic semiconductor film contains one or more surfactants selected from the group consisting of a silicone-based surfactant, a fluorine-based surfactant, and a hydrocarbon-based surfactant.
<3> The organic transistor described in <1> or <2>, in which the first gate insulating film contains one or more surfactants selected from the group consisting of a silicone-based surfactant, a fluorine-based surfactant, and a hydrocarbon-based surfactant.
<4> The organic transistor described in any one of <1> through <3>, in which a material used for the first gate insulating film is a cycloolefin copolymer represented by general formula (4z-1) or (4z-2) below, 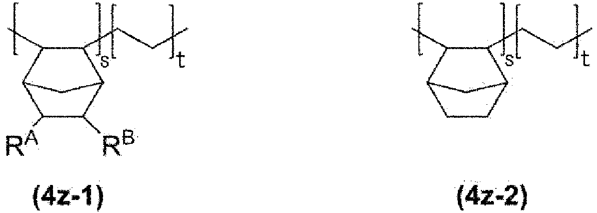 where s and t each represent the number of repetitions, s represents an integer of 1 or more, and t represents an integer of 0 or more, R^{A} and R^{B} each independently represent hydrogen, an alkyl group having 1 to 50 carbon atoms, or an alkoxy group having 1 to 50 carbon atoms, and R^{A} and R^{B} may be bonded to each other to form a ring together with a carbon atom to which R^{A} and R^{B} are bonded.
<5> The organic transistor described in any one of <1> through <4>, in which in the structural unit represented by the general formula (2), either one or both of A and B are linking groups represented by general formulae (4-1) through (4-8) below, 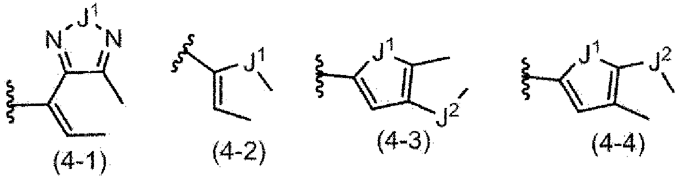 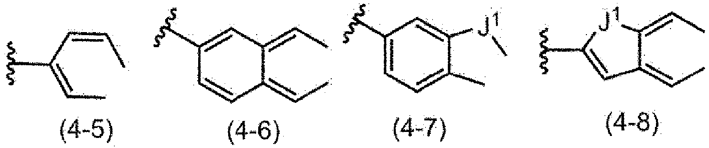 where J¹ and J² each independently represent a chalcogen atom.
<6> The organic transistor described in any one of <1> through <5>, in which the structural unit represented by the general formula (2) is a structural unit represented by general formula (2-1) below, 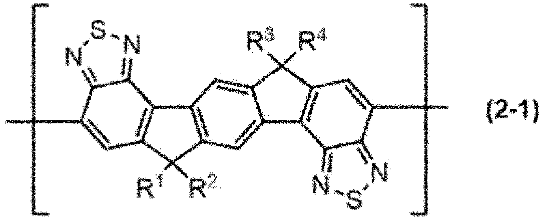 where R¹, R², R³, and R⁴ each independently represent an alkyl group having 1 to 50 carbon atoms, R¹ and R² may be bonded to each other to form a ring together with a carbon atom to which R¹ and R² are bonded, and R³ and R⁴ may be bonded to each other to form a ring together with a carbon atom to which R³ and R⁴ are bonded.
<7> The organic transistor described in any one of <1> through <6>, in which the conjugated polymer is composed of a structural unit represented by general formula (24) below, and X in the general formula (24) is a linking group selected from the group consisting of general formulae (3-1) through (3-3) below, 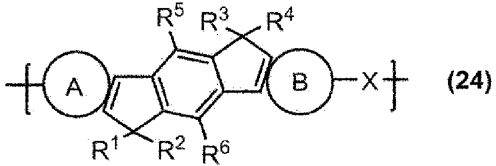
   where A and B each independently represent a monovalent aromatic ring linking group which may be substituted with an alkyl group having 1 to 50 carbon atoms or an alkoxy group having 1 to 50 carbon atoms, and R¹, R², R³, and R⁴ each independently represent an alkyl group having 1 to 50 carbon atoms, R¹ and R² may be bonded to each other to form a ring together with a carbon atom to which R¹ and R² are bonded, R³ and R⁴ may be bonded to each other to form a ring together with a carbon atom to which R³ and R⁴ are bonded, and R⁵ and R⁶ each independently represent a hydrogen atom, a fluorine atom, or an alkyl group having 1 to 50 carbon atoms, 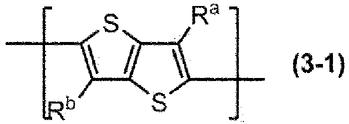 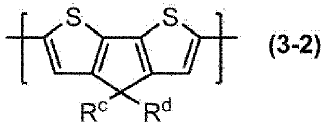 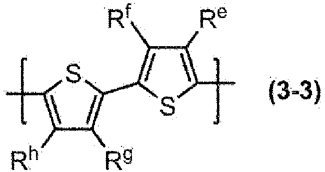
   where R^{a}, R^{b}, R^{c}, R^{d}, R^{e}, R^{f}, R^{g}, and R^{h} each independently represent hydrogen, an alkyl group having 1 to 50 carbon atoms, or an alkoxy group having 1 to 50 carbon atoms.
<8> The organic transistor described in any one of <1> through <7>, in which the conjugated polymer is composed of a structural unit represented by general formula (24) below, 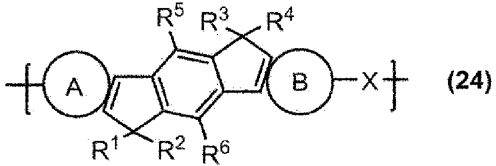
   where R¹, R², R³ and R⁴ each independently represent an alkyl group having 1 to 50 carbon atoms, R¹ and R² may be bonded to each other to form a ring together with a carbon atom to which R¹ and R² are bonded, R³ and R⁴ may be bonded to each other to form a ring together with a carbon atom to which R³ and R⁴ are bonded, and R⁵ and R⁶ each independently represent a hydrogen atom, a fluorine atom, or an alkyl group having 1 to 50 carbon atoms,
   A and B in the general formula (24) are linking groups represented by general formula (4-1) below, and X is a linking group selected from the group consisting of general formulae (3-1) through (3-3) below, 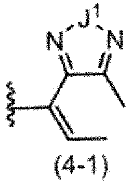
   where J¹ independently represents a chalcogen atom, 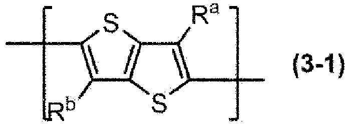 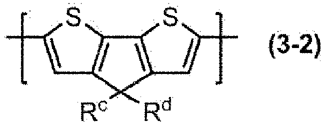 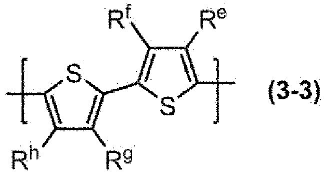
   where R^{a}, R^{b}, R^{c}, R^{d}, R^{e}, R^{f}, R^{g}, and R^{h} each independently represent hydrogen, an alkyl group having 1 to 50 carbon atoms, or an alkoxy group having 1 to 50 carbon atoms.
<9> The organic transistor described in any one of <1> through <8>, in which the interfacial energy is 1.5 mJ/m² or less.

### Examples

The following description will discuss the present invention in further detail with reference to Examples. Note, however, that the present invention is not limited to those Examples below.

A molecular weight and a molecular weight distribution of the conjugated polymer obtained in Examples were estimated by gel permeation chromatography (GPC) measurement. Commercially available reagents were used.

### <NMR measurement conditions>

Measurement device: Bruker ASCEND^{™} ADVANCE III HD (400 MHz)
Measurement solvent: deuterated chloroform (CDCl₃) or deuterated DMSO (DMSO-d₆)
Internal standard substance: tetramethylsilane (TMS)

### [Reference Synthesis Example 1]

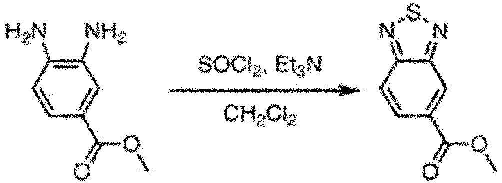

Thionyl chloride (5.30 mL, 72.6 mmol) was added to a mixture of methyl 3,4-diaminobenzoate (6.00 g, 36.1 mmol), triethylamine (20.0 mL, 143 mmol), and dichloromethane (180 mL), and the mixture was refluxed for 4 hours. After the resultant mixture was cooled to room temperature, a saturated sodium hydrogen carbonate aqueous solution was added, and extraction was carried out with chloroform. The collected organic layer was washed with water and saturated brine, dried over anhydrous magnesium sulfate and filtered, and the solvent was evaporated under reduced pressure. The resultant residue was purified by silica gel column chromatography (hexane/chloroform) to obtain methyl benzo-2,1,3-thiadiazole-5-carboxylate (4.49 g, 64%) as a white solid. ¹H-NMR (CDCl₃, 400 MHz) δ (ppm): 8.76 (dd, J = 1.6, 0.8 Hz, 1H), 8.23 (dd, J = 9.2, 1.6 Hz, 1H), 8.06 (dd, J = 9.2, 0.8 Hz, 1H), 4.02 (s, 3H).

### [Reference Synthesis Example 2]

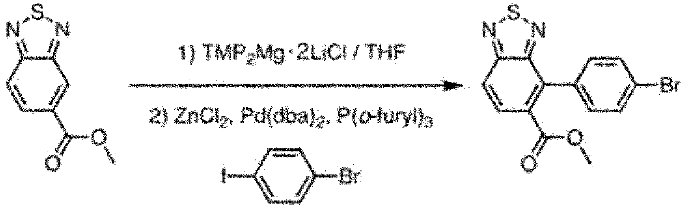

A hexane solution of n-butyllithium (3.3 mL, 1.6 mol/L, 5.1 mmol) was added at -40°C to a mixture of 2,2,6,6-tetramethylpiperidine (880 µL, 5.17 mmol) and tetrahydrofuran (5.1 mL), and the mixture was stirred at 0°C for 30 minutes. After that, a solution of 2,2,6,6-tetramethylpiperidinylmagnesium chloride lithium chloride complex (5.1 mL, 5.1 mmol) was added to the mixture and the mixture was stirred at 0°C for 30 minutes and at room temperature for 1 hour. The resultant mixture was evaporated under reduced pressure to remove the solvent, and tetrahydrofuran (7.4 mL) was added to prepare a TMP₂Mg·2LiCl solution.

The TMP₂Mg·2LiCl solution was added at -40°C to a mixture of methyl benzo-2,1,3-thiadiazole-5-carboxylate (1.50 g, 7.72 mmol) obtained in Reference Synthesis Example 1 and tetrahydrofuran (34 mL), and the mixture was stirred for 3.5 hours. After that, a zinc chloride solution (6.2 mL, 6.2 mmol), bis(dibenzylideneacetone)palladium(0) (59.0 mg, 103 µmol), tri(2-furyl)phosphine (48.0 mg, 207 µmol), and 1-bromo-4-iodobenzene (2.19 g, 7.74 mmol) were added to the mixture under an argon gas stream, and the mixture was stirred at 70°C for 15.5 hours. After the resultant mixture was cooled to room temperature, a saturated ammonium chloride aqueous solution was added, and extraction was carried out with ethyl acetate. The collected organic layer was washed with water and saturated brine, dried over anhydrous magnesium sulfate and filtered, and the solvent was evaporated under reduced pressure. The resultant residue was purified by silica gel column chromatography (hexane/chloroform) to obtain methyl 4-(4-bromo-phenyl)-2,1,3-benzothiadiazole-5-carboxylate (1.81 g, 69%) as a light yellow solid.
¹H-NMR (CDCl₃, 400 MHz) δ (ppm): 8.08-8.03 (m, 2H), 7.65-7.63 (m, 2H), 7.36-7.26 (m, 2H), 3.72 (s, 3H).

### [Reference Synthesis Example 3]

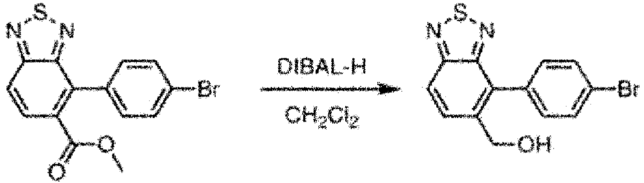

Diisobutylaluminum hydride (6.40 mL, 6.40 mmol) was added at -10°C to a mixture of methyl 4-(4-bromo-phenyl)-2,1,3-benzothiadiazole-5-carboxylate (900 mg, 2.58 mmol) obtained in Reference Synthesis Example 2 and dichloromethane (26 mL), and the mixture was stirred for 3 hours. Methanol and a Rochelle salt aqueous solution were added to the resultant mixture, and the mixture was stirred at room temperature for 30 minutes. The resultant mixture was extracted with chloroform, and the collected organic layer was dried over anhydrous magnesium sulfate and filtered. The solvent was evaporated under reduced pressure to obtain 4-(4-bromo-phenyl)-5-hydroxymethyl-2,1,3-benzothiadiazole (750 mg, 91%) as a light yellow solid.
¹H-NMR (CDCl₃, 400 MHz) δ (ppm): 8.04 (d, J = 9.2 Hz, 1H), 7.91 (d, J = 9.2 Hz, 1H), 7.68-7.66 (m, 2H), 7.37-7.35 (m, 2H), 4.74 (d, J = 5.6 Hz, 2H), 1.77 (t, J = 5.6 Hz, 1H).

### [Reference Synthesis Example 4]

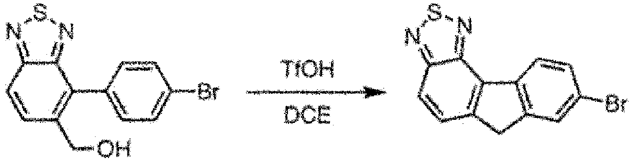

Trifluoromethanesulfonic acid (3.10 mL, 35.0 mmol) was added to a mixture of 4-(4-bromo-phenyl)-5-hydroxymethyl-2,1,3-benzothiadiazole (750 mg, 2.34 mmol) obtained in Reference Synthesis Example 3 and 1,2-dichloroethane (23 mL), and the mixture was stirred at 90°C for 3 hours. After the resultant mixture was cooled to room temperature, a saturated sodium hydrogen carbonate aqueous solution was added, and extraction was carried out with chloroform. The collected organic layer was washed with water and saturated brine, dried over anhydrous magnesium sulfate, and then filtered, and the solvent was evaporated under reduced pressure to obtain 8-bromo-6H-fluoreno[3,4-c][1,2,5]thiadiazole (591 mg, 84%) as a light yellow solid.
¹H-NMR (CDCl₃, 400 MHz) δ (ppm): 8.40 (d, J = 8.0 Hz, 1H), 7.98 (d, J = 8.4 Hz, 1H), 7.81-7.78 (m, 2H), 7.66 (dd, J = 8.0, 1.6 Hz, 1H), 4.05 (s, 2H).

### [Reference Synthesis Example 5]

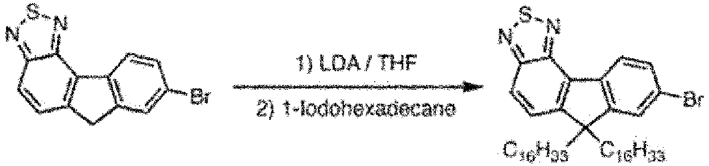

Lithium diisopropylamide (11 mL, 11 mmol) was added at -10°C to a mixture of 8-bromo-6H-fluoreno[3,4-c][1,2,5]thiadiazole (1.68 g, 5.54 mmol) obtained in Reference Synthesis Example 4 and tetrahydrofuran (28 mL), and the mixture was stirred for 1.5 hours. After that, 1-iodohexadecane (5.2 mL, 16.6 mmol) was added to the mixture and the mixture was stirred at room temperature for 24 hours. Water was added to the reaction solution, and extraction was carried out with hexane. The collected organic layer was washed with water and saturated brine, dried over anhydrous magnesium sulfate and filtered off, and the solvent was evaporated under reduced pressure. The resultant residue was purified by silica gel column chromatography (hexane/chloroform) to obtain 8-bromo-6,6-dihexadecyl-6H-fluoreno[3,4-c][1,2,5]thiadiazole (2.38 g, 57%) as a light yellow solid.
¹H-NMR (CDCl₃, 400 MHz) δ (ppm): 8.31 (d, J = 8.0 Hz, 1H), 7.98 (d, J = 8.8 Hz, 1H), 7.63-7.54 (m, 3H), 2.05 (t, J = 8.2 Hz, 4H), 1.31-1.02 (m, 52H), 0.87 (t, J = 7.0 Hz, 6H), 0.64-0.47 (m, 4H).

### [Reference Synthesis Example 6]

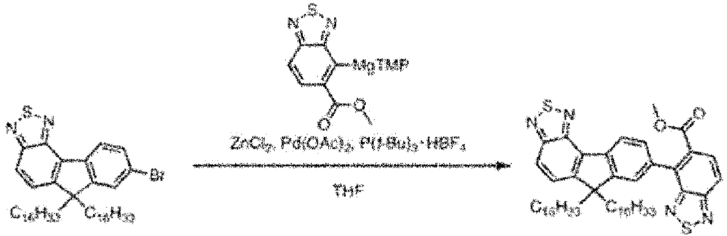

A hexane solution of n-butyllithium (2.4 mL, 1.6 mol/L, 3.7 mmol) was added at -40°C to a mixture of 2,2,6,6-tetramethylpiperidine (0.62 mL, 3.7 mmol) and tetrahydrofuran (3.7 mL), and the mixture was stirred at 0°C for 30 minutes. After that, a solution of 2,2,6,6-tetramethylpiperidinylmagnesium chloride lithium chloride complex (3.7 mL, 3.7 mmol) was added to the mixture and the mixture was stirred at 0°C for 30 minutes and at room temperature for 1 hour. The resultant mixture was evaporated under reduced pressure to remove the solvent, and tetrahydrofuran (5.2 mL) was added to prepare a TMP₂Mg·2LiCl solution.

The TMP₂Mg·2LiCl solution was added at -40°C to a mixture of methyl benzo-2,1,3-thiadiazole-5-carboxylate (713 mg, 3.67 mmol) obtained in Reference Synthesis Example 1 and tetrahydrofuran (11 mL), and the mixture was stirred for 3 hours. After that, a zinc chloride solution (8.0 mL, 4.0 mmol), palladium(II) acetate (14 mg, 60 µmol), tri-t-butylphosphonium tetrafluoroborate (36 mg, 120 µmol), and 8-bromo-6,6-dihexadecyl-6H-fluoreno[3,4-c][1,2,5]thiadiazole (2.30 g, 3.06 mmol) obtained in Reference Synthesis Example 5 were added to the mixture under an argon gas stream, and the mixture was stirred at 70°C for 62 hours. After the resultant mixture was cooled to room temperature, a saturated ammonium chloride aqueous solution was added, and extraction was carried out with ethyl acetate. The collected organic layer was washed with water and saturated brine, dried over anhydrous magnesium sulfate and then filtered, and the solvent was evaporated under reduced pressure. The resultant residue was purified by silica gel column chromatography (hexane/chloroform) to obtain methyl (6,6-dihexadecyl-6H-fluoreno[3,4-c][1,2,5]thiadiazo-8-yl)-2,1,3-benzothiadiazole-5-carboxylate (1.57 g, 60%) as a light yellow solid.
¹H-NMR (CDCl₃, 400 MHz) δ (ppm): 8.61 (d, J = 7.2 Hz, 1H), 8.08-7.99 (m, 3H), 7.98 (d, J = 8.8 Hz, 1H), 7.62 (dd, J = 8.0, 1.6 Hz, 1H), 7.53 (d, J = 1.6 Hz, 1H), 3.63 (s, 3H), 2.13-2.08 (m, 4H), 1.30-1.10 (m, 52H), 0.87 (t, J = 7.0 Hz, 6H), 0.82-0.60 (m, 4H).

### [Reference Synthesis Example 7]

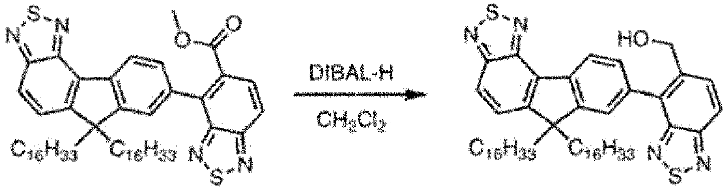

Diisobutylaluminum hydride (4.3 mL, 4.3 mmol) was added at -10°C to a mixture of methyl (6,6-dihexadecyl-6H-fluoreno[3,4-c][1,2,5]thiadiazo-8-yl)-2,1,3-benzothiadiazole-5-carboxylate (1.50 g, 1.73 mmol) obtained in Reference Synthesis Example 6 and dichloromethane (17 mL), and the mixture was stirred for 3.5 hours. Methanol and a Rochelle salt aqueous solution were added to the resultant mixture, and the mixture was stirred at room temperature for 30 minutes. The resultant mixture was extracted with chloroform, and the collected organic layer was dried over anhydrous magnesium sulfate and filtered off. The solvent was evaporated under reduced pressure to obtain 8-(5-hydroxymethyl-2,1,3-benzothiadiazo-4-yl)-6,6-dihexadecyl-6H-fluoreno[3,4-c][1,2,5]thiadiazole (1.30 g, 90%) as a light yellow solid.
¹H-NMR (CDCl₃, 400 MHz) δ (ppm): 8.62 (d, J = 7.6 Hz, 1H), 8.06 (d, J = 8.8 Hz, 1H), 8.01 (d, J = 8.8 Hz, 1H), 7.96 (d, J = 9.2 Hz, 1H), 7.69 (d, J = 9.2 Hz, 1H), 7.61 (dd, J = 7.6, 1.2 Hz, 1H), 7.54 (d, J = 1.2 Hz, 1H), 4.83 (d, J = 5.6 Hz, 2H), 2.17-2.04 (m, 4H), 1.76 (t, J = 5.6 Hz, 1H), 1.30-1.05 (m, 52H), 0.90-0.85 (m, 6H), 0.78-0.63 (m, 4H).

### [Reference Synthesis Example 8]

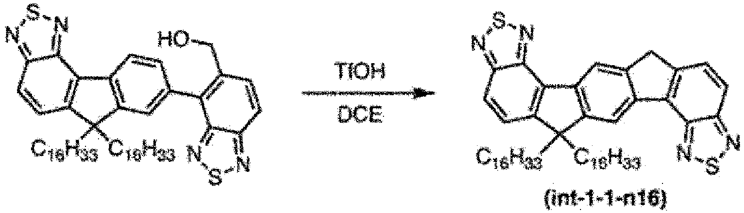

Trifluoromethanesulfonic acid (2.0 mL, 23 mmol) was added to a mixture of 8-(5-hydroxymethyl-2,1,3-benzothiadiazo-4-yl)-6,6-dihexadecyl-6H-fluoreno[3,4-c][1,2,5]thiadiazole (1.30 g, 1.55 mmol) obtained in Reference Synthesis Example 7 and 1,2-dichloroethane (16 mL), and the mixture was stirred at room temperature for 3 hours. After the resultant mixture was cooled to room temperature, a saturated sodium hydrogen carbonate aqueous solution was added, and extraction was carried out with chloroform. The collected organic layer was washed with water and saturated brine, dried over anhydrous magnesium sulfate and filtered off, and the solvent was evaporated under reduced pressure. The resultant residue was purified by silica gel column chromatography (hexane/ethyl acetate) to obtain (int-1-1-n16) (951 mg, 75%) as a light yellow solid.
¹H-NMR (CDCl₃, 400 MHz) δ (ppm): 8.75 (s, 1H), 8.59 (s, 1H), 8.00-7.96 (m, 2H), 7.87 (d, J = 8.8 Hz, 1H), 7.71 (d, J = 8.8 Hz, 1H), 4.21 (s, 2H), 2.35-2.28 (m, 2H), 2.21-2.14 (m, 2H), 1.32-1.05 (m, 52H), 1.00-0.84 (m, 6H), 0.72-0.56 (m, 4H).

### [Reference Synthesis Example 9]

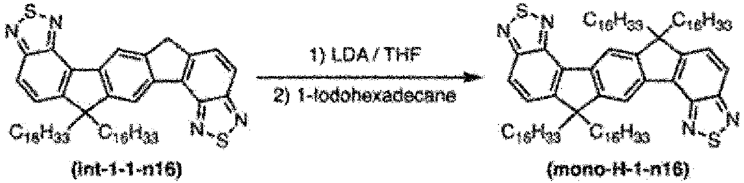

Lithium diisopropylamide (2.2 mL, 2.4 mmol) was added at -10°C to a mixture of (int-1-1-n16) (900 mg, 1.10 mmol) obtained in Reference Synthesis Example 8 and tetrahydrofuran (22 mL), and the mixture was stirred for 2.5 hours. After that, 1-iodohexadecane (0.76 mL, 2.4 mmol) was added to the mixture and the mixture was stirred at room temperature for 17 hours. Water was added to the reaction solution, and extraction was carried out with ethyl acetate. The collected organic layer was washed with water and saturated brine, dried over anhydrous magnesium sulfate and then filtered, and the solvent was evaporated under reduced pressure. The resultant residue was purified by silica gel column chromatography (hexane/chloroform) to obtain (mono-H-1-n16) (835 mg, 60%) as a light yellow solid.
¹H-NMR (CDCl₃, 400 MHz) δ (ppm): 8.49 (s, 2H), 7.98 (d, J = 8.8 Hz, 2H), 7.70 (d, J = 8.8 Hz, 2H), 2.33-2.26 (m, 4H), 2.21-2.14 (m, 4H), 1.29-0.98 (m, 104H), 0.88 (t, J = 7.0 Hz, 12H), 0.71-0.58 (m, 8H).

### [Reference Synthesis Example 10]

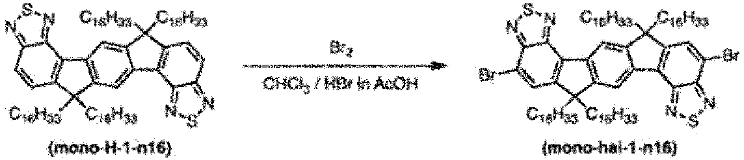

Bromine (0.20 mL, 3.8 mmol) was added to a mixture of (mono-H-1-n16) (800 mg, 0.631 mmol) obtained in Reference Synthesis Example 9, chloroform (8.4 mL), and hydrogen bromide (30% acetic acid solution) (4.2 mL), and the mixture was refluxed for 3 hours. After the resultant mixture was cooled to room temperature, a saturated sodium hydrogen carbonate aqueous solution and a saturated sodium thiosulfate aqueous solution were added, and extraction was carried out with chloroform. The collected organic layer was washed with water and saturated brine, dried over anhydrous magnesium sulfate and then filtered, and the solvent was evaporated under reduced pressure. The resultant residue was purified by silica gel column chromatography (hexane/chloroform) to obtain (mono-hal-1-n16) (832 mg, 92%) as a yellow solid.
¹H-NMR (CDCl₃, 400 MHz) δ (ppm): 8.43 (s, 2H), 7.92 (s, 2H), 2.32-2.24 (m, 4H), 2.18-2.10 (m, 4H), 1.29-1.00 (m, 104H), 0.86 (t, J = 7.0 Hz, 12H), 0.66-0.58 (m, 8H).

### [Reference Synthesis Example 11]

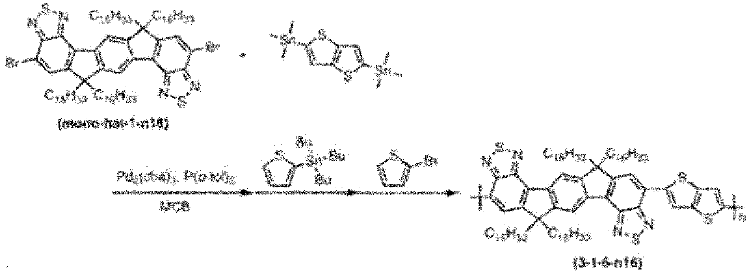

A mixture of (mono-hal-1-n16) (110 mg, 77.0 µmol) obtained in Reference Synthesis Example 10, 2,5-bis(trimethylstannyl)thieno[3,2-b]thiophene (35.9 mg, 77.0 µmol), and chlorobenzene (2.0 mL) was bubbled with argon for 30 minutes. Pd₂(dba)₃·CHCl₃ (1.6 mg, 1.5 µmol) and tri(o-tolyl)phosphine (1.9 mg, 6.2 µmol) were added to this mixed solution, and the mixture was stirred at 180°C for 2 hours using a microwave reactor. After that, 2-(tributylstannyl)thiophene (220 µL, 690 µmol) was added to the reaction solution, and the mixture was stirred at 180°C for 10 minutes using a microwave reactor. Further, 2-bromothiophene (70 µL, 770 µmol) was added to the mixture and the mixture was stirred at 180°C for 10 minutes using a microwave reactor. After the resultant mixture was cooled to room temperature, the resultant mixture was precipitated in a mixed solution of methanol/concentrated hydrochloric acid (150 mL/15 mL), and a deposited solid was filtered. The obtained solid was subjected to Soxhlet extraction using methanol, acetone, hexane, and cyclohexane to remove components soluble in these solvents. Further, the filter residue was dissolved in THF. The resultant mixture was concentrated under reduced pressure, the resultant concentrate was precipitated in methanol, and a deposited solid was filtered. The obtained solid was washed with methanol and then dried under reduced pressure at 90°C to obtain (3-1-6-n16) as a black solid (38 mg, 35%).
GPC (THF): Mn = 31600 g/mol, Mw = 51100 g/mol, PDI = 1.62. GPC (TCB, 140°C): Mn = 35000 g/mol, Mw = 56000 g/mol, PDI = 1.6.

### [Example 1]

A composition for forming an organic semiconductor film was prepared by heating a 0.5 wt% o-DCB solution of the conjugated polymer (3-1-6-n16) synthesized in Reference Synthesis Example 11 in a glove box under a nitrogen atmosphere.

After cooling to room temperature, the entire amount was filtered through a 0.22 µm filter, and thus it was confirmed that the solution state was maintained and that the compound was suitable for film formation.

Next, Parylene C was formed as an underlayer on a glass substrate by a CVD method, then a shadow mask having a channel length of 100 µm and a channel width of 500 µm was placed on the Parylene C layer, and a source electrode and a drain electrode were provided by deposition of silver under vacuum. The solution prepared above was spin-coated in a glove box under a nitrogen atmosphere. The coated film was heated to 150°C and maintained for 15 minutes to form an organic semiconductor film of the conjugated polymer (3-1-6-n16). Next, a 4 wt% toluene solution of TOPAS (Sigma-Aldrich) was spin-coated in air as a first gate insulating film in contact with the organic semiconductor film. The coated film was heated to 120°C and maintained for 10 minutes to form a TOPAS insulating film.

Further, after Parylene C was formed as a second gate insulating film by a CVD method, a silver electrode was formed by a deposition method to produce a top gate-bottom contact type organic transistor (the gate electrode was silver, the gate insulating layers were TOPAS and Parylene C, and the source electrode and the drain electrode were silver).

### (Calculation of interfacial energy)

The interfacial energy calculated from a contact angle of water and a contact angle of diiodomethane with respect to the organic semiconductor film, and a contact angle of water and a contact angle of diiodomethane with respect to the insulating film was 0.34 mJ/m².

### (Measurement of semiconductor/electrical properties)

In air, the organic transistor was connected to a semiconductor parameter analyzer (manufactured by Keithley Instruments, Model 4200A-SCS), and transfer characteristics were evaluated by scanning a gate voltage (Vg) from +10 to - 50 V in 1 V increments at a drain voltage Vd of -50 V. The organic transistor exhibited p-type characteristics, and the hole carrier mobility of the organic transistor was 7.05 cm²/Vs.

### [Example 2]

Operations similar to those in Example 1 were repeated, except that parylene was used instead of TOPAS for the first gate insulating film in contact with the organic semiconductor film and gold was used for the source electrode and the drain electrode. The interfacial energy calculated from a contact angle of water and a contact angle of diiodomethane with respect to the organic semiconductor film, and a contact angle of water and a contact angle of diiodomethane with respect to the insulating film was 1.48 mJ/m². The obtained organic transistor exhibited p-type characteristics, and the hole carrier mobility thereof was 1.76 cm²/Vs.

### [Example 3]

Operations similar to those in Example 1 were repeated, except that a 5 wt% m-xylene solution of polystyrene (Sigma-Aldrich) was used instead of the 4 wt% toluene solution of TOPAS for the formation of the first gate insulating film in contact with the organic semiconductor film. The interfacial energy calculated from a contact angle of water and a contact angle of diiodomethane with respect to the organic semiconductor film, and a contact angle of water and a contact angle of diiodomethane with respect to the insulating film was 1.26 mJ/m². The obtained organic transistor exhibited p-type characteristics, and the hole carrier mobility thereof was 5.51 cm²/Vs.

### [Example 4]

Operations similar to those in Example 1 were repeated, except that a 5 wt% m-xylene solution (containing 0.5 wt% of poly(methylphenylsiloxane) [silicone-based surfactant]) of an insulating material synthesized by the method described in WO2023/074606 (resin 1 in the said publication) was used instead of the 4 wt% toluene solution of TOPAS for the formation of the first gate insulating film in contact with the organic semiconductor film. The interfacial energy calculated from a contact angle of water and a contact angle of diiodomethane with respect to the organic semiconductor film, and a contact angle of water and a contact angle of diiodomethane with respect to the insulating film was 0.82 mJ/m². The obtained organic transistor exhibited p-type characteristics, and the hole carrier mobility thereof was 3.97 cm²/Vs.

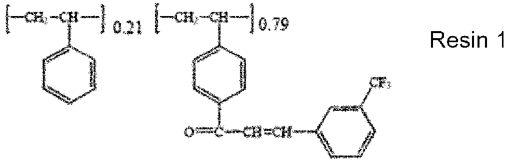

### [Comparative Example 1]

Operations similar to those in Example 1 were repeated, except that a 5 wt% m-xylene solution of an insulating material synthesized by the method described in WO2023/074606 (resin 1 in the said publication) was used instead of the 4 wt% toluene solution of TOPAS for the formation of the first gate insulating film in contact with the organic semiconductor film. The interfacial energy calculated from a contact angle of water and a contact angle of diiodomethane with respect to the organic semiconductor film, and a contact angle of water and a contact angle of diiodomethane with respect to the insulating film was 3.16 mJ/m². The obtained organic transistor exhibited p-type characteristics, and the hole carrier mobility thereof was 0.46 cm²/Vs.

### Reference Signs List

1: Active layer (organic semiconductor film)
2: Substrate
3: Gate electrode
4: First gate insulating film
5: Source electrode
6: Drain electrode
7: Second gate insulating film

## Claims

1. An organic transistor comprising:
a gate electrode, a source electrode, a drain electrode, and an organic semiconductor film; and
a first gate insulating film in contact with the organic semiconductor film, or a first gate insulating film in contact with the organic semiconductor film and a second gate insulating film not in contact with the organic semiconductor film,
the organic semiconductor film containing a conjugated polymer composed of a structural unit represented by general formula (2) below and a structural unit represented by general formula (3) below, and
an interfacial energy between the first gate insulating film and the organic semiconductor film being 2.0 mJ/m² or less, 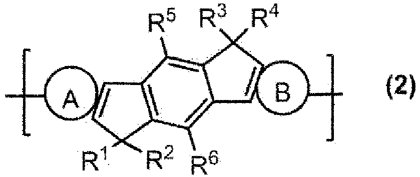
where A and B each independently represent a monovalent aromatic ring linking group which may be substituted with an alkyl group having 1 to 50 carbon atoms or an alkoxy group having 1 to 50 carbon atoms, and R¹, R², R³, and R⁴ each independently represent an alkyl group having 1 to 50 carbon atoms, R¹ and R² may be bonded to each other to form a ring together with a carbon atom to which R ¹ and R² are bonded, R³ and R⁴ may be bonded to each other to form a ring together with a carbon atom to which R³ and R⁴ are bonded, and R⁵ and R⁶ each independently represent a hydrogen atom, a fluorine atom, or an alkyl group having 1 to 50 carbon atoms, 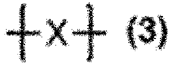
where X represents a divalent heteroaromatic ring linking group which may be substituted with an alkyl group having 1 to 50 carbon atoms or an alkoxy group having 1 to 50 carbon atoms.

2. The organic transistor as set forth in claim 1, wherein the organic semiconductor film contains one or more surfactants selected from the group consisting of a silicone-based surfactant, a fluorine-based surfactant, and a hydrocarbon-based surfactant.

3. The organic transistor as set forth in claim 1, wherein the first gate insulating film contains one or more surfactants selected from the group consisting of a silicone-based surfactant, a fluorine-based surfactant, and a hydrocarbon-based surfactant.

4. The organic transistor as set forth in claim 1, wherein a material used for the first gate insulating film is a cycloolefin copolymer represented by general formula (4z-1) or (4z-2) below, 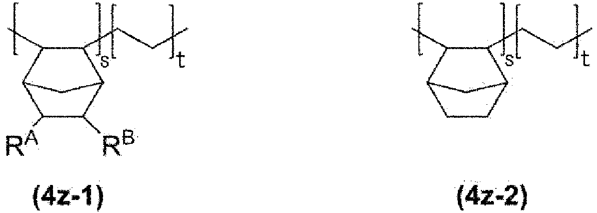 where s and t each represent the number of repetitions, s represents an integer of 1 or more, and t represents an integer of 0 or more, R^{A} and R^{B} each independently represent hydrogen, an alkyl group having 1 to 50 carbon atoms, or an alkoxy group having 1 to 50 carbon atoms, and R^{A} and R^{B} may be bonded to each other to form a ring together with a carbon atom to which R^{A} and R^{B} are bonded.

5. The organic transistor as set forth in claim 1, wherein in the structural unit represented by the general formula (2) in the organic semiconductor film, either one or both of A and B are linking groups represented by general formulae (4-1) through (4-8) below, 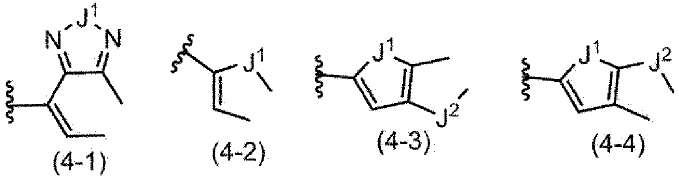 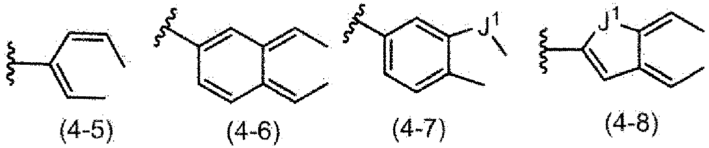 where J¹ and J² each independently represent a chalcogen atom.

6. The organic transistor as set forth in claim 1, wherein the structural unit represented by the general formula (2) in the organic semiconductor film is a structural unit represented by general formula (2-1) below, 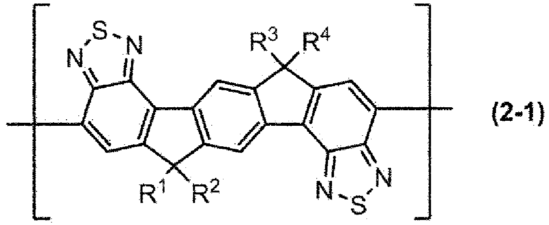 where R¹, R², R³, and R⁴ each independently represent an alkyl group having 1 to 50 carbon atoms, R¹ and R² may be bonded to each other to form a ring together with a carbon atom to which R¹ and R² are bonded, and R³ and R⁴ may be bonded to each other to form a ring together with a carbon atom to which R³ and R⁴ are bonded.

7. The organic transistor as set forth in claim 1, wherein the conjugated polymer in the organic semiconductor film is composed of a structural unit represented by general formula (24) below, and X in the general formula (24) is a linking group selected from the group consisting of general formulae (3-1) through (3-3) below, 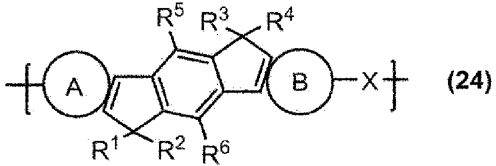
where A and B each independently represent a monovalent aromatic ring linking group which may be substituted with an alkyl group having 1 to 50 carbon atoms or an alkoxy group having 1 to 50 carbon atoms, and R¹, R², R³, and R⁴ each independently represent an alkyl group having 1 to 50 carbon atoms, R¹ and R² may be bonded to each other to form a ring together with a carbon atom to which R¹ and R² are bonded, R³ and R⁴ may be bonded to each other to form a ring together with a carbon atom to which R³ and R⁴ are bonded, and R⁵ and R⁶ each independently represent a hydrogen atom, a fluorine atom, or an alkyl group having 1 to 50 carbon atoms, 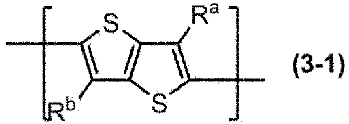 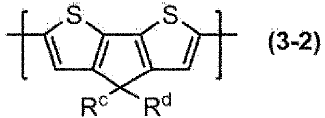 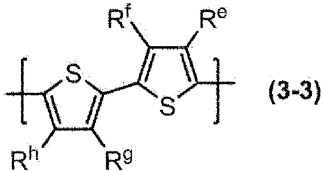
where R^{a}, R^{b}, R^{c}, R^{d}, R^{e}, R^{f}, R^{g}, and R^{h} each independently represent hydrogen, an alkyl group having 1 to 50 carbon atoms, or an alkoxy group having 1 to 50 carbon atoms.

8. The organic transistor as set forth in claim 1, wherein:
the conjugated polymer in the organic semiconductor film is composed of a structural unit represented by general formula (24) below, 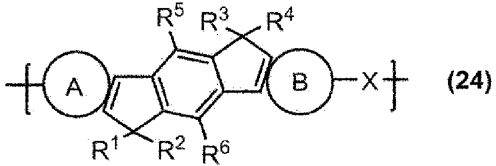
where R¹, R², R³ and R⁴ each independently represent an alkyl group having 1 to 50 carbon atoms, R¹ and R² may be bonded to each other to form a ring together with a carbon atom to which R¹ and R² are bonded, R³ and R⁴ may be bonded to each other to form a ring together with a carbon atom to which R³ and R⁴ are bonded, and R⁵ and R⁶ each independently represent a hydrogen atom, a fluorine atom, or an alkyl group having 1 to 50 carbon atoms; and
A and B in the general formula (24) are linking groups represented by general formula (4-1) below, and X is a linking group selected from the group consisting of general formulae (3-1) through (3-3) below, 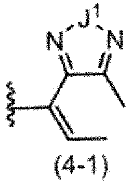
where J¹ independently represents a chalcogen atom, 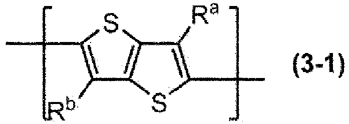 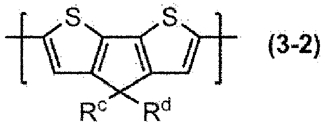 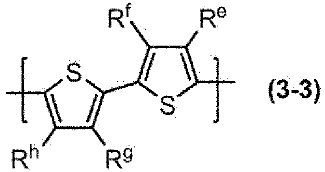
where R^{a}, R^{b}, R^{c}, R^{d}, R^{e}, R^{f}, R^{g}, and R^{h} each independently represent hydrogen, an alkyl group having 1 to 50 carbon atoms, or an alkoxy group having 1 to 50 carbon atoms.

9. The organic transistor as set forth in claim 1, wherein the interfacial energy is 1.5 mJ/m² or less.
